(19) Europäisches Patentamt
European Patent Office
Office européen des brevets

(11) **EP 3 842 547 A1**

(12) **EUROPEAN PATENT APPLICATION**
published in accordance with Art. 153(4) EPC

(43) Date of publication:
**30.06.2021 Bulletin 2021/26**

(21) Application number: **19853005.7**

(22) Date of filing: **21.08.2019**

(51) Int Cl.:
*C12Q 1/6809* (2018.01)     *C12Q 1/02* (2006.01)
*C12N 15/13* (2006.01)     *C12Q 1/6869* (2018.01)
*C12Q 1/6883* (2018.01)

(86) International application number:
**PCT/JP2019/032673**

(87) International publication number:
**WO 2020/040210 (27.02.2020 Gazette 2020/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **22.08.2018   JP 2018155380
12.03.2019   JP 2019044885**

(71) Applicants:
• **NATIONAL CENTER OF NEUROLOGY AND
PSYCHIATRY
Kodaira-shi
Tokyo 187-8551 (JP)**
• **Repertoire Genesis Incorporation
Ibaraki-shi, Osaka 567-0085 (JP)**

(72) Inventors:
• **YAMAMURA, Takashi
Kodaira-shi, Tokyo 187-8551 (JP)**
• **SATO, Wakiro
Kodaira-shi, Tokyo 187-8551 (JP)**
• **ONO, Hirohiko
Kodaira-shi, Tokyo 187-8551 (JP)**
• **MATSUTANI, Takaji
Ibaraki-shi, Osaka 567-0085 (JP)**
• **NAKAMURA, Yukio
Ibaraki-shi, Osaka 567-0085 (JP)**
• **KITAURA, Kazutaka
Ibaraki-shi, Osaka 567-0085 (JP)**

(74) Representative: **Uexküll & Stolberg
Partnerschaft von
Patent- und Rechtsanwälten mbB
Beselerstraße 4
22607 Hamburg (DE)**

(54) **BIOMARKER FOR MYALGIC ENCEPHALOMYELITIS/CHRONIC FATIGUE SYNDROME (ME/CFS)**

(57)     A method for diagnosing myalgic encephalomyelitis/chronic fatigue syndrome (ME/CFS) is provided in the present disclosure. The present disclosure provides a method that uses the B cell receptor (BCR) repertoire as an indicator of myalgic encephalomyelitis/chronic fatigue syndrome (ME/CFS). One or more variables selected from the group consisting of the frequency of use of one or more genes in the IgGH heavy chain variable region of the BCR in a subject, the BCR diversity index in a subject, and the level of one or more immune cell subpopulations in a subject can be used as an indicator of ME/CFS in the subject.

FIG.11

Fig.11

EP 3 842 547 A1

**Description**

[Technical Field]

The present disclosure relates to the field of diagnosis of diseases, especially myalgic encephalomyelitis/chronic fatigue syndrome (ME/CFS).

[Background Art]

Myalgic encephalomyelitis/chronic fatigue syndrome (ME/CFS) is a disease with severe fatigue, exhaustion after exertion, sleep disorder, cognitive dysfunction, or orthostatic intolerance as the core symptom and involving various symptoms such as pain, autonomic neuropathy, or hypersensitivity to light, sound, food, or chemical substance. While the syndrome develops after an infection in some cases, the pathology is still not elucidated. Since there is no biomarker for identifying the development thereof, various diagnostic criteria are used, which is an impediment to diagnosis or research. Further, an effective therapeutic method has not been established.

[Summary of Invention]

[Solution to Problem]

The present disclosure provides a method of using a B cell receptor (BCR) repertoire as an indicator of myalgic encephalomyelitis/chronic fatigue syndrome (ME/CFS). It is demonstrated herein that usage frequency of a gene in an IgG H chain variable region of a BCR varies in an ME/CFS patient in comparison to a healthy control. Such information can be used to diagnose ME/CFS. In addition to a gene in an IgG H chain variable region of a BCR, the immune cell subpopulation (B cell, regulatory T cell, or the like) count in a subject can also be used as an indicator. The usage frequency of a gene can be determined by a method comprising large scale highly efficient BCR repertoire analysis.
Examples of embodiments of the present disclosure are specified in the following items.

(Item 1)

A method of using a B cell receptor (BCR) repertoire in a subject as an indicator of myalgic encephalomyelitis/chronic fatigue syndrome (ME/CFS) in the subject.

(Item 2)

The method of the preceding item, using one or more variables comprising a usage frequency of one or more genes in an IgG H chain variable region of a BCR in a subject as an indicator of myalgic encephalomyelitis/chronic fatigue syndrome (ME/CFS) in the subject.

(Item 2A)

The method of any of the preceding items, further characterized in that the one or more variables is an indicator of the subject suffering from ME/CFS, and not another disease.

(Item 3)

The method of any of the preceding items, wherein the one or more genes comprise at least one gene selected from the group consisting of IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81, IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27, IGHJ1, IGHJ2, IGHJ3, IGHJ4, IGHJ5, IGHJ6, IGHG1, IGHG2, IGHG3, IGHG4, and IGHGP.

(Item 3-1)

The method of any of the preceding items, wherein the one or more genes comprise at least one gene selected from the group consisting of IGHV3-73, IGHV1-69-2, IGHV5-51, IGHV4-31, IGHV3-23D, IGHV1/OR15-9, IGHV4-39, IGHD5-12, IGHV3-43D, IGHD4-17, IGHV5-10-1, IGHD4/OR15-4a/b, IGHG4, IGHV1/OR15-5, IGHV3/OR16-9, IGHD1-7, IGHV3-21, IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22.

(Item 3-2)

The method of any of the preceding items, wherein the one or more genes comprise at least one gene selected from the group consisting of IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22.

(Item 3-3)

The method of any of the preceding items, wherein the one or more genes comprise at least one gene selected from the group consisting of IGHV3-49, IGHV3-30-3, IGHD1-26, IGHJ6, IGHV3-30, IGHGP, IGHV1-3, and IGHD3-22.

(Item 3-4)

The method of any of the preceding items, wherein the one or more genes comprise at least one gene selected from the group consisting of IGHV1-3, IGHV3-30, IGHV3-30-3, IGHV3-49, IGHD1-26, and IGHJ6.

(Item 4-1)

The method of any of the preceding items, wherein the one or more variables further comprise one or more immune cell subpopulation counts.

(Item 4-2)

The method of any of the preceding items, wherein the one or more variables exhibit AUC $\geq$ 0.7 under a ROC curve in regression analysis for differentiating a normal control from ME/CFS.

(Item 4-3)

The method of any of the preceding items, wherein the one or more variables exhibit AUC $\geq$ 0.8 under a ROC curve in regression analysis for differentiating a normal control from ME/CFS.

(Item 4-4)

The method of any of the preceding items, wherein the immune cell subpopulation count is selected from a B cell count, a naive B cell count, a memory B cell count, a plasmablast count, an activated naive B cell count, a transitional B cell count, a regulatory T cell count, a memory T cell count, a follicular helper T cell count, a Tfh1 cell count, a Tfh2 cell count, a Tfh17 cell count, a Th1 cell count, a Th2 cell count, and a Th17 cell count.

(Item 5-1)

The method of any of the preceding items, wherein the one or more variables comprise usage frequencies of two or more genes in an IgG H chain variable region of a BCR in the subject.

(Item 5-2)

The method of any of the preceding items, wherein the one or more variables exhibit AUC $\geq$ 0.7 under a ROC curve in regression analysis for differentiating a normal control from ME/CFS.

(Item 5-2)

The method of any of the preceding items, wherein the one or more variables exhibit AUC $\geq$ 0.8 under a ROC curve in regression analysis for differentiating a normal control from ME/CFS.

(Item 5-2)

The method of any of the preceding items, wherein the one or more variables exhibit AUC $\geq$ 0.9 under a ROC curve in regression analysis for differentiating a normal control from ME/CFS.

(Item 6-1)

The method of any of the preceding items, wherein the one or more variables comprise a combination of two or more variables selected from the group consisting of a usage frequency of one or more genes in an IgG H chain variable region of a BCR in the subject, a BCR diversity index in the subject, and one or more immune cell subpopulation counts in the subject, and wherein the one or more variables exhibit AUC $\geq$ 0.7 under a ROC curve in regression analysis for differentiating a normal control from ME/CFS.

(Item 6-2)

The method of any of the preceding items, wherein the one or more variables comprise three of more variables selected from the group.

(Item 6-3)

The method of any of the preceding items, wherein the one or more variables comprise four of more variables selected from the group.

(Item 6-4)

The method of any of the preceding items, wherein the one or more variables comprise five of more variables selected from the group.

(Item 6-5)

The method of any of the preceding items, wherein the one or more variables comprise six of more variables selected from the group.

(Item 6-7)

The method of any of the preceding items, wherein the one or more variables exhibit AUC $\geq$ 0.8 under a ROC curve in regression analysis for differentiating a normal control from ME/CFS.

(Item 6-3)

The method of any of the preceding items, wherein the one or more variables exhibit AUC $\geq$ 0.9 under a ROC curve in regression analysis for differentiating a normal control from ME/CFS.

(Item 7)

The method of any of the preceding items, wherein the one or more genes comprise IGHV1-3, IGHV3-30, IGHV3-30-3, IGHV3-49, IGHD1-26, and IGHJ6.

(Item 8)

The method of any of the preceding items, wherein the one or more variables comprise a B cell count in the subject.

(Item 9)

The method of any of the preceding items, wherein the one or more variables comprise a regulatory T cell (Treg) count in the subject.

(Item 10)

The method of any of the preceding items, wherein the usage frequency of the one or more genes is determined by a method comprising large scale highly efficient BCR repertoire analysis.

(Item 11)

The method of any of the preceding items, wherein the one or more variables comprise a usage frequency of at least one gene selected from the group consisting of IGHV3-49, IGHV3-30-3, IGHD1-26, IGHV3-30, IGHJ6, IGHGP, IGHV4-31, IGHV3-64, IGHD3-22, IGHV3-33, IGHV3-73, IGHV5-10-1, and IGHV4-34.

(Item 12)

The method of any of the preceding items, comprising:

(a) using a part of the one or more variables as an indicator of ME/CFS in the subject; and
(b) using a part of the one or more variables as an indicator of the subject having ME/CFS, and not another disease.

(Item 13)

The method of any of the preceding items, wherein (b) is performed a plurality of times for a plurality of other diseases.

(Item 14)

The method of any of the preceding items, wherein the another disease comprises multiple sclerosis (MS).

(Item 15)

A method of using one or more variables comprising a usage frequency of one or more genes in an IgG H chain variable region of a BCR in a subject as an indicator of the subject suffering from myalgic encephalomyelitis/chronic fatigue syndrome (ME/CFS), and not multiple sclerosis (MS).

(Item 15A)

The method of the preceding item, comprising a feature according to one or more of the preceding items.

(Item 16)

The method of any of the preceding items, wherein the one or more genes comprise at least one gene selected from the group consisting of IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81, IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27, IGHJ1, IGHJ2, IGHJ3, IGHJ4, IGHJ5, IGHJ6, IGHG1, IGHG2, IGHG3, IGHG4, and IGHGP.

(Item 17)

The method of any of the preceding items, wherein the one or more genes comprise at least one gene selected from the group consisting of IGHV1-3, IGHV3-30, IGHV3-30-3, IGHD1-26, IGHV3-49, and IGHJ6.

(Item 18)

The method of any of the preceding items, wherein the one or more variables comprise usage frequencies of two or more genes in an IgG H chain variable region of a BCR in the subject.

(Item 19)

The method of any of the preceding items, wherein the one or more variables exhibit AUC ≥ 0.7 under a ROC curve in regression analysis for differentiating MS from ME/CFS.

(Item 20)

The method of any of the preceding items, wherein the one or more variables exhibit AUC ≥ 0.8 under a ROC curve in regression analysis for differentiating MS from ME/CFS.

(Item 21)

The method of any of the preceding items, wherein the one or more variables exhibit AUC ≥ 0.9 under a ROC curve in regression analysis for differentiating MS from ME/CFS.

(Item 22)

The method of any of the preceding items, wherein the one or more variables comprise usage frequencies of three or more genes in an IgG H chain variable region of a BCR in the subject.

(Item 23)

The method of any of the preceding items, wherein the one or more variables exhibit AUC ≥ 0.7 under a ROC curve in regression analysis for differentiating MS from ME/CFS.

(Item 24)

The method of any of the preceding items, wherein the one or more variables exhibit AUC ≥ 0.8 under a ROC curve in regression analysis for differentiating MS from ME/CFS.

(Item 25)

The method of any of the preceding items, wherein the one or more variables exhibit AUC ≥ 0.9 under a ROC curve in regression analysis for differentiating MS from ME/CFS.

(Item 26)

The method of any of the preceding items, wherein (b) is performed by the method of any of the preceding items.

(Item A1)

A method of diagnosing a subject as suffering from myalgic encephalomyelitis/chronic fatigue syndrome (ME/CFS), comprising: determining a B cell receptor (BCR) repertoire in a subject; and diagnosing the subject as suffering from myalgic encephalomyelitis/chronic fatigue syndrome (ME/CFS) based on the BCR repertoire.

(Item A1-1)

The method of the preceding item, comprising a feature according to one or more of the preceding items.

(Item A2)

A method of treating myalgic encephalomyelitis/chronic fatigue syndrome (ME/CFS), comprising: determining a B cell receptor (BCR) repertoire in a subject; diagnosing the subject as suffering from myalgic encephalomyelitis/chronic fatigue syndrome (ME/CFS) based on the BCR repertoire; and administering therapy on the subject.

(Item A2-1)

The method of the preceding item, comprising a feature according to one or more of the preceding items.

(Item A3)

The method of any of the preceding items for diagnosing the subject as suffering from myalgic encephalomyelitis/chronic fatigue syndrome (ME/CFS) based on one or more variables comprising a usage frequency of one or more genes in an IgG H chain variable region of a BCR in the subject.

(Item A4)

The method of any of the preceding items, wherein a value of a formula comprising the variables is computed for the subject, and the value is compared with a threshold value to diagnose the subject as suffering from myalgic encephalomyelitis/chronic fatigue syndrome (ME/CFS).

(Item A5)

The method of any of the preceding items, comprising:

(A) providing a plurality of variables comprising a usage frequency of one or more genes in an IgG H chain variable region of a BCR with regard to myalgic encephalomyelitis/chronic fatigue syndrome (ME/CFS);
(B) providing a differentiation formula generated by multivariate analysis for the variables;
(C) computing a probability of suffering from ME/CFS by fitting values of the variables of the subject into the differentiation formula; and
(D) determining the subject as suffering from ME/CFS if the probability of suffering from ME/CFS is greater than a predetermined value.

(Item A6)

The method of any of the preceding items, comprising:

(A) providing a plurality of variables comprising a usage frequency of one or more genes in an IgG H chain variable region of a BCR with regard to myalgic encephalomyelitis/chronic fatigue syndrome (ME/CFS);
(B) providing a differentiation formula generated by multivariate analysis for the variables, (B) comprising:

(B-1) performing univariate or multivariate logistic regression for the variables, with a distinction between patient/healthy individual as a response variable;
(B-2) computing a constant and a coefficient of a differentiation formula from a constant and a partial regression coefficient of a logit model formula generated in the logistic regression; and
(B-3) generating a differentiation formula based on the constant and the coefficient obtained from processing of B-2;

(C) computing a probability of suffering from ME/CFS by fitting values of the variables of the subject into the differentiation formula; and
(D) determining the subject as suffering from ME/CFS if the probability of suffering from ME/CFS is greater than a predetermined value.

(Item A6-A)

The method of any of the preceding items, comprising:

(A) specifying a combination of variables, (A) comprising:

(A-1) comparing a healthy individual with a myalgic encephalomyelitis/chronic fatigue syndrome (ME/CFS) patient and providing a plurality of variables comprising a usage frequency of one or more genes in an IgG H chain variable region of a BCR for which a significant difference is detected, for subjects including the ME/CFS patient and the healthy individual; and/or

(A-2) performing univariate logistic analysis using a gene in an IgG H chain variable region of the BCR as an independent variable or multivariate logistic analysis using two or more genes in an IgG H chain variable region of the BCR as independent variables to obtain a logistic regression model formula, and performing ROC analysis for measuring a degree of fit of the logistic regression model formula to select a gene exhibiting an AUC value equal to or greater than a predetermined value as a variable for a differentiation formula;

(B) providing a differentiation formula generated by multivariate analysis for the variables for the differentiation formula, (B) comprising:

(B-1) performing univariate or multivariate logistic regression for the variables for the differentiation formula, with a distinction between patient/healthy individual as a response variable;

(B-2) computing a constant and a coefficient of a differentiation formula from a constant and a partial regression coefficient of a logit model formula generated in the logistic regression of B-1; and

(B-3) generating a differentiation formula based on the constant and the coefficient obtained from processing of B-2;

(C) computing a probability of suffering from ME/CFS by fitting values of the variables of the subject into the differentiation formula; and

(D) determining the subject as suffering from ME/CFS if the probability of suffering from ME/CFS is greater than a predetermined value.

(Item A7)

The method of any of the preceding items, comprising:

(A) providing a plurality of variables comprising a usage frequency of one or more genes in an IgG H chain variable region of a BCR with regard to myalgic encephalomyelitis/chronic fatigue syndrome (ME/CFS);

(B) providing a differentiation formula generated by multivariate analysis for the variables;

(C) computing a probability of suffering from ME/CFS by fitting values of the variables of the subject into the differentiation formula;

(AA) providing a plurality of variables comprising a usage frequency of one or more genes in an IgG H chain variable region of a BCR for a disease other than myalgic encephalomyelitis/chronic fatigue syndrome (ME/CFS);

(BB) providing a differentiation formula generated by multivariate analysis for the variables;

(CC) computing a probability of suffering from a disease other than ME/CFS by fitting values of the variables of the subject into the differentiation formula; and

(D) determining the subject as suffering from ME/CFS if the probability of suffering from ME/CFS is greater than a predetermined value and the probability of suffering from a disease other than ME/CFS is lower than a predetermined value.

(Item A8)

An in vitro method of diagnosing a subject as suffering from myalgic encephalomyelitis/chronic fatigue syndrome (ME/CFS), comprising diagnosing the subject as suffering from myalgic encephalomyelitis/chronic fatigue syndrome (ME/CFS) based on a B cell receptor (BCR) repertoire in a subject.

(Item A8-1)

The method of the preceding item, comprising a feature according to one or more of the preceding items.

(Item B1)

A program comprising an instruction which, when executed by one or more processors, causes the processors to obtain one or more variables comprising a variable associated with a BCR repertoire of a subject and to determine whether the subject has ME/CFS based on the one or more variables.

(Item B1-1)

The program of the preceding item, comprising a feature according to one or more of the preceding items.

(Item B2)

A storage medium for recording a program comprising an instruction which, when executed by one or more processors, causes the processors to obtain one or more variables comprising a variable associated with a BCR repertoire of a subject and to determine whether the subject has ME/CFS based on the one or more variables.

(Item B2-1)

The storage medium of the preceding item, comprising a feature according to one or more of the preceding items.

(Item B2-2)

The storage medium of any of the preceding items, which is a non-transitory storage medium.

(Item C1)

A system comprising a recording unit configured to record information on one or more variables comprising a variable associated with a BCR repertoire of a subject and a determination unit configured to obtain the information and determine whether the subject has ME/CFS

(Item C1-1)

The system of preceding item, comprising a feature according to one or more of the preceding items.

[Advantageous Effects of Invention]

Myalgic encephalomyelitis/chronic fatigue syndrome (ME/CFS) can be accurately diagnosed with the present disclosure. The present disclosure can also be utilized in prediction of development of ME/CFS and prognostic diagnosis thereof. The present disclosure can also be utilized as a marker in the development of a therapeutic agent for ME/CFS.

[Brief Description of Drawings]

[Figure 1] Figure **1** is a diagram showing a result of comparing usage frequencies of each IGHV gene (IGHV family) of a BCR obtained from samples of ME/CFS patients and healthy controls. The vertical axis indicates the usage frequency (%) of each gene, and the bar indicates the standard error. HC: healthy control, ME/CFS: ME/CFS patient.
[Figure 2] Figure **2** is a diagram showing a result of comparing usage frequencies of each of the IGHD and IGHJ families of a BCR obtained from samples of ME/CFS patients and healthy controls. The vertical axis indicates the usage frequency (%) of each gene, and the bar indicates the standard error. HC: healthy control, ME/CFS: ME/CFS patient.
[Figure 3A] Figure **3A** is a dot plot of the usage frequency of the described IGHV gene of samples of ME/CFS patients and healthy controls. HC: healthy control, ME/CFS: ME/CFS patient.
[Figure 3B] Figure **3A** is a dot plot of the usage frequency of the described IGHV gene of samples of ME/CFS patients and healthy controls. HC: healthy control, ME/CFS: ME/CFS patient.
[Figure 4] Figure **4** is a dot plot of the described B cell subpopulation count of samples of ME/CFS patients and healthy controls. HC: healthy control, ME/CFS: ME/CFS patient.
[Figure 5] Figure **5** is a dot plot of the regulatory T cell population count of samples of ME/CFS patients and healthy controls. HC: healthy control, ME/CFS: ME/CFS patient.
[Figure 6] Figure **6** is a dot plot of the described T cell population count of samples of ME/CFS patients and healthy controls. HC: healthy control, ME/CFS: ME/CFS patient.
[Figure 7] Figure **7** is a diagram showing the usage frequencies of IGHV1-3, IGHV3-30, IGHV3-30-3, IGHV3-49, IGHD1-26, and IGHJ6 and an ROC curve in regression analysis when B cell count (%) is used as a variable.
[Figure 8] Figure **8** is a diagram showing the usage frequencies of IGHV1-3, IGHV3-30, IGHV3-30-3, IGHV3-49, IGHD1-26, and IGHJ6 and an ROC curve in regression analysis when Treg count (%) is used as a variable.
[Figure 9] Figure **9** is a diagram showing the difference in usage frequencies of IGH genes between ME/CFS patients (n = 37) and MS patients (n = 10). ME/CFS: ME/CFS patient, MS: MS patient.
[Figure 10] Figure **10** is a diagram showing the difference in diversity indices between ME/CFS patients (n = 37) and MS patients (n = 10). ME/CFS: ME/CFS patient, MS: MS patient. A significant difference was not detected between ME/CFS patients (n = 37) and MS patients (n = 10) for any of the diversity indices.
[Figure 11] Figure **11** is a diagram showing the difference in usage frequencies of IGH genes between ME/CFS patients (n = 37) and non-ME/CFS patients (n = 33) . ME/CFS: ME/CFS patient, non-ME/CFS: healthy controls + MS patients.

[Description of Embodiments]

The present disclosure is explained hereinafter while showing the best mode of the disclosure. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.
The definitions of the terms and/or the detailed basic technology that are particularly used herein are described hereinafter as appropriate.

(ME/CFS)

Myalgic encephalomyelitis/chronic fatigue syndrome (ME/CFS) is a severe chronic disease with unexplained high level of fatigue, inordinate exhaustion after exertion, sleep disorder, or cognitive dysfunction that lasts 6 months or longer as the core symptom and often involving orthostatic intolerance, pain, digestive symptom, hypersensitivity to light, sound, odor, or chemical substance, or the like. While the number of patients is estimated to be 100000 or more domestically, the pathology is still not elucidated with no disease-specific testing method or effective therapeutic method, so that suitable medical treatment is currently unavailable.
In view of the recent report from Norway of the effectiveness of B cell depletion therapy using rituximab, the immunological pathology of ME/CFS has drawn global attention. Among the acquired immune systems, B cells are responsible for antibody production, but B cell receptors are very diverse due to gene rearrangement in order to respond to diverse antigens. A specific B cell receptor (clone) is elevated in autoimmune diseases such as systemic erythematodes or hematological tumor of B cell system and is detected as a bias in the entire collection of diverse B cell receptors of an individual (repertoire). Use of a next generation sequencer can enable repertoire analysis with a method eliminating biases and is potentially useful in the detection of abnormalities in a B cell system of ME/CFS.
ME/CFS is diagnosed from a symptom/medical interview in accordance with criteria such as Fukuda criteria, Canadian criteria, or International consensus criteria. A biomarker indicating the disease has not been developed yet for ME/CFS. There are a plurality of diagnostic criteria for diagnosis using a combination of symptoms. The Fukuda criteria is the oldest (1994), and then the Canadian criteria (2003) and subsequently the International criteria (2011) were created. In the meantime, however, the common understanding of experts on the fundamental symptom of the disease (core symptom) has advanced, such that creation of new diagnostic criteria is currently being considered. The reasons why multiple diagnostic criteria including old criteria co-exist are understood to be the need for both 1) detailed/strict criteria (research criteria) for promoting research and advancing understanding of the pathology of the disease/development of a therapeutic method, and 2) simple criteria (diagnostic criteria) for a physician to render a diagnosis and promote medical/social intervention to a patient. Specifically, the Fukuda criteria is still considered essential today for research as the research criteria, while newer criteria are meant for diagnosis. Such a double standard is generally unacceptable for other diseases, but is recognized/accepted among experts as an exception for ME/CFS. The diagnostic criteria prepared by the Japanese Ministry of Health, Labour and Welfare are based on international criteria that are adjusted for domestic practice (PS setting and the like). As used herein, ME-CFS can refer to a disease specified by research criteria, the Fukuda criteria, but can also reference diseases defined by other diagnostic criteria. ME/CFS can be confirmed herein by any of the diagnostic criteria accepted in the art.

In the present disclosure, a subject shown to have ME/CFS, subject shown to possibly have ME/CFS, a subject shown to have the possibility of developing ME/CFS, or a subject shown to be suffering from ME/CFS with poor prognosis can be suitably treated. ME/CFS treatment can used an immunomodulator (rituximab or the like), nonsteroidal antiinflammatory agent, antidepressant, anxiolytic, or the like.

(BCR repertoire)

In one embodiment, the present disclosure provides a method of using a B cell receptor (BCR) repertoire as an indicator of myalgic encephalomyelitis/chronic fatigue syndrome (ME/CFS) in a subject. As used herein, "B cell receptor (BCR)" is also called a B cell antigen receptor, referring to those comprised of Igα/Igβ (CD79a/CD79b) heterodimer (α/β) associated with a membrane bound immunoglobulin (mIg) molecule. An mIg subunit binds to an antigen to induce aggregation of receptors, while an α/β subunit transmits a signal toward the cell. Aggregation of BCRs is understood to quickly activate Lyn, Blk, and Fyn of an Src family kinase in the same manner as Syk and Btk of tyrosine kinase. Many different results are produced depending on the complexity of BCR signaling. Examples thereof include survival, resistance (anergy; lack of hypersensitive reaction to an antigen) or apoptosis, cell division, differentiation into an antibody producing cell or memory B cell, and the like. Hundreds of millions of types of T cells with different TCR variable region sequences are produced, and hundreds of millions of types of B cells with different BCR (or antibody) variable region sequences are produced. Since the individual sequences of TCRs and BCRs vary due to rearrangement or mutation of the genomic sequence, a clue for antigen specificity of a T cell or B cell can be found by determining the TCR/BCR genomic sequence or the mRNA (cDNA) sequence.

As used herein, "V region" refers to a variable (V) region of a variable region of a TCR chain or BCR chain.

As used herein, "D region" refers to a D region of a variable region of a TCR chain or BCR chain.

As used herein, "J region" refers to a J region of a variable region of a TCR chain or BCR chain.

As used herein, "C region" refers to a constant (C) region of a TCR chain or BCR chain.

As used herein, "repertoire of a variable region" refers to a collection of V(D)J regions optionally created by gene rearrangement in TCR or BCR. The phrases TCR repertoire, BCR repertoire and the like are used, but they can also be called, for example, a T cell repertoire, B cell repertoire, or the like. A repertoire can be considered as having two aspects, i.e., information on the degree of diversity as a whole and information on how frequently each gene is used. One embodiment provides a method of using one or more variables comprising a usage frequency of one or more genes in an IgG H chain variable region of a BCR in a subject as an indicator of myalgic encephalomyelitis/chronic fatigue syndrome (ME/CFS) in the subject. The usage frequency of one or more genes in an IgG H chain variable region of a BCR can be derived as follows.

An example of a method of determining a BCR repertoire is a method of analyzing the ratio of T cells expressing individual Vβ chains by flow cytometry using a specific Vβ chain specific antibody for how much of individual V chains is used by a B cell in a sample (FACS analysis). TCR repertoire analysis through a molecular biological approach has been conceived based on information on a TCR gene obtained from a human genome sequence. This includes a method of extracting RNA from a cell sample and synthesizing a complementary DNA, and then subjecting a TCR gene to PCR amplification for quantification.

A nucleic acid can be extracted from a cell sample by using a tool that is known in the art such as RNeasy Plus Universal Mini Kit (QIAGEN). Total RNA can be extracted and purified from a cell dissolved in a TRIzol LS reagent by using an RNeasy Plus Universal Mini Kit (QIAGEN). A complementary DNA can be synthesized from an extracted RNA by using any reverse transcriptase known in the art such as Superscript III™ (Invitrogen).

Those skilled in the art can appropriately perform PCR amplification of a BCR gene using any polymerase known in the art. However, an "unbiased" amplification of a gene with large variation such as a BCR gene can result in an advantageous effect for accurate measurement.

A method of designing numerous individual BCR V chain specific primers as primers used for PCR amplification and quantifying each separately by real-time PCR or the like, or a method of concurrently amplifying such specific primers (Multiple PCR) has been used. However, even for quantification of each V chain using an endogenous control, an accurate analysis cannot be performed if many primers are used. Furthermore, Multiple PCR has a disadvantage in that a difference in amplification efficiencies among primers leads to a bias during PCR amplification.

A preferred embodiment of the present disclosure determines BCR diversity by amplifying, without changing the frequency of presence, BCR genes including all isotype and subtype genes with one set of primers consisting of one type of forward primer and one type of reverse primer as described in WO 2015/075939 (Repertoire Genesis Inc.). The following primer design is advantageous for unbiased amplification.

Focus was placed on the genetic structure of a BCR gene. An adaptor sequence is added, without setting a primer to a V region with a high degree of diversity, to the 5' terminus thereof to amplify all V region containing genes.

Such an adaptor can have any length or sequence in a base sequence. About 20 base pairs are optimal, but a sequence from 10 bases to 100 bases can be used. An adaptor added to the 3' terminus is removed with a restriction enzyme, and all BCR genes are amplified by amplification with a reverse primer specific to a C region which has a common sequence with an adaptor primer with the same sequence as a 20 base pair adaptor.

A complementary strand DNA is synthesized with a reverse transcriptase from a BCR gene messenger RNA and then a double stranded complementary DNA is synthesized. A double stranded complementary DNA comprising V regions with different lengths is synthesized by a reverse transcription reaction or a double strand synthesizing reaction. Adaptors consisting of 20 base pairs and 10 base pairs are added to the 5' terminal section of such genes by a DNA ligase reaction.

The genes can be amplified by setting a reverse primer to a C region of a heavy chain, i.e., $\mu$ chain, $\alpha$ chain, $\delta$ chain, $\gamma$ chain, or $\epsilon$ chain, or a light chain, i.e., $\kappa$ chain or $\lambda$ chain of BCRs. As reverse primers set in a C region, primers are set which match the sequences of each of C$\mu$, C$\alpha$, C$\delta$, C$\gamma$, C$\epsilon$, C$\kappa$, and C$\lambda$ and have a mismatch to an extent that other C region sequences are not primed for BCRs. A reverse primer of a C region is optimally produced while considering the base sequence, base composition, DNA melting temperature (Tm), or presence/absence of a self-complementary sequence, so that amplification with an adaptor primer is possible. A primer can be set in a region other than the base sequence that is different among allelic sequences in a C region sequence to uniformly amplify all alleles. Nested PCR with a plurality of stages is performed in order to enhance the specificity of an amplification reaction.

While the length (number of bases) of a primer candidate sequence is not particularly limited for a sequence not comprising a sequence that is different among allelic sequences for each primer, the number of bases is 10 to 100, preferably 15 to 50, and more preferably 20 to 30. Use of such unbiased amplification is advantageous and preferred for identification of a low frequency (1/10,000 to 1/100,000 or less) gene.

A BCR repertoire can be determined from read data that is obtained by sequencing a BCR gene amplified in the above manner.

The sequencing approach is not limited, as long as a sequence of a nucleic acid sample can be determined. While any approach known in the art can be utilized, it is preferable to use next generation sequencing (NGS). Examples of next generation sequencing include, but are not limited to, pyrosequencing, sequencing by synthesis, sequencing by ligation, ion semiconductor sequencing, and the like.

The obtained read data can be mapped to a reference sequence comprising V, D, and J genes to derive the number of unique reads and determine the BCR repertoire.

One embodiment prepares a reference database to be used for each of V, D, J, and C gene regions. Typically, a nucleic acid sequence data set for each allele or each region published by the IMGT is used, but the data set is not limited thereto. Any data set with a unique ID assigned to each sequence can be used.

The obtained read data (including those subjected to appropriate processing such as trimming as needed) is used as the input sequence set to search for homology with a reference database for each gene region, and an alignment with the closest reference allele and the sequence thereof are recorded. In this regard, an algorithm with high tolerance for a mismatch except for C is used for homology search. When a common homology search program BLAST is used, shortening of the window size, reduction in mismatch penalty, and reduction in gap penalty are set for each region. The closest reference allele is selected by using a homology score, alignment length, kernel length (length of consecutively matching base sequence), and number of matching bases as indicators, which are applied in accordance with a defined order or priority. For an input sequence with determined V and J used in the present disclosure, a CDR3 sequence is extracted with the front of CDR3 on reference V and end of CDR3 on reference J as guides. This is translated into an amino acid sequence for use in classification of a D region. When a reference database of a D region is prepared, a combination of results of homology search and results of amino acid sequence translation is used as a classification result.

In view of the above, each allele of V, D, J, and C is assigned for each sequence in an input set. The frequency of appearance by each of V, D, J, and C or frequency of appearance of a combination thereof is subsequently calculated in the entire input set to derive a BCR repertoire. The frequency of appearance is calculated in a unit of allele or unit of gene name depending on the precision required in classification. The latter is made possible by translating each allele into a gene name.

After a V region, D region, J region, and C region are assigned to read data, matching reads can be tallied to calculate the number of reads detected in a sample and the ratio to the total number of reads (frequency) for each unique read (read without the same sequence).

In addition, a diversity index or similarly index can be computed with a statistical analysis software such as ESTIMATES or R (vegan) by using data such as number of samples, read type, or the number of reads. In a preferred embodiment, TCR repertoire analysis software (Repertoire Genesis Inc.) is used.

As used herein, "BCR diversity" refers to diversity of the repertoire of B cell receptors of a subject. Those skilled in the art can determine the BCR diversity using various means known in the art. Any BCR diversity index that is known in the art can be used. Examples of BCR diversity indices include Shannon-Weaver index, Simpson index, inverse Simpson index, Pielou's evenness index, normalized Shannon-Weaver index, DE index (e.g., DE50 index, DE30 index, or DE80 index), or Unique index (e.g., Unique50 index, Unique30 index, or Unique80 index) applied to BCRs.

(Large-scale high efficiency BCR repertoire analysis)

A preferred embodiment of the present disclosure determines the BCR diversity using large-scale high efficiency BCR repertoire analysis. "Large-scale high efficiency repertoire analysis" herein is described in WO 2015/075939 (the entire disclosure thereof is incorporated herein by reference as needed) and is referred to as "large-scale high efficiency BCR repertoire analysis" when targeting BCRs. Large-scale high efficiency repertoire analysis is a method of quantitatively analyzing a repertoire (variable region of a T cell receptor (TCR) or B cell receptor (BCR)) of a subject by using a database. This method can be materialized by a method of quantitatively analyzing a repertoire of a variable region of a T cell receptor (TCR) or B cell receptor (BCR) by using a database, the method comprising (1) providing a nucleic acid sample comprising a nucleic acid sequence of the T cell receptor (TCR) or the B cell receptor (BCR) which is amplified from the subject in an unbiased manner; (2) determining the nucleic acid sequence comprised in the nucleic acid sample; and (3) computing a frequency of appearance of each gene or a combination thereof based on the determined nucleic acid sequence to derive a TCR or BCR repertoire of the subject, wherein the nucleic acid sample comprises nucleic acid sequences of a plurality of types of T cell receptor (TCR) or B cell receptor (BCR), and wherein (2) determines the nucleic acid sequence by single sequencing using a common adaptor primer. This method preferably comprises (1) providing a nucleic acid sample comprising a nucleic acid sequence of a T cell receptor (TCR) or a B cell receptor (BCR) which is amplified from the subject in an unbiased manner; (2) determining the nucleic acid sequence comprised in the nucleic acid sample; and (3) computing a frequency of appearance of each gene or a combination thereof based on the determined nucleic acid sequence to derive a repertoire of the subject, (1) comprising: (1-1) synthesizing a complementary DNA by using an RNA sample derived from a target cell as a template; (1-2) synthesizing a double stranded complementary DNA by using the complementary DNA as a template; (1-3) synthesizing an adaptor-added double stranded complementary DNA by adding a common adaptor primer sequence to the double stranded complementary DNA; (1-4) performing a first PCR amplification reaction by using the adaptor-added double stranded complementary DNA, a common adaptor primer consisting of the common adaptor primer sequence, and a first TCR or BCR C region specific primer, wherein the first TCR or BCR C region specific primer is designed to comprise a sequence that is sufficiently specific to a C region of interest of the TCR or BCR and not homologous to other genetic sequences, and comprise a mismatching base between subtypes downstream when amplified; (1-5) performing a second PCR amplification reaction by using a PCR amplicon of (1-4), the common adaptor primer, and a second TCR or BCR C region specific primer, wherein the second TCR or BCR C region specific primer is designed to have a sequence that is a complete match with the TCR or BCR C region in a sequence downstream the sequence of the first TCR C region specific primer, but comprise a sequence that is not homologous to other genetic sequences, and comprise a mismatching base between subtypes downstream when amplified; and (1-6) performing a third PCR amplification reaction by using a PCR amplicon of (1-5), an added common adaptor primer in which a nucleic acid sequence of the common adaptor primer comprises a first additional adaptor nucleic acid sequence, and an adaptor-added third TCR C region specific primer in which a second additional adaptor nucleic acid sequence and a molecule identification (MID Tag) sequence are added to a third TCR or BCR C region specific sequence; wherein the third TCR C region specific primer is designed to have a sequence that is a complete match with the TCR or BCR C region in a sequence downstream to the sequence of the second TCR or BCR C region specific primer, but comprise a sequence that is not homologous to other genetic sequences, and comprise a mismatching base between subtypes downstream when amplified, the first additional adaptor nucleic acid sequence is a sequence that is suitable for binding to a DNA capturing bead and for an emPCR reaction, the second additional adaptor nucleic acid sequence is a sequence that is suitable for an emPCR reaction, and the molecule identification (MID Tag) sequence is a sequence for imparting uniqueness such that an amplicon can be identified. The specific detail of this method is described in WO 2015/075939. Those skilled in the art can perform analysis by appropriately referring to this document, the Examples of the present specification, and the like.

(Diagnosis)

One embodiment of the present disclosure provides a method of using a B cell receptor (BCR) repertoire in a subject as an indicator of myalgic encephalomyelitis/chronic fatigue syndrome (ME/CFS) in the subject. This method can comprise providing prognostic diagnosis or prediction on the development or ME/CFS, or an indicator for the development of a therapeutic agent for ME/CFS, in addition to diagnosis of a subject developing ME/CFS diagnosis. This can be an in vitro or in silico method.

The method can comprise using one or more variables comprising a variable associated with a BCR repertoire. If one or more variables are used as an indicator of ME/CFS, the method can be performed, for example, by computing a value of a suitable formula comprising the one or more variables by using the formula for a subject, comparing the value with a suitable criteria, and the like. The formula can be found by logistic regression or the like. Examples of variables used include, but are not limited to, one or more variables selected from a usage frequency of one or more genes in an IgG H chain variable region of a BCR in the subject, a BCR diversity index in the subject, and one or more immune cell subpopulation counts in the subject, and a combination of two or more variables.

An embodiment of the present disclosure can preferably use one or more variables comprising a usage frequency of one or more IGH genes of a BCR in a subject as an indicator of ME/CFS. This can enable use of the one or more variables as an indicator of a subject suffering from ME/CFS and not another disease (i.e., differential diagnosis). Although not wishing to be bound by any theory, the immune cell subpopulation count and BCR repertoire diversity index can also vary due to another disease that affects the entire immunological state, so that the variables can suggest the presence of ME/CFS in a subject, but may not be able to rule out other diseases. Meanwhile, the usage frequency of an IGH gene is understood to vary by reflecting the mechanism of some type of disease, so that the usage frequency can be an indicator of a subject suffering from ME/CFS and not another disease. Therefore, the usage frequency is understood to be very useful for diagnosis in actual clinical settings.

Examples of "another disease" with respect to ME/CFS include any disease that can affect the immunological status in addition to any disease that exhibits a symptom similar to "unexplained high level of fatigue, inordinate exhaustion after exertion, sleep disorder, or cognitive dysfunction that lasts 6 months or longer as the core symptom and often involving orthostatic intolerance, pain, digestive symptom, hypersensitivity to light, sound, odor, or chemical substance, or the like", which is a symptom of ME/CFS. Examples thereof include psychiatric disorders (e.g., depression, adjustment disorder, and somatoform disorder), primary sleep disorders (e.g., sleep apnea and narcolepsy), endocrine diseases (e.g., hypopituitarism and thyroid disease), infectious diseases (e.g., AIDS, hepatitis B, hepatitis C, and other chronic infectious diseases), autoimmune diseases (e.g., rheumatoid arthritis, systemic erythematodes, Sjogren's syndrome, and the like), inflammatory diseases (e.g., inflammatory bowel disease, chronic pancreatitis, and other chronic inflammatory diseases), and nervous system diseases (e.g., multiple sclerosis (MS) and autoimmune encephalitis). An embodiments of the present disclosure provides a method of distinguishing ME/CFS from another disease in a subject. Multiple sclerosis (MS) is an example of anther disease. The description regarding a method of using a B cell receptor (BCR) repertoire in a subject as an indicator of myalgic encephalomyelitis/chronic fatigue syndrome (ME/CFS) in the subject can be applied as need to carry out the method of distinguishing ME/CFS from anther disease herein.

ME/CFS is diagnosed with a "combination of clinical symptoms", in which a process of ruling out other diseases and causes is very critical. The "criteria for a combination of clinical symptoms" can also be satisfied in some cases by lifestyle habits (inconsistent schedule, severe exertion, etc.). The criteria for a clinical symptom can also be satisfied by other causes such as malignant tumor, metabolic disease, cranial nerve disease, or the like. For this reason, a B cell repertoire, which is understood to reflect the cause of a disease, and usage frequency of each gene therein are considered very useful in clinical diagnosis as shown in the Examples herein. For example, more accurate differential diagnosis can be provided by adding the differentiation method using a BCR repertoire (IGH gene usage frequency) described herein. Alternatively, clinical information (clinical observation, MRI image observation, cerebrospinal fluid observation, or the like) can be used in combination with the method of the disclosure as needed. One embodiment can ultimately identify one disease by determining the possibility of a patient, who is exhibiting a systemic symptom including ME/CFS, suffering from each disease with a differentiation model or differentiation formula using a usage frequency of each IGH gene.

As used herein, "sensitivity" refers to the probability of correctly determining what should be determined as positive as positive, with high sensitivity related to reduced false-positive. High sensitivity is useful in rule-out diagnosis.

As used herein, "specificity" refers to the probability of correctly determining what is negative as negative, with high specificity related to reduced false-positive. High sensitivity is useful in rule-in diagnosis.

As used herein, "ROC curve" refers to a curve plotting (sensitivity) and (1-specificity) when a cutoff value based on a regression formula of a marker is varied as a parameter. Those skilled in the art can appropriately find the AUC (area under the curve) under a ROC curve. It is understood that the AUC of a ROC curve indicates the performance of a prediction model. The Examples herein demonstrate a large number of variables or combinations of variables exhibiting high predictive performance of AUC $\geq$ 0.7, AUC $\geq$ 0.8, or AUC $\geq$ 0.9 under a ROC curve in regression analysis. It is understood that a diagnostic model with an AUC of about 7 can be used.

Diagnosis of ME/CFS, which has been challenging up to this point, is enabled by use of one or more variables (can be a combination of variables as needed) used as an indicator of ME/CFS in a subject, exhibiting AUC $\geq$ 0.7 under a ROC curve in regression analysis.

The method can comprise obtaining one or more variables used as an indicator of ME/CFS in a subject. In one embodiment, the step of obtaining a variable can comprise analyzing a sample of a subject such as determining a repertoire of a BCR in a subject and/or measuring the cell subpopulation count in a subject. The step of determining a repertoire of a BCR can comprise determining a BCR diversity in a subject and/or determining a usage frequency of one or more genes in an IgG H chain variable region of a BCR of a subject. The cell subpopulation count in a subject can be measured by any methodology that is known to those skilled in the art including flow cytometry. It is also possible to obtain data for a value determined for a subject previously.

The method can comprise determining whether a subject has ME/CFS based on one or more variables described herein. The determining step can be performed by comparing the values of dependent variables of a function including the one or more variables with a suitable threshold value.

In one embodiment, the method described herein is performed in silico. The method comprises obtaining one or more variables comprising a variable associated with a BCR repertoire of a subject and determining whether the subject has ME/CFS based on the one or more variables. Programs, storage media for recording a program, and systems implementing any method described herein are also within the scope of the disclosure.

One embodiment provides a program comprising an instruction which, when executed by one or more processors, causes the processors to obtain one or more variables comprising a variable associated with a BCR repertoire of a subject and to determine whether the subject has ME/CFS based on the one or more variables, or a storage medium for recording the program. A system comprising a recording unit configured to record information on one or more variables comprising a variable associated with a BCR repertoire of a subject and a determination unit configured to obtain said information and determine whether the subject has ME/CFS is also provided. The system can be a computer, and can comprise a program described herein or a storage medium for recording the program as needed.

(Indicator)

(IGH gene)

One embodiment of the present disclosure provides a method of using one or more variables comprising a usage frequency of one or more genes (IGH gene) in an IgG H chain variable region of a BCR in a subject as an indicator of ME/CFS in the subject. As an IGH gene, at least one gene selected from the group consisting of IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81, IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27, IGHJ1, IGHJ2, IGHJ3, IGHJ4, IGHJ5, IGHJ6, IGHG1, IGHG2, IGHG3, IGHG4, and IGHGP, and any combination thereof can be used.

The Examples herein show that usage frequencies of various IGH genes can be an indicator of ME/CFS. In the present disclosure, the number of at least one IGH genes that are used is not particularly limited. Any number of genes from 1 to 117 genes can be used. One or more variables used as an indicator in the present disclosure can comprise a usage frequency of about 1, about 2, about 3, about 4, about 5, about 6, about 7, about 8, about 9, about 10, about 15, about 20, about 30, about 40, about 50, about 60, about 70, about 80, about 90, about 100, about 110, or a greater number of IGH genes. When suitable, one or more variables can also be one variable.

In a preferred embodiment, one or more IGH genes comprise at least one gene selected from the group consisting of IGHV3-73, IGHV1-69-2, IGHV5-51, IGHV4-31, IGHV3-23D, IGHV1/OR15-9, IGHV4-39, IGHD5-12, IGHV3-43D, IGHD4-17, IGHV5-10-1, IGHD4/OR15-4a/b, IGHG4, IGHV1/OR15-5, IGHV3/OR16-9, IGHD1-7, IGHV3-21, IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22. More preferably, one or more IGH genes comprise at least one gene selected from the group consisting of IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22. Still more preferably, one or more IGH genes comprise at least one gene selected from the group consisting of IGHV3-49, IGHV3-30-3, IGHD1-26, IGHJ6, IGHV3-30, IGHGP, IGHV1-3, and IGHD3-22. In another embodiment, one or more genes comprise at least one gene selected from the group consisting of IGHV1-3, IGHV3-30, IGHV3-30-3, IGHV3-49, IGHD1-26, and IGHJ6. The Examples herein suggest that the aforementioned genes can be used alone for prediction.

Another embodiment of the present disclosure provides a method using variables comprising usage frequencies of two or more genes in an IgG H chain variable region of a BCR in a subject as one or more variables. It is understood that the precision of the method can be further enhanced by using usage frequencies of two more genes. Those skilled in the art can select and use a combination of usage frequencies of two or more genes that are suitable in view of the descriptions herein. One or more variables can be selected so that AUC ≥ 0.7, AUC ≥ 0.8, or AUC ≥ 0.9 is exhibited under a ROC curve in regression analysis. An example of a combination of genes include IGHV1-3, IGHV3-30, IGHV3-30-3, IGHV3-49, IGHD1-26, and IGHJ6. Those skilled in the art can appropriately compute AUC under a ROC curve in regression analysis for a combination of genes in accordance with the method described herein, and determine whether the combination of genes exhibits a desired AUC. For example, regression analysis can be for differentiating a normal control from ME/CFS.

Examples herein show, for a combination of two IGH genes, that any of the 117 genes can materialize a combination exhibiting AUC $\geq$ 0.7 under a ROC curve in regression analysis when combined with another suitable IGH gene.

(Diversity index)

The present disclosure can use a BCR diversity index in a subject as a variable, in place of or in addition to another variable in diagnosis of myalgic encephalomyelitis/chronic fatigue syndrome (ME/CFS) in a subject.

Any index that is known in the art can be used as a BCR diversity index. Examples thereof include the Shannon-Weaver index, Simpson index, inverse Simpson index, Pielou's evenness index, normalized Shannon-Weaver index, DE index (e.g., DE50 index, DE30 index, and DE80 index), and the like.

In Example 3-2 and Table 7 herein, some diversity indices are extracted with significance of $0.1 \leq P < 0.2$ in simple regression analysis. Example 6 herein has found that some of the multiple combinations of variables comprising a diversity index exhibits an AUC value of 0.8 or greater in ROC analysis (Table 18).

(Cell subpopulation)

The present disclosure can use a cell subpopulation count as a variable in place of or in addition to another variable in the diagnosis of myalgic encephalomyelitis/chronic fatigue syndrome (ME/CFS) in a subject. For example, an immune cell subpopulation count can be used as a cell subpopulation count. As used herein, "cell subpopulation" refers to any population of cells having some type of a common feature among a cell population comprising cells with diverse characteristics. For a cell subpopulation with a specific name that is known in the art, the cell subpopulation can be mentioned using such a term. A specific cell subpopulation can also be mentioned by describing any property (e.g., expression of cell surface marker).

Examples of cell subpopulations include, but are not limited to, B cells, naive B cells, memory B cells, plasmablasts, activated naive B cells, transitional B cells, regulatory T cells, memory T cells, follicular helper T cells, Tfh1 cells, Tfh2 cells, Tfh17 cells, Th1 cells, Th2 cells, and Th17 cells.

The cell subpopulation count can be determined, for example, by flow cytometry by those skilled in the art. For example, the count can be observed by collecting a sample, reacting the sample with a fluorescently labeled antibody (an antibody to antigen of interest and a control antibody thereof) after removing red blood cells by hemolysis or specific gravity centrifugation, thoroughly washing the sample, and using flow cytometry. Detected scattered light or fluorescence is converted into an electric signal and analyzed with a computer. As a result, the intensity of FSC represents the size of cells, and the intensity of SSC represents the intracellular structure, which enable distinguishing lymphocytes, monocytes, and granulocytes. Subsequently, the cell population of interest is gated as needed to study the antigen expression form in the cells. In practicing the method of the present disclosure, those skilled in the art can appropriately identify the indicated cell surface marker to fractionate or count the cells.

Examples of particularly useful cell subpopulations include B cells and regulatory T cells (Treg). The present disclosure can use one or more variables comprising the B cell count and/or Treg count in addition to or in place of another variable as an indicator of ME/CFS.

As the cell subpopulation count, the ratio with respect to a suitable baseline can be used. The B cell count is, for example, the frequency (%) of B cells in peripheral blood mononuclear cells. The Treg count is, for example, the frequency (%) of Treg in all CD4 positive T cells.

In Examples 3-2 and Table 7 herein, some cell subpopulation variables are extracted with significance at $0.1 \leq P < 0.2$ in simple regression analysis. Example 6 herein has found that some of the multiple combinations of variables comprising a cell subpopulation variable exhibits an AUC value of 0.8 or greater in ROC analysis (Table 18).

(Combination)

As shown herein, one or more variables comprising a usage frequency of one or more genes in an IgG H chain variable region of a BCR in a subject can be used as an indicator of ME/CFS in the subject. One or more variables can be any combination of variables described herein. Preferably, the variables comprise a combination of two or more variables selected from the group consisting of a usage frequency of one or more genes in an IgG H chain variable region of a BCR in a subject, a BCR diversity index in a subject, and one or more immune cell subpopulation counts in a subject. Two or more variables can be three or more, four or more, five or more, six or more, or any greater number of variables.

The Examples herein demonstrate that a combination of a large number of variables exhibits a high AUC under a ROC curve in regression analysis for differentiating a normal control from ME/CFS. Those skilled in the art can appropriately combine variables and use a combination exhibiting a specified AUC. In one embodiment, a combination of variables can exhibit AUC $\geq$ 0.7, AUC $\geq$ 0.8, AUC $\geq$ 0.85, AUC $\geq$ 0.9, AUC $\geq$ 0.95, or AUC $\geq$ 0.99 under a ROC curve in regression analysis. Those skilled in the art can appropriately compute the AUC under a ROC curve in regression analysis for a particular combination of variables in accordance with the method described herein, and determine whether the particular combination of variables exhibits a desired AUC. Examples of combinations of variables include, but are not limited to, a combination of usage frequencies of IGHV1-3, IGHV3-30, IGHV3-30-3, IGHV3-49, IGHD1-26, and IGHJ6 and B cell count in a subject, a combination of usage frequencies of IGHV1-3, IGHV3-30, IGHV3-30-3, IGHV3-49, IGHD1-26, and IGHJ6 and Treg count in a subject, and the like.

Although not essential, further combining a variable shown to be predictive by simple regression analysis can lead to higher predictive performance. In the Examples herein, 46% of combination of two variables, for combinations of variables exhibiting a significant difference at p < 0.2 between a patient group and a control group in simple regression analysis, exhibited AUC $\geq$ 0.7 in ROC analysis, 81% of combinations of three variables exhibited AUC $\geq$ 0.7 in ROC analysis, and 96% of combinations of four variables exhibited AUC $\geq$ 0.7 in ROC analysis.

In the present disclosure, a subject can be diagnosed as suffering from myalgic encephalomyelitis/chronic fatigue syndrome (ME/CFS) by, for example, the following steps. A plurality of variables comprising a usage frequency of one or more genes in an IgG H chain variable region of a BCR can be provided with regard to myalgic encephalomyelitis/chronic fatigue syndrome (ME/CFS). Next, a differentiation formula generated by multivariate analysis for said variables can be provided. Providing the differentiation formula can comprise, for example, performing univariate or multivariate logistic regression for the variables, with distinction between patient/healthy individual as an objective variable; computing a constant and coefficient of a differentiation formula from a constant and a partial regression coefficient of a logit model formula generated in the logistic regression; and generating a differentiation formula based on the constant and the coefficient obtained from said processing. Next, a value of a variable for a subject can be fitted into the differentiation formula to compute the probability of suffering from ME/CFS. If the probability of suffering from ME/CFS is greater than a predetermined value, the subject can be determined as suffering from ME/CFS.

More specifically, a subject can be diagnosed as suffering from myalgic encephalomyelitis/chronic fatigue syndrome (ME/CFS) in the present disclosure by, for example, the following steps. In the method, a combination of variables is specified. Specifying the combination of variables can comprise (1) comparing a healthy individual and a myalgic encephalomyelitis/chronic fatigue syndrome (ME/CFS) patient and providing a plurality of variables comprising a usage frequency of one or more genes in an IgG H chain variable region of a BCR for which a significant difference is detected, for subjects including the ME/CFS patient and the healthy individual; and/or (2) performing univariate logistic analysis using a gene in an IgG H chain variable region of the BCR as an independent variable or multivariate logistic analysis using two or more genes in an IgG H chain variable region of the BCR as independent variables to obtain a logistic regression model formula, and performing ROC analysis for measuring a degree of fit of the logistic regression model formula to select a gene exhibiting a higher AUC value as a variable for a differentiation formula

Next, a differentiation formula generated by multivariate analysis for the variables for the differentiation formula can be provided. Providing the differentiation formula can comprise, for example, performing univariate or multivariate logistic regression for the variables, with distinction between patient/healthy individual as a response variable; computing a constant and a coefficient of a differentiation formula from a constant and a partial regression coefficient of a logit model formula generated in the logistic regression; and generating a differentiation formula based on the constant and the coefficient obtained from said processing. Next, a probability of suffering from ME/CFS can be computed by fitting values of the variables of the subject into the differentiation formula. The subject can be determined as suffering from ME/CFS if the probability of suffering from ME/CFS is greater than a predetermined value.

(Differential diagnosis)

An embodiment of the present disclosure provides a method of distinguishing ME/CFS from another disease in a subject. In one embodiment of the present disclosure, differential diagnosis is performed by using a formula comprising an indicator (variable) resulting in a difference between a non-ME/CFS group, including a normal control and a patient of another disease, and an ME/CFS group. In such a case, a differentiation formula including IGH gene that is selected as being effective in differentiation from the ME/CFS group, with healthy individual + MS as the non-ME/CFS group, can be used. IGH genes with a significant difference are found from a significance test between the ME/CFS group and non-ME/CFS group in the Examples herein. As one or more variables, variables comprising a usage frequency of at least one gene selected from the group consisting of IGHV3-49, IGHV3-30-3, IGHD1-26, IGHV3-30, IGHJ6, IGHGP, IGHV4-31, IGHV3-64, IGHD3-22, IGHV3-33, IGHV3-73, IGHV5-10-1, and IGHV4-34 can be used.

Alternatively, another embodiment envisions a form where, after differentiation with a differentiation formulation for distinguishing a normal control from an ME/CFS patient, each of the differentiation formulas for differentiating from another disease (e.g., MS) is fitted to rule out the possibility of having another disease. In such a case, the differentiation for distinguishing ME/CFS from a normal control described herein is performed, and subsequently (or before or concurrently), yet another differentiation formula is used for differentiation from another disease. Specifically, a method comprising: (a) using a part of the one or more variables as an indicator of ME/CFS in the subject; and (b) using a part of the one or more variables as an indicator of the subject suffering from ME/CFS, and not another disease, can be provided. Another disease can comprise multiple sclerosis (MS). (b) can be performed a plurality of times for a plurality of other diseases.

One embodiment of the present disclosure is a method of using one or more variables comprising a usage frequency of one or more genes in an IgG H chain variable region of a BCR in a subject as an indicator of the subject suffering from myalgic encephalomyelitis/chronic fatigue syndrome (ME/CFS), and not another disease (e.g., multiple sclerosis, MS). Such a method can be performed in combination with differentiation for distinguishing ME/CFS from a normal control described herein as needed. In this regard, if it is desirable to distinguish MS, one or more genes can comprise at least one gene selected from the group consisting of IGHV1-3, IGHV3-30, IGHV3-30-3, IGHD1-26, IGHV3-49, and IGHJ6. In addition, the one or more genes can be genes selected so that AUC ≥ 0.7, AUC ≥ 0.8, or AUC ≥ 0.9 is exhibited under a ROC curve in regression analysis for differentiating MS from ME/CFS. In the present disclosure, the number of at least one IGH genes used for differentiation of MS from ME/CFS is not particularly limited. Any number of genes from 1 to 117 genes can be used. The one or more variables used as an indicator in the present disclosure can comprise a usage frequency of about 1, about 2, about 3, about 4, about 5, about 6, about 7, about 8, about 9, about 10, about 15, about 20, about 30, about 40, about 50, about 60, about 70, about 80, about 90, about 100, about 110, or a greater number of IGH genes. When suitable, one or more variables can also be one variable.

As used herein, "subject" refers to any organism that can be subjected to the diagnosis, detection, or the like of the present disclosure, and is preferably a human.

As used herein, "sample" refers to any substance obtained from a subject. Examples thereof include peripheral blood, tissue biopsy sample, cell sample, lymph, saliva, urine, and the like. Those skilled in the art can appropriately select a preferred sample based on the descriptions herein.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

[Examples]

The Examples described hereinafter in the present specification use the following abbreviations when appropriate.

[Table A]

| Denotation | Formal name | Computation method |
|---|---|---|
| IGHV | Immunoglobulin heavy chain V region | Usage frequency (%) of each IGHV gene in all IGHV |
| IGHD | Immunoglobulin heavy chain D region | Usage frequency (%) of each IGHD gene in all IGHD |
| IGHJ | Immunoglobulin heavy chain J region | Usage frequency (%) of each IGHJ gene in all IGHJ |
| IGHG | Immunoglobulin heavy chain G chain C region | Usage frequency (%) of each IGHG gene in all IGHV |

(continued)

| Denotation | Formal name | Computation method |
|---|---|---|
| Shannon | Shannon index | $$H' = -\sum_{i=1}^{S} \frac{n_i}{N} \ln \frac{n_i}{N}$$ wherein N: total number of reads, $n_i$: number of ith unique read, S: number of unique reads |
| Inverse | Inverse Simpson index | Inverse of Simpson index (1-λ) $$1-\lambda = 1 - \sum_{i=1}^{S} \left( \frac{n_i(n_i-1)}{N(N-1)} \right)$$ |
| Pielou | Pielou's evenness index | Same as normalized Shannon index found by the following computational formula $$H' = -\sum_{i=1}^{S} \frac{n_i}{N} \ln \frac{n_i}{N} \Big/ \ln N$$ |
| DE50 | DE50 index | Ratio of the number of top unique reads accounting for 50% of all reads to the number of all unique reads |
| B cell | B cell count | Frequency (%) in peripheral blood mononuclear cells |
| nB | Naive B cell count | Frequency (%) in B cells |
| mB | Memory B cell count | Frequency (%) in B cells |
| PB | Plasmablast count | Frequency (%) in B cells |
| aN | Activated naïve B cell count | Frequency (%) in B cells |
| TrB | Transitional B cell count | Frequency (%) in B cells |
| Treg | Regulatory T cell count | Frequency (%) in CD4 positive T cells |
| mCD4T | Memory T cell count | Frequency (%) in CD4 positive T cells |
| Tfh | follicular helper T cell count | Frequency (%) in CD4 positive T cells |
| Tfh1 | Tfh1 cell count | Frequency (%) in Tfh cells |
| Tfh2 | Tfh2 cell count | Frequency (%) in Tfh cells |
| Tfh17 | Tfh17 cell count | Frequency (%) in Tfh cells |
| Th1 | Th1 cell count | Frequency (%) in CD4 positive T cells |
| Th2 | Th2 cell count | Frequency (%) in CD4 positive T cells |
| Th17 | Th17 cell count | Frequency (%) in CD4 positive T cells |

[Example 1]

Next generation B cell receptor repertoire analysis using ME/CFS patient peripheral blood mononuclear cells

1. Materials and Methods

1.1. Separation of peripheral blood mononuclear cells and RNA extraction

10 mL of whole blood was collected into a heparin containing blood collection tube from the ME/CFS patients (37 cases) and healthy controls (23 cases) shown in Table 1, and peripheral blood mononuclear cells (PBMCs) were separated by Ficoll-Paque PLUS density gradient centrifugation. Total RNA was extracted/purified using RNeasy Lipid Tissue Mini Kit (Qiagen, Germany) from the isolated PBMCs. RNA was quantified using an Agilent 2100 Bioanalyzer (Agilent)

[Table 1]

Table 1 ME/CFS patients

|  | ME/CFS patients (37 cases) | Healthy controls (23 cases) | p value |
| --- | --- | --- | --- |
| Age (mean ± SD) | 38.8±11.4 | 39.4±8.2 | 0.99 |
| Sex (male: female) | 8:29 | 7:16 | 0.54 |
| Duration of disease (years, mean (range)) | 10.9 (1 - 33) | - | - |
| Performance status (mean ± SD) | 5.9 ±1.6 | - | - |
| Onset triggered by infection-like symptom (cases, %) | 22 cases (59.5%) | - | - |

Performance status evaluated the severity in 10 levels from 0 to 9, 9 being the most severe. For p value, age was computed by Mann-Whitney U-test, and sex was computed by Fisher's exact test, where $p < 0.05$ was considered significant.

1.2. Complementary DNA and double-stranded complementary DNA synthesis

BCR genes were amplified using adaptor-ligation PCR. Complementary DNA (cDNA) was synthesized using a restriction enzyme digestion site-containing Oligo dT primer (BSL-18E: Table 2) and a reverse transcriptase. Subsequently, E.coli DNA Ligase (Invitrogen), DNA polymerase I (Invitrogen), and RNaseH (Invitrogen) were used for double stranded complementary DNA (ds-cDN) synthesis. A 5' end blunting reaction was then performed using a T4 DNA polymerase, and the end was cleaved with a restriction enzyme NotI. After column purification using a MinElute Reaction Cleanup Kit (QIAGEN), a P20EA/P10EA adaptor was added by a ligation reaction using a T4 ligase. The adaptor added ds-cDNA was digested by NotI restriction enzyme.

1.3. PCR

To specifically amplify a heavy chain (IGH) gene of an immunoglobulin γ chain (IgG) of a B cell receptor (BCR), nested PCR was performed three times using a thermal cycler T 100 (Bio Rad) with KAPA HiFi HS ReadyMix (Nippon Genetics) . P20EA and CG1 were used to perform the 1st PCR, and P20EA and CG2 were subsequently used to perform the 2nd PCR reaction. Furthermore, a tag sequence required for sequencing was added with P22EA-ST1-R and CG-ST1-R. After removing exogenous residual primer using Agencourt AMPure beads, index was added using Nextera XT Index Kit v2 Set A (Illumina).

[Table 2]

Table 2 Primer sequences

| Primer | Sequence |
| --- | --- |
| BSL18E | AAAGCGGCCGCATGCTTTTTTTTTTTTTTTTTTTVN |
| P20EA | GGGAATTCGG |
| P10EA | TAATACGACTCCGAATTCCC |
| CG1 | CACCTTGGTGTTGCTGGGCTT |
| CG2 | TCCTGAGGACTGTAGGACAGC |
| P22EA-ST1-R | GTCTCGTGGGCTCGGAGATGTGTATAAGAGACAGCTAATACGACT CCGAATTCCC |
| CG-ST1-R | TCGTCGGCAGCGTCAGATGTGTATAAGAGACAGTGAGTTCCACGA CACCGTCAC |
| (corresponding to SEQ ID NO: 1 to 7 from the top) | |

1.4. Next generation sequence analysis

The concentrations of amplicons were measured using Qubit® 3.0 fluorometer (Thermo Fisher Scientific). After diluting the amplicons to 4 nM, PhiX Control v3 (Illumina) was partially mixed to prepare the final prepared specimen. Paired-end sequencing was performed with Miseq Reagent Kit v3 (600 Cycle, Illumina) and the final prepared specimen using Illumina's Miseq sequencer.

1.5. Analysis using repertoire analysis software

The IGH gene V region sequence (IGHV), D region sequence (IGHD), J region sequence (IGHJ), and C region sequence (IGHC) were collated and the CDR3 sequence was determined with repertoire analysis software Repertoire Genesis by using a pair of Fastq base sequence data sets acquired by Miseq sequencing. The number of copies of unique reads in each sample was counted, with reads having the same IGHV, IGHD, IGHJ, IGHC, and CDR3 amino acid sequences being a unique read. The diversity index and usage frequencies of IGHV, IGHD, IGHJ, and IGHC of each sample were computed from the results of tallying unique reads.

2. Results

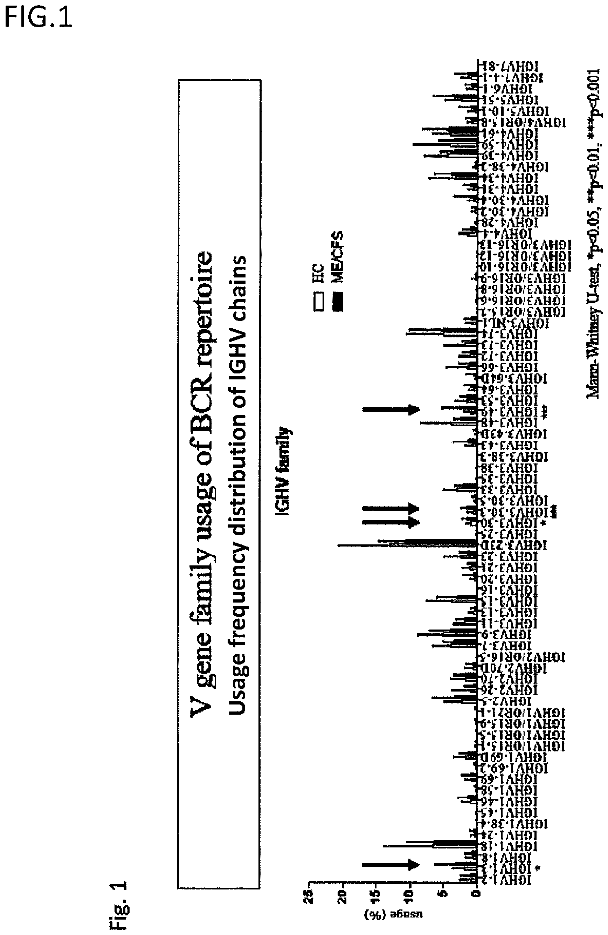

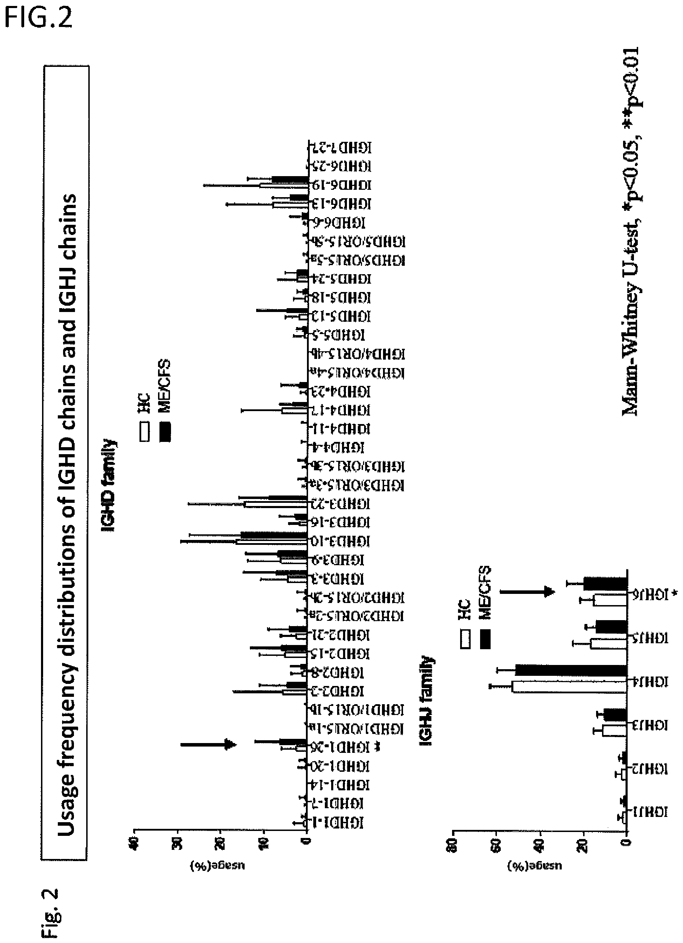

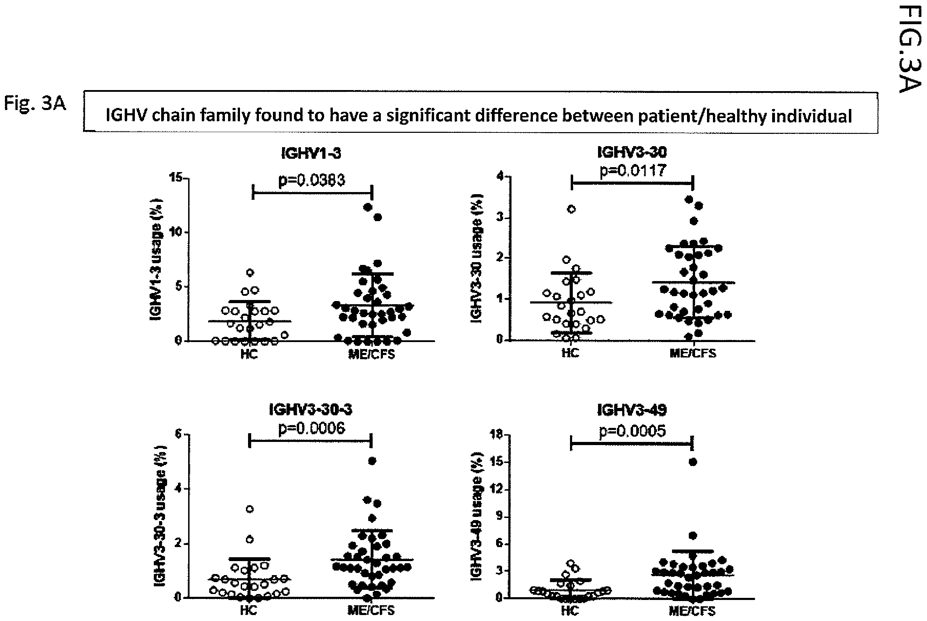

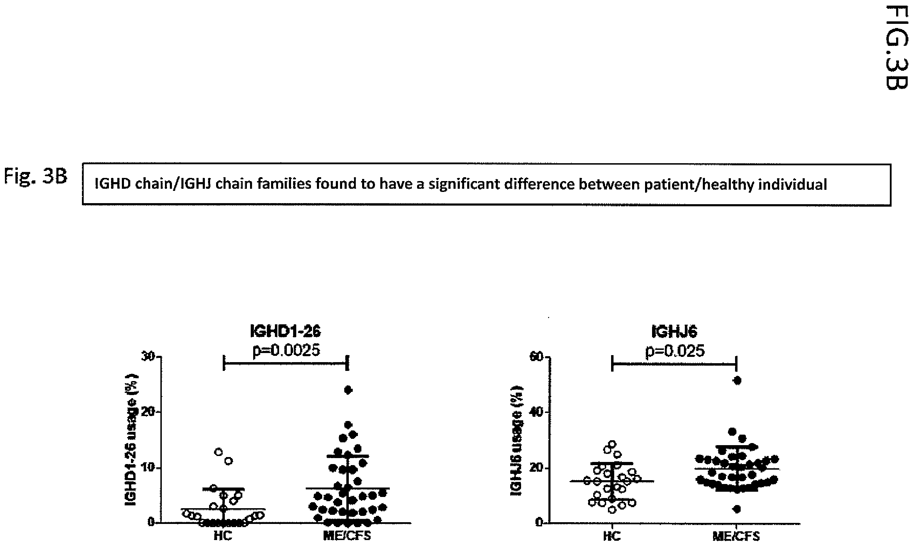

Sequence data for 200 thousand to 300 thousand reads was acquired from samples of ME/CFS patients and healthy controls (Table 3). A difference was not found in the total number of reads, assigned reads, number of in-frame reads, and number of unique reads between ME/CFS patients and healthy controls. IGHV, IGHD, IGHJ, and IGHC usage frequencies and diversity index computed from read data were compared between ME/CFS patients and healthy controls. For IGHV, IGHV1-3, IGHV3-30, IGHV3-30-3, and IGHV3-49 usage frequencies were significantly higher in ME/CFS patients as compared to healthy controls (Figure **1,** significance level of Mann-Whitney test; $P < 0.05$, $P < 0.05$, $P < 0.001$, and $P < 0.001$). IGHD1-26 for IGHD, and IGHJ6 for IGHJ exhibited a significantly higher frequency in ME/CFS patients as compared to healthy controls (Figure **2,** $P < 0.01$ and $P < 0.05$). Dot plots are shown in Figures **3A** and **3B.** These results suggest that measurement of usage frequencies of IGHV1-3, IGHV3-30, IGHV3-30-3, IGHD1-26, IGHV3-49, and IGHJ6 by next generation BCR repertoire analysis can be utilized in the differentiation of ME/CFS, which does not have an effective biomarker (Table 4).

[Table 3]

Table 3 Number of reads acquired by NGS sequencing

| | Number of reads (mean ± SD) | | |
|---|---|---|---|
| | ME/CFS patients (37 cases) | Healthy controls (23 cases) | P value |
| Total reads | 289342.6±112471.3 | 304907.2±146736.6 | 0.67 |
| Assigned reads | 162337.5±40656.5 | 166100.2±56946.8 | 0.78 |
| In-frame reads | 158459±39046.6 | 162362.7±54851.7 | 0.77 |
| Unique reads | 16936.5±9067.3 | 15377.6±6703.1 | 0.45 |

[Table 4]

Table 4 Biomarker candidate I for ME/CFS differentiation

| Use | Indicator | Variable |
|---|---|---|
| Independent | % frequency | IGHV1-3, IGHV3-30, IGHV3-30-3,-IGHD1-26, IGHV3-49, IGHJ6 |

[Example 2]

Comparison and investigation of ME/CFS patient lymphocytes by flow cytometry

1. Method

Peripheral blood mononuclear cells (PBMCs) were separated by the method described in [Example 1]. The cells were then stained with various fluorescent dye labeled monoclonal antibodies, and the frequencies of the following lymphocyte subfractions were calculated with a flow cytometer (FACS Canto II and FACS Aria II flow cytometer (BD Biosciences)). (%) B cells: CD19+ cells/PBMC; naive B cells (nB): CD19+CD27-/CD19+ cells; memory B cells (mBs), CD19+CD27+CD180+/CD19+; plasmablasts (PBs): CD19+CD27+CD180-CD38high/CD19+; transitional B cells (TrB): CD19+CD27-CD24+Mito tracker green high/CD19+; memory CD4T cells (mCD4T): CD3+CD4+CD127+CD45PA-/CD3+CD4+; follicular helper T cells (Tfh): CD3+CD4+CD127+CD45RA-CXCR5+/CD3+CD4+; helper T cells 1 (Th1 cells): CD3+CD4+CD127+CD45PA-CXCR5-CXCR3+CCR6-/CD3+CD4+CD127+CD45RA-; helper T cells 2 (Th2 cells): CD3+CD4+CD127+CD45RA-CXCR5-CXCR3-CCR6-/CD3+CD4+CD127+CD45RA-; helper T cells 17 (Th17 cells): CD3+CD4+CD127+CD45RA-CXCR5-CXCR3-CCR6+/CD3+CD4+CD127+CD45RA-; follicular helper T cells 1 (Tfh1 cells): CD3+CD4+CD127+CD45RA-CXCR5+CXCR3+CCR6-/CD3+CD4+CD127+CD45RA-CXCR5+; follicular helper T cells 2 (Tfh2 cells): CD3+CD4+CD127+CD45RA-CXCR5+CXCR3-CCR6-/CD3+CD4+CD127+CD45RA-CXCR5+; follicular helper T cells 17 (Tfh17 cells): CD3+CD4+CD127+CD45RA-CXCR5+CXCR3-CCR6+/CD3+CD4+CD127+CD45RA-CXCR5+; regulatory T cells (Treg): CD3+CD4+CD45RA-CD127-CD25++/CD3+CD4+. A significant difference between the ME/CFS patients and healthy subjects in the resulting frequencies between the two groups was tested by Mann Whitney U test. $P < 0.05$ was considered statistically significant.

2. Results

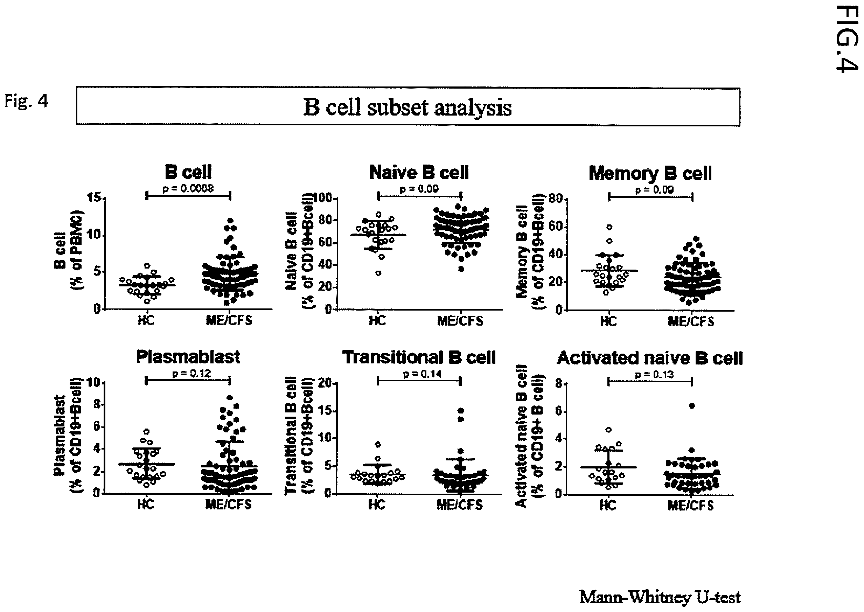

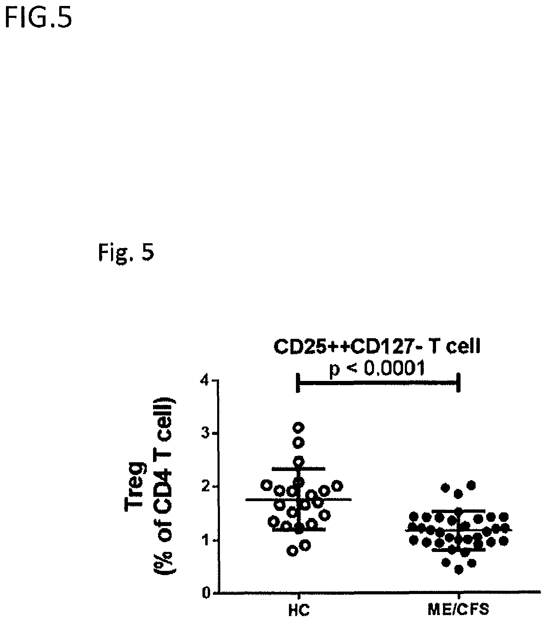

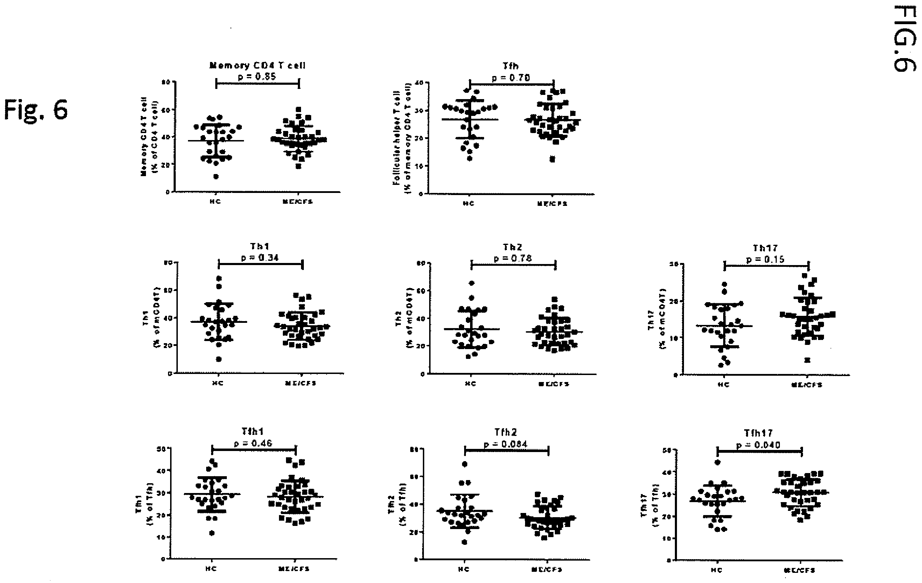

As show in Figures **4, 5,** and **6,** (%) B cells was significantly higher in the disease group, the frequency of regulatory T cells (Treg) was significantly lower in the disease group, and the frequency of follicular helper T cells 17 (Tfh17) was significantly higher in the disease group.

[Example 3-1]

Prediction and differentiation of ME/CFS utilizing IGH gene and cell subpopulation frequency data observed to have a significant difference between the two groups 1. Method
Frequency data for cell subpopulations and six IGH genes (Table 5) for which a significant difference was detected between ME/CFS patients (37 cases) and healthy controls (23 cases), i.e., (%) B cells or (%) regulatory T cells, were added to study whether ME/CFS can be predicted. Multivariate logistic analysis was performed using SPSS software (IBM). A Receiver Operating Characteristic (ROC) curve was created using prediction values for dependent variables of a regression formula. The ROC curve Area Under the Curve (AUC) value was found as a performance evaluation value for the prediction and determination of these variables.

2. Results

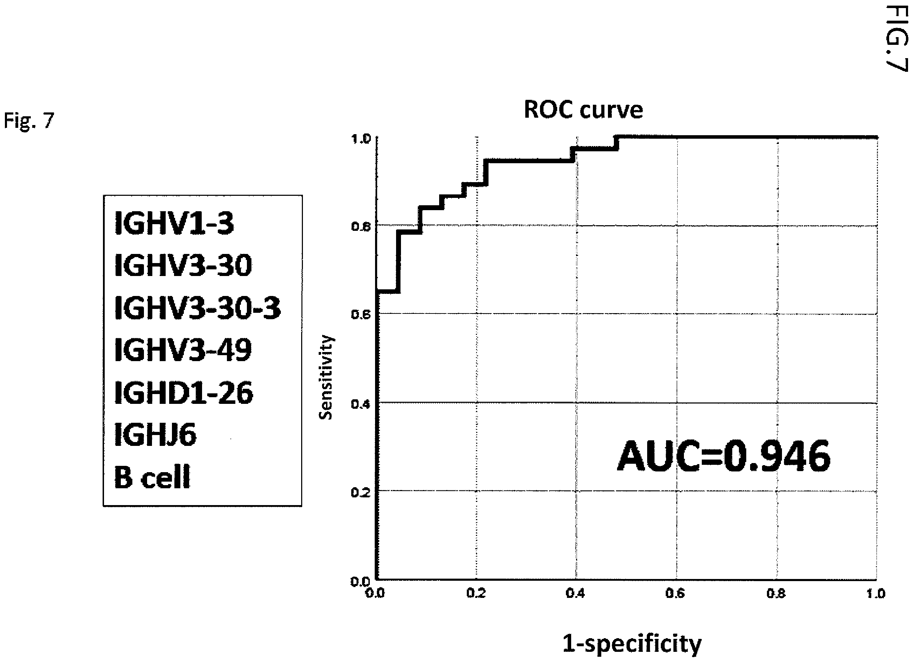

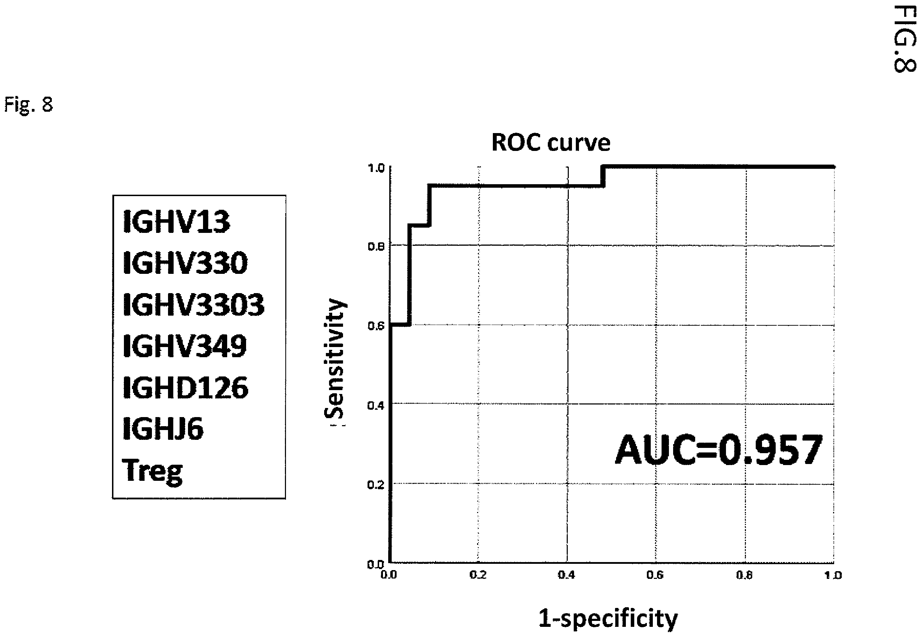

High evaluation was obtained at an AUC value of 0.946 for 6 IGH gene variables and (%) B cells (Figure **7)**. A very high evaluation was obtained at an AUC value of 0.957 in the analysis based on a regression formula using 6 IGH gene variables and (%) regulatory T cells (Figure **8)**.

[Table 5]

Table 5 Biomarker candidate II for ME/CFS differentiation

| Use | Indicator | Variable |
|---|---|---|
| Composite, logistic regression formula | % frequency | IGHV1-3, IGHD1-26, cell IGHV3-30, IGHV3-49, IGHV3-30-3, IGHJ6, B- |
| Composite, logistic regression formula | % frequency | IGHV1-3, IGHD1-26, IGHV3-30, IGHV3-49, IGHV3-30-3, IGHJ6, Treg |

[Example 3-2]

Extraction of variables for IGH genes and cell subpopulations with high predictive performance of ME/CFS by simple regression analysis

1. Method

Simple regression analysis was performed using one out of 74 types of IGHV, 32 types of IGHD, 6 types of IGHJ, 5 types of IGHC, 4 types of diversity indices, and 15 types of cell subpopulation frequency data as an independent variable and a binary variable between ME/CFS and healthy control as a dependent variable. The variables used are shown in Table 6. Variables satisfying the significance level P < 0.05, 0.05 ≤ P < 0.1, and 0.1 ≤ P < 0.2 were extracted. Simple regression and logistic regression analysis utilized glm () of R, and ROC analysis utilized pROC of the R package.

[Table 6]

Table 6 Variables used in regression analysis and ROC analysis

| Classification | Name of variable |
|---|---|
| IGHV | IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81 (total of 74 types) |
| IGHD | IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27 (total of 32 types) |
| IGHJ | IGHJ1, IGHJ2, IGHJ3, IGHJ4, IGHJ5, IGHJ6 (total of 6 types) |
| IGHC | IGHG1, IGHG2, IGHG3, IGHG4, IGHGP (total of 5 types) |
| Diversity index | Shannon, Inverse, Pielou, DE50 (total of 4 types) |
| Cell subpopulation data | B cell, nB, mB, PB, aN, TrB, Treg, mCD4T, Tfh, Tfh1, Tfh2, Tfh17, Th1, Th2, Th17 (total of 15 types) |

2. Results

From 74 types of IGHV, 32 types of IGHD, 6 types of IGHJ, 5 types of IGHC, 4 types of diversity indices, and 15 types of cell subpopulation frequency data for a total of 136 types of variables, 8 types of IGH genes and 2 types of cell subpopulation variables for a total of 8 types were extracted as variable satisfying the significance level of p < 0.05 (Table 7). 10 types of IGH genes and 4 types of cell subpopulation frequency data were variables satisfying 0.05 ≤ P < 0.1. 16 types of IGH genes, 3 types of diversity indices, and 2 types of cell subpopulation variables were variables satisfying 0.1 ≤ P < 0.2. It is suggested that variables satisfying the significance level of P < 0.2 are independently effective in predicting ME/CFS. In particular, it is suggested that variables satisfying the significance level of P < 0.05 are independently very effective in predicting ME/CFS (Table 8). Variables satisfying the significance level of P < 0.2 were used in analysis using multivariate logistic regression analysis.

When ROC analysis was performed with some of the variables independently, AUC values of IGHV3-49: 0.764, IGHV3-30-3: 0.759, IGHD1-26: 0.731, IGHJ6: 0.672, IGHV3-30: 0.693, IGHV1-3: 0.659, B cell: 0.771, Treg: 0.817, Shannon: 0.617, and Inverse: 0.636 were obtained.

[Table 7]

Table 7 Variables satisfying the significance level in simple regression analysis

| Significance level | Variable |
|---|---|
| P<0.05 | (IGH) IGHV3-49, IGHV3-30-3, IGHD1-26, IGHJ6, IGHV3-30, IGHGP, IGHV1-3, IGHD3-22 (total of 8 types) |
| | (Cell subpopulation) B cell, Treg (total of 2 types) |
| 0.05≦P<0.1 | (IGH) IGHD6-6, IGHV3-33, IGHD4-23, IGHV3-30-5, IGHV3-23, IGHD6-13, IGHV3-64D, IGHV3-48, IGHV3-64, IGHG1 (total of 10 types) |
| | (Cell subpopulation) Tfh17, mB, Tfh2, nB (total of 4 types) |
| 0.1≦P<0.2 | (IGH) IGHV3-73, IGHV1-69-2, IGHV5-51, IGHV4-31, IGHV3-23D, IGHV1/OR15-9, IGHV4-39, IGHD5-12, IGHV3-43D, IGHD4-17, IGHV5-10-1, IGHD4/OR15-4a/b, IGHG4, IGHV1/OR15-5, IGHV3/OR16-9, IGHD1-7, IGHV3-21 (total of 17 types) |

(continued)

Table 7 Variables satisfying the significance level in simple regression analysis

| Significance level | Variable |
|---|---|
| | (Diversity index) Shannon, Inverse, Pielou (total of 3 types) (Cell subpopulation) Th17, aN (total of 2 types) |

[Table 8]

Table 8 Biomarker candidate III for ME/CFS differentiation

| Use | Indicator | Variable |
|---|---|---|
| Independent, regression formula | % frequency | IGHV3-49, IGHV3-30-3, IGHD1-26, IGHJ6, IGHV3-30, IGHGP, IGHV1-3, IGHD3-22, B-cell, Treg |

[Example 4]

Prediction and differentiation of ME/CFS using two types of IGH genes

1. Method

Multivariate logistic regression analysis was performed for a combination of any two types of IGH genes from 74 types of IGHV, 32 types of IGHD, 6 types of IGHJ, and 5 types of IGHC for a total of 117 types of IGH genes, and ROC curves were created using prediction values for dependent variables of each regression formula. An AUC value exhibiting predictive performance of ME/CFS was computed with respect to the ROC curves. Combinations of IGH genes exhibiting an AUC value of 0.7 or 0.8 or greater and a list of IGH genes used in the combinations were extracted. Logistic regression analysis utilized glm() of R, and ROC analysis utilized pROC of the R package.

2. Results

Combinations of IGH genes exhibiting an AUC value of 0.8 or 0.7 or greater in ROC analysis using any two types of IGH genes were 30 and 637 sets, respectively. Table 11 shows combinations of variables exhibiting AUC ≥ 0.7 by binary logistic regression and ROC analysis. 26 IGH genes used in the combinations had an AUC value of 0.8 or greater, and 117 genes had an AUC value of 0.7 or greater. (Table 9). 10 (33%) of the 30 sets of gene combinations exhibiting an AUC value of 0.8 or greater are combinations of variables exhibiting significance in simple regression analysis, and 20 sets (67%) had one of the IGHs exhibiting significance in simple regression analysis (Table 10). These results show that ME/CFS patients can be predicted and differentiated by combining any IGH genes.

[Table 9]

Table 9 IGH genes that can be utilized in prediction and differentiation of ME/CFS

| AUC value≥0.8 | IGHV3-49, IGHV3-30-3, IGHGP, IGHD6-6, IGHV3-30, IGHV3-64D, IGHD1-26, IGHG4, IGHV1-3, IGHV1-69-2, IGHV1/OR15-9, IGHV1/OR21-1, IGHV3-30-5, IGHV3-38, IGHV3-38-3, IGHV3-43D, IGHV3-64, IGHV3-73, IGHV3-NL1, IGHV3/OR16-6, IGHV4-31, IGHD3-22, IGHD4-23, IGHD5-12, IGHD6-13, IGHJ6 (26 genes) |
|---|---|

(continued)

Table 9 IGH genes that can be utilized in prediction and differentiation of ME/CFS

| AUC value≥0.7 | IGHV3-30-3, IGHV3-49, IGHD1-26, IGHV3-30, IGHV3-64, IGHJ6, IGHV3-30-5, IGHD4-23, IGHV3-64D, IGHV1-3, IGHV3-23, IGHD6-6, IGHGP, IGHV1/OR15-9, IGHD3-22, IGHV4-34, IGHV1-8, IGHV4-31, IGHD1/OR15-1a/b, IGHV3-33, IGHV3-73, IGHV3/OR15-7, IGHV5-10-1, IGHV5-51, IGHD6-13, IGHG1, IGHV1/OR15-5, IGHV1/OR21-1, IGHD5-12, IGHV3-43D, IGHV7-81, IGHD2-21, IGHV1-69-2, IGHV3-48, IGHV3/OR16-8, IGHD1-7, IGHG4, IGHD4-4, IGHD4-11, IGHV3-38, IGHV3-NL1, IGHD4/OR15-4a/b, IGHD6-25, IGHV3-9, IGHV3-72, IGHV3/OR16-6, IGHV3/OR16-9, IGHV3/OR16-10, IGHV4-39, IGHD1-20, IGHD3-3, IGHD4-17, IGHG2, IGHV1-58, IGHV2/OR16-5, IGHV3-38-3, IGHV3-66, IGHV3/OR16-13, IGHV4-28, IGHD2-15, IGHD3-10, IGHD7-27, IGHV1-45, IGHV1-69, IGHV1-69D, IGHV1/OR15-1, IGHV2-5, IGHV2-26, IGHV3-7, IGHV3-15, IGHV3-16, IGHV3-25, IGHV3-35, IGHV3/OR16-12, IGHV4-4, IGHV4-30-4, IGHV7-4-1, IGHD1-14, IGHD2-2, IGHD2-8, IGHD3-9, IGHJ1, IGHG3, IGHV1-2, IGHV1-18, IGHV1-24, IGHV1-38-4, IGHV1-46, IGHV2-70, IGHV2-70D, IGHV3-11, IGHV3-13, IGHV3-20, IGHV3-21, IGHV3-23D, IGHV3-43, IGHV3-53, IGHV3-74, IGHV4-30-2, IGHV4-38-2, IGHV4-59, IGHV4-61, IGHV4/OR15-8, IGHV6-1, IGHD1-1, IGHD2/OR15-2a/b, IGHD3-16, IGHD3/OR15-3a/b, IGHD5-5, IGHD5-18, IGHD5-24, IGHD5/OR15-5a/b, IGHD6-19, IGHJ2, IGHJ3, IGHJ4, IGHJ5 (117 genes) |
|---|---|

[Table 10]

Table 10 Prediction of ME/CFS patients from combination of any two types of IGH genes

| Variable 1 | Variable 2 | Number of data | AUC |
|---|---|---|---|
| IGHGP*1 | IGHV3-30-3 | 60 | 0.848414 |
| IGHV3-30-3 | IGHV3-49 | 60 | 0.843713 |
| IGHV3-49 | IGHV3-64D | 60 | 0.842538 |
| IGHV1-3 | IGHV3-49 | 60 | 0.841363 |
| IGHV3-49 | IGHD3-22 | 60 | 0.840188 |
| IGHV3-30-5 | IGHV3-49 | 60 | 0.831962 |
| IGHD1-26 | IGHD6-6 | 60 | 0.829612 |
| IGHV3-49 | IGHD1-26 | 60 | 0.826087 |
| IGHV3-30-3 | IGHD6-6 | 60 | 0.824912 |
| IGHV3-38 | IGHV3-49 | 60 | 0.823737 |
| IGHV3-49 | IGHD4-23 | 60 | 0.823737 |
| IGHV3-30 | IGHV3-49 | 60 | 0.819036 |
| IGHV3-30-3 | IGHJ6 | 60 | 0.815511 |
| IGHV3-49 | IGHV3-64 | 60 | 0.811986 |
| IGHGP | IGHV3-30 | 60 | 0.810811 |
| IGHV3-30-3 | IGHV3-64D | 60 | 0.810811 |
| IGHV3-49 | IGHV3-73 | 60 | 0.810811 |
| IGHV1/OR15-9 | IGHV3-30-3 | 60 | 0.809636 |
| IGHV1/21-1 | IGHV3-49 | 60 | 0.808461 |
| IGHV3-49 | IGHD6-6 | 60 | 0.808461 |
| IGHV3-30-3 | IGHD5-12 | 60 | 0.807286 |
| IGHV3-30-3 | IGHD6-13 | 60 | 0.807286 |
| IGHV3-49 | IGHV4-31 | 60 | 0.807286 |
| IGHV3-49 | IGHV3/OR16-6 | 60 | 0.806111 |
| IGHG4 | IGHV3-49 | 60 | 0.80376 |
| IGHV1-69-2 | IGHV3-49 | 60 | 0.80376 |
| IGHGP | IGHV3-49 | 60 | 0.802585 |
| IGHV3-30-3 | IGHV3-43D | 60 | 0.802585 |
| IGHV3-49 | IGHV3-NL1 | 60 | 0.80141 |

(continued)

Table 10 Prediction of ME/CFS patients from combination of any two types of IGH genes

| Variable 1 | Variable 2 | Number of data | AUC |
|---|---|---|---|
| IGHV3-30-3 | IGHV3-38-3 | 60 | 0.800235 |

*1: underlined genes are genes found to have significance at significance level of P < 0.05 by simple regression analysis

[Table 11-1]

Combinations of variables exhibiting AUC ≥ 0.7 from binary logistic regression and ROC analysis

| 1- | Gene 1 | Gene 2 | 101- | Gene 1 | Gene 2 |
|---|---|---|---|---|---|
| 1 | IGHGP | IGHV3-30-3 | 101 | IGHV1/OR15-1 | IGHV3-49 |
| 2 | IGHV3-30-3 | IGHV3-49 | 102 | IGHV1/OR15-5 | IGHV3-30-3 |
| 3 | IGHV3-49 | IGHV3-64D | 103 | IGHV3-30-3 | IGHD3-10 |
| 4 | IGHV1-3 | IGHV3-49 | 104 | IGHV3-49 | IGHV4-59 |
| 5 | IGHV3-49 | IGHD3-22 | 105 | IGHGP | IGHD6-6 |
| 6 | IGHV3-30-5 | IGHV3-49 | 106 | IGHV3-30-3 | IGHV4-61 |
| 7 | IGHD1-26 | IGHD6-6 | 107 | IGHV3-30-3 | IGHD3-16 |
| 8 | IGHV3-49 | IGHD1-26 | 108 | IGHV3-30-3 | IGHJ5 |
| 9 | IGHV3-30-3 | IGHD6-6 | 109 | IGHV3-49 | IGHV3-53 |
| 10 | IGHV3-38 | IGHV3-49 | 110 | IGHV3-49 | IGHD5-12 |
| 11 | IGHV3-49 | IGHD4-23 | 111 | IGHV2-26 | IGHV3-30-3 |
| 12 | IGHV3-30 | IGHV3-49 | 112 | IGHV3-30-3 | IGHD1-14 |
| 13 | IGHV3-30-3 | IGHJ6 | 113 | IGHV3-49 | IGHD2-2 |
| 14 | IGHV3-49 | IGHV3-64 | 114 | IGHD1-7 | IGHD1-26 |
| 15 | IGHGP | IGHV3-30 | 115 | IGHV3-30-3 | IGHV3-38 |
| 16 | IGHV3-30-3 | IGHV3-64D | 116 | IGHV3-49 | IGHD2-15 |
| 17 | IGHV3-49 | IGHV3-73 | 117 | IGHG3 | IGHV3-30-3 |
| 18 | IGHV1/OR15-9 | IGHV3-30-3 | 118 | IGHV1-2 | IGHV3-30-3 |
| 19 | IGHV1/OR21-1 | IGHV3-49 | 119 | IGHV1-3 | IGHD1-26 |
| 20 | IGHV3-49 | IGHD6-6 | 120 | IGHV1-38-4 | IGHV3-49 |
| 21 | IGHV3-30-3 | IGHD5-12 | 121 | IGHV1/OR15-5 | IGHD1-26 |
| 22 | IGHV3-30-3 | IGHD6-13 | 122 | IGHV2/OR16-5 | IGHV3-30-3 |
| 23 | IGHV3-49 | IGHV4-31 | 123 | IGHV3-30-3 | IGHV3/OR16-6 |
| 24 | IGHV3-49 | IGHV3/OR16-6 | 124 | IGHV3-30-3 | IGHV3/OR16-8 |
| 25 | IGHG4 | IGHV3-49 | 125 | IGHV3-30-3 | IGHD1-7 |
| 26 | IGHV1-69-2 | IGHV3-49 | 126 | IGHV3-30-3 | IGHD2-8 |
| 27 | IGHGP | IGHV3-49 | 127 | IGHV3-43 | IGHV3-49 |
| 28 | IGHV3-30-3 | IGHV3-43D | 128 | IGHV3-49 | IGHV3/OR15-7 |
| 29 | IGHV3-49 | IGHV3-NL1 | 129 | IGHV3-49 | IGHV4-61 |
| 30 | IGHV3-30-3 | IGHV3-38-3 | 130 | IGHV3-49 | IGHV7-4-1 |
| 31 | IGHV1-69-2 | IGHV3-30-3 | 131 | IGHV3-49 | IGHV7-81 |
| 32 | IGHV3-30-3 | IGHD4/OR15-4a/b | 132 | IGHV1-45 | IGHV3-30-3 |

[Table 11-2]

| 33 | IGHV3-20 | IGHV3-49 | 133 | IGHV1/OR21-1 | IGHD1-26 |
|---|---|---|---|---|---|
| 34 | IGHV3-49 | IGHD4-11 | 134 | IGHV3-7 | IGHV3-49 |
| 35 | IGHG4 | IGHV3-30-3 | 135 | IGHV3-30-3 | IGHV3-73 |
| 36 | IGHV1/OR15-9 | IGHD1-26 | 136 | IGHV3-30-3 | IGHV4-31 |
| 37 | IGHV3-49 | IGHV4-30-4 | 137 | IGHV3-49 | IGHV3-74 |

(continued)

| | | | | | |
|---|---|---|---|---|---|
| 38 | IGHV3-30-3 | IGHV4-39 | 138 | IGHV3-49 | IGHV5-51 |
| 39 | IGHV3-49 | IGHV4-30-2 | 139 | IGHV1-69 | IGHV3-30-3 |
| 40 | IGHV2-5 | IGHV3-30-3 | 140 | IGHV3-23 | IGHV3-33 |
| 41 | IGHV3-49 | IGHD4-4 | 141 | IGHV3-43D | IGHV3-49 |
| 42 | IGHV1/OR15-5 | IGHV3-49 | 142 | IGHV3-49 | IGHD2-21 |
| 43 | IGHV3-23 | IGHV3-49 | 143 | IGHV3-49 | IGHD4-17 |
| 44 | IGHV3-30-3 | IGHD4-4 | 144 | IGHV3-49 | IGHD6-19 |
| 45 | IGHV1/OR21-1 | IGHV3-30-3 | 145 | IGHV3-49 | IGHD6-25 |
| 46 | IGHV3-30-3 | IGHD1/OR15-1a/b | 146 | IGHV3-49 | IGHJ3 |
| 47 | IGHV3-30-3 | IGHD6-25 | 147 | IGHV3-30-5 | IGHD5-12 |
| 48 | IGHV3-48 | IGHV3-49 | 148 | IGHV3-49 | IGHV4-38-2 |
| 49 | IGHV3-30-3 | IGHD4-11 | 149 | IGHV3-49 | IGHD2-8 |
| 50 | IGHV3-35 | IGHV3-49 | 150 | IGHV3-30-3 | IGHD1-1 |
| 51 | IGHV3-49 | IGHV3/OR16-9 | 151 | IGHV3-30-3 | IGHJ4 |
| 52 | IGHV3-49 | IGHD3-16 | 152 | IGHG1 | IGHV3-49 |
| 53 | IGHV1/OR15-9 | IGHV3-49 | 153 | IGHV1-69 | IGHV3-49 |
| 54 | IGHV3-30-3 | IGHD2-21 | 154 | IGHV2-70D | IGHV3-49 |
| 55 | IGHV3-33 | IGHV3-49 | 155 | IGHV3-11 | IGHV3-30-3 |
| 56 | IGHV3-49 | IGHV5-10-1 | 156 | IGHV3-13 | IGHV3-49 |
| 57 | IGHV3-23D | IGHV3-30-3 | 157 | IGHV3-25 | IGHV3-30-3 |
| 58 | IGHV3-30-3 | IGHV7-81 | 158 | IGHV3-30-3 | IGHV3-66 |
| 59 | IGHV3-64D | IGHD1-26 | 159 | IGHV3-30-3 | IGHV4/OR15-8 |
| 60 | IGHV3-30-3 | IGHD4-17 | 160 | IGHV3-30-3 | IGHV7-4-1 |
| 61 | IGHV3-49 | IGHV3/OR16-12 | 161 | IGHV3-49 | IGHD7-27 |
| 62 | IGHV3-49 | IGHJ6 | 162 | IGHV3-49 | IGHJ4 |
| 63 | IGHV3-30-3 | IGHD3-22 | 163 | IGHGP | IGHV3-23 |
| 64 | IGHV3-49 | IGHD4/OR15-4a/b | 164 | IGHV2-26 | IGHV3-49 |
| 65 | IGHV3-49 | IGHD5-5 | 165 | IGHV3-16 | IGHV3-49 |
| 66 | IGHV3-73 | IGHD1-26 | 166 | IGHV3-30-3 | IGHV3/OR16-13 |

[Table 11-3]

| | | | | | |
|---|---|---|---|---|---|
| 67 | IGHGP | IGHV3-30-5 | 167 | IGHV3-30-3 | IGHV4-30-4 |
| 68 | IGHV2/OR16-5 | IGHV3-49 | 168 | IGHV3-49 | IGHD3-10 |
| 69 | IGHV3-49 | IGHD6-13 | 169 | IGHG2 | IGHV3-49 |
| 70 | IGHV2-70 | IGHV3-30-3 | 170 | IGHV1-8 | IGHV3-49 |
| 71 | IGHV3-15 | IGHV3-49 | 171 | IGHV1-24 | IGHV3-49 |
| 72 | IGHV3-30-3 | IGHD2-15 | 172 | IGHV3-7 | IGHV3-30-3 |
| 73 | IGHV3-49 | IGHD1/OR15-1a/b | 173 | IGHV3-30-3 | IGHV3-43 |
| 74 | IGHV3-49 | IGHD3-3 | 174 | IGHV3-30-3 | IGHD5/OR15-5a/b |
| 75 | IGHV3-49 | IGHD5-18 | 175 | IGHV3-30-3 | IGHD7-27 |
| 76 | IGHV3-30-3 | IGHV5-10-1 | 176 | IGHV3-49 | IGHV3-72 |
| 77 | IGHV3-49 | IGHV4-34 | 177 | IGHV3-49 | IGHV4/OR15-8 |
| 78 | IGHV3-49 | IGHJ2 | 178 | IGHV3-49 | IGHD3-9 |
| 79 | IGHV3-25 | IGHV3-49 | 179 | IGHV3-23 | IGHV3-30-3 |
| 80 | IGHV3-30-3 | IGHD3-3 | 180 | IGHV1-18 | IGHV3-49 |
| 81 | IGHV3-30-3 | IGHJ3 | 181 | IGHV1-38-4 | IGHV3-30-3 |
| 82 | IGHV3-49 | IGHV3/OR16-8 | 182 | IGHV1-46 | IGHV3-30-3 |
| 83 | IGHV3-49 | IGHV4-4 | 183 | IGHV1-58 | IGHV3-49 |
| 84 | IGHV3-49 | IGHD1-7 | 184 | IGHV1-58 | IGHD1-26 |
| 85 | IGHV3-49 | IGHD1-20 | 185 | IGHV1-69D | IGHV3-49 |

(continued)

| | Gene 1 | Gene 2 | | Gene 1 | Gene 2 |
|---|---|---|---|---|---|
| 86 | IGHD1-26 | IGHD1/0R15-1a/b | 186 | IGHV1/OR15-9 | IGHV3-30-5 |
| 87 | IGHGP | IGHV3-64 | 187 | IGHV3-30 | IGHD6-6 |
| 88 | IGHV3-30-3 | IGHV4-28 | 188 | IGHV3-30-3 | IGHV3-72 |
| 89 | IGHV3-49 | IGHV3-66 | 189 | IGHV3-30-3 | IGHJ1 |
| 90 | IGHV3-49 | IGHV3/OR16-13 | 190 | IGHV3-30-5 | IGHD1-26 |
| 91 | IGHV3-49 | IGHD5-24 | 191 | IGHV3-38-3 | IGHV3-49 |
| 92 | IGHV3-9 | IGHV3-49 | 192 | IGHV3-43D | IGHD1-26 |
| 93 | IGHV3-11 | IGHV3-49 | 193 | IGHV3-49 | IGHD1-1 |
| 94 | IGHV3-23D | IGHV3-49 | 194 | IGHV3-49 | IGHD2/OR15-2a/b |
| 95 | IGHV3-49 | IGHV4-39 | 195 | IGHV3/OR15-7 | IGHD1-26 |
| 96 | IGHV2-5 | IGHV3-49 | 196 | IGHD1-26 | IGHD4-4 |
| 97 | IGHV3-30-3 | IGHD1-26 | 197 | IGHV1-18 | IGHV3-30-3 |
| 98 | IGHG1 | IGHV3-30-3 | 198 | IGHV1-45 | IGHV3-49 |
| 99 | IGHV1-2 | IGHV3-49 | 199 | IGHV3-30-3 | IGHV3-33 |
| 100 | IGHV1-8 | IGHV3-30-3 | 200 | IGHV3-30-3 | IGHV3/OR16-10 |

[Table 11-4]

| 201- | Gene 1 | Gene 2 | 301- | Gene 1 | Gene 2 |
|---|---|---|---|---|---|
| 201 | IGHV3-30-3 | IGHV3/OR16-12 | 301 | IGHV1/OR21-1 | IGHD4-23 |
| 202 | IGHV3-30-3 | IGHV4-30-2 | 302 | IGHV3-15 | IGHD1-26 |
| 203 | IGHV3-30-3 | IGHD1-20 | 303 | IGHV3-64D | IGHV3/OR16-8 |
| 204 | IGHV3-30-3 | IGHD2/OR15-2a/b | 304 | IGHV5-10-1 | IGHD1-26 |
| 205 | IGHV3-30-3 | IGHD5-5 | 305 | IGHV5-51 | IGHD1-26 |
| 206 | IGHV3-30-3 | IGHD6-19 | 306 | IGHD6-13 | IGHJ6 |
| 207 | IGHV3-30-5 | IGHD6-6 | 307 | IGHV3-30 | IGHV7-81 |
| 208 | IGHV3-49 | IGHV6-1 | 308 | IGHD1-26 | IGHD3-9 |
| 209 | IGHV3-49 | IGHD3/OR15-3a/b | 309 | IGHD1-26 | IGHD3-16 |
| 210 | IGHV3-49 | IGHJ1 | 310 | IGHV3-16 | IGHD1-26 |
| 211 | IGHD1-26 | IGHD4-11 | 311 | IGHV3-48 | IGHJ6 |
| 212 | IGHV2-70D | IGHV3-30-3 | 312 | IGHV3/OR16-10 | IGHD1-26 |
| 213 | IGHV3-30-3 | IGHD3-9 | 313 | IGHV4-34 | IGHD1-26 |
| 214 | IGHG3 | IGHV3-49 | 314 | IGHD1-26 | IGHD3-3 |
| 215 | IGHV1-69-2 | IGHD1-26 | 315 | IGHD1-26 | IGHJ3 |
| 216 | IGHV2-70 | IGHV3-49 | 316 | IGHGP | IGHD1-26 |
| 217 | IGHV3-16 | IGHV3-30-3 | 317 | IGHV3-21 | IGHD1-26 |
| 218 | IGHV3-20 | IGHV3-30-3 | 318 | IGHV3-30-5 | IGHV3/OR16-8 |
| 219 | IGHV3-30 | IGHV3-64D | 319 | IGHV3/OR16-8 | IGHD1-26 |
| 220 | IGHV3-30-3 | IGHV3/OR16-9 | 320 | IGHV3-73 | IGHD3-22 |
| 221 | IGHV3-30-3 | IGHV4-34 | 321 | IGHV3/OR16-6 | IGHD1-26 |
| 222 | IGHD1-26 | IGHD4/OR15-4a/b | 322 | IGHD1-26 | IGHJ2 |
| 223 | IGHGP | IGHV4-34 | 323 | IGHV1/OR15-1 | IGHD1-26 |
| 224 | IGHV1-3 | IGHV3-30-3 | 324 | IGHV3-13 | IGHD1-26 |
| 225 | IGHV1-46 | IGHV3-49 | 325 | IGHV3-64D | IGHV4-31 |
| 226 | IGHV3-21 | IGHV3-49 | 326 | IGHV3-64D | IGHV5-51 |
| 227 | IGHV3-23D | IGHD1-26 | 327 | IGHV4/OR15-8 | IGHD1-26 |
| 228 | IGHV3-30-3 | IGHV3-NL1 | 328 | IGHG3 | IGHD1-26 |
| 229 | IGHV3-30-3 | IGHV3/OR15-7 | 329 | IGHG2 | IGHV3-23 |
| 230 | IGHV3-30-3 | IGHV4-38-2 | 330 | IGHG2 | IGHD1-26 |
| 231 | IGHV3-30-3 | IGHV6-1 | 331 | IGHV3-53 | IGHD1-26 |

(continued)

| 201- | Gene 1 | Gene 2 | 301- | Gene 1 | Gene 2 |
|---|---|---|---|---|---|
| 232 | IGHV3-30-3 | IGHD2-2 | 332 | IGHD1-26 | IGHD2-21 |

[Table 11-5]

| 201- | Gene 1 | Gene 2 | 301- | Gene 1 | Gene 2 |
|---|---|---|---|---|---|
| 233 | IGHV3-30-3 | IGHD4-23 | 333 | IGHD1-26 | IGHD5-24 |
| 234 | IGHV3-30-3 | IGHD5-24 | 334 | IGHV1-3 | IGHV3-64D |
| 235 | IGHV3-49 | IGHV3/OR16-10 | 335 | IGHV1-24 | IGHD1-26 |
| 236 | IGHV7-81 | IGHD1-26 | 336 | IGHV1-46 | IGHD1-26 |
| 237 | IGHD1-26 | IGHD5-12 | 337 | IGHV3-48 | IGHD1-26 |
| 238 | IGHG1 | IGHV3-23 | 338 | IGHV3-64 | IGHV7-81 |
| 239 | IGHV3-13 | IGHV3-30-3 | 339 | IGHV3-30 | IGHD1/OR15-1a/b |
| 240 | IGHV3-30 | IGHV3-30-3 | 340 | IGHV3-30 | IGHD5-12 |
| 241 | IGHV3-30-3 | IGHV3-53 | 341 | IGHV3-30-5 | IGHD1/OR15-1a/b |
| 242 | IGHV3-30-3 | IGHV4-4 | 342 | IGHV3-38-3 | IGHD1-26 |
| 243 | IGHV3-30-3 | IGHV4-59 | 343 | IGHV2/OR16-5 | IGHD1-26 |
| 244 | IGHV3-30-3 | IGHJ2 | 344 | IGHV7-4-1 | IGHD1-26 |
| 245 | IGHV3-49 | IGHJ5 | 345 | IGHG1 | IGHV3-30 |
| 246 | IGHD1-26 | IGHD6-25 | 346 | IGHV1-2 | IGHD1-26 |
| 247 | IGHD1-26 | IGHJ6 | 347 | IGHV2/OR16-5 | IGHV3-30 |
| 248 | IGHV3-30-3 | IGHV3-48 | 348 | IGHV3-23 | IGHD1-26 |
| 249 | IGHV3-49 | IGHV4-28 | 349 | IGHV3-30 | IGHV5-10-1 |
| 250 | IGHV3-49 | IGHD1-14 | 350 | IGHV3-30 | IGHD3-3 |
| 251 | IGHG2 | IGHV3-30-3 | 351 | IGHV4-28 | IGHD1-26 |
| 252 | IGHV1-24 | IGHV3-30-3 | 352 | IGHD1-26 | IGHD2-15 |
| 253 | IGHV1-58 | IGHV3-30-3 | 353 | IGHGP | IGHD3-22 |
| 254 | IGHV1-69D | IGHV3-30-3 | 354 | IGHV1-3 | IGHD4/OR15-4a/b |
| 255 | IGHV3-30 | IGHJ6 | 355 | IGHV3-9 | IGHD6-6 |
| 256 | IGHV3-30-3 | IGHV3-30-5 | 356 | IGHV3/OR16-12 | IGHD1-26 |
| 257 | IGHV3-30-3 | IGHV5-51 | 357 | IGHV4-4 | IGHD1-26 |
| 258 | IGHV3-30-3 | IGHD5-18 | 358 | IGHV4-30-4 | IGHD1-26 |
| 259 | IGHV3-49 | IGHD5/OR15-5a/b | 359 | IGHD1-26 | IGHD5-5 |
| 260 | IGHV3-64 | IGHD6-6 | 360 | IGHD1-26 | IGHJ5 |
| 261 | IGHD1-26 | IGHD4-17 | 361 | IGHG1 | IGHD3-22 |
| 262 | IGHG4 | IGHD1-26 | 362 | IGHG4 | IGHV3-30 |
| 263 | IGHGP | IGHV1-3 | 363 | IGHV1-69-2 | IGHV3-30 |
| 264 | IGHGP | IGHD1-20 | 364 | IGHV2-70 | IGHD1-26 |
| 265 | IGHV3-30 | IGHD1-26 | 365 | IGHV2-70D | IGHD1-26 |
| 266 | IGHV3-30-3 | IGHV3-35 | 366 | IGHV3-30 | IGHV4-31 |

[Table 11-6]

| 201- | Gene 1 | Gene 2 | 301- | Gene 1 | Gene 2 |
|---|---|---|---|---|---|
| 267 | IGHV3-30-3 | IGHD3/OR15-3a/b | 367 | IGHV3-30 | IGHD2-21 |
| 268 | IGHV3-64 | IGHV3-64D | 368 | IGHV3-64 | IGHD3-22 |
| 269 | IGHV3-64D | IGHJ6 | 369 | IGHV3-64D | IGHV4-34 |
| 270 | IGHV3-30-3 | IGHV3-64 | 370 | IGHV3-66 | IGHD1-26 |
| 271 | IGHV3-30-3 | IGHV3-74 | 371 | IGHV3-72 | IGHD1-26 |
| 272 | IGHD1-26 | IGHD4-23 | 372 | IGHV5-51 | IGHD4-23 |
| 273 | IGHV3-64 | IGHV3/OR16-8 | 373 | IGHD1-26 | IGHD2/OR15-2a/b |
| 274 | IGHV4-31 | IGHD1-26 | 374 | IGHD1-26 | IGHD5-18 |

(continued)

| | Gene 1 | Gene 2 | | Gene 1 | Gene 2 |
|---|---|---|---|---|---|
| 275 | IGHV1/OR15-1 | IGHV3-30-3 | 375 | IGHD1-26 | IGHD5/OR15-5a/b |
| 276 | IGHV3-9 | IGHV3-30-3 | 376 | IGHD1-26 | IGHD6-19 |
| 277 | IGHV3-21 | IGHV3-30-3 | 377 | IGHD1-26 | IGHJ1 |
| 278 | IGHV3-7 | IGHD1-26 | 378 | IGHD3-22 | IGHJ6 |
| 279 | IGHV3-15 | IGHV3-30-3 | 379 | IGHV1-3 | IGHV7-81 |
| 280 | IGHV4-61 | IGHD1-26 | 380 | IGHV3-23 | IGHD3-22 |
| 281 | IGHD1-26 | IGHD3-22 | 381 | IGHV3-43 | IGHD1-26 |
| 282 | IGHV3-23 | IGHD6-13 | 382 | IGHV3-73 | IGHD3-3 |
| 283 | IGHV3-64D | IGHD3-22 | 383 | IGHD1-20 | IGHD1-26 |
| 284 | IGHD1-26 | IGHD7-27 | 384 | IGHD1-26 | IGHD2-8 |
| 285 | IGHV1-3 | IGHJ6 | 385 | IGHD1-26 | IGHD6-13 |
| 286 | IGHV1-8 | IGHD4-23 | 386 | IGHV1/OR15-9 | IGHV3-64 |
| 287 | IGHV3-30-5 | IGHJ6 | 387 | IGHV1/OR21-1 | IGHV3-30 |
| 288 | IGHV3-64 | IGHD1-26 | 388 | IGHV3-30 | IGHD4-4 |
| 289 | IGHV3-64 | IGHD4/OR15-4a/b | 389 | IGHV3-30 | IGHD4-23 |
| 290 | IGHV4-39 | IGHD1-26 | 390 | IGHD4-23 | IGHD5-12 |
| 291 | IGHD4-23 | IGHJ6 | 391 | IGHV3-9 | IGHD1-26 |
| 292 | IGHV1-8 | IGHD1-26 | 392 | IGHV4-30-2 | IGHD1-26 |
| 293 | IGHV3-30 | IGHD3-22 | 393 | IGHD1-26 | IGHD3-10 |
| 294 | IGHV3-73 | IGHJ6 | 394 | IGHD1-26 | IGHD3/OR15-3a/b |
| 295 | IGHG1 | IGHJ6 | 395 | IGHG1 | IGHD1-26 |
| 296 | IGHV1-69 | IGHD1-26 | 396 | IGHV3-25 | IGHD1-26 |
| 297 | IGHV1/OR15-9 | IGHV3-30 | 397 | IGHV3-30 | IGHD4/OR15-4a/b |
| 298 | IGHV1/OR21-1 | IGHV3-30-5 | 398 | IGHV3-30 | IGHD6-13 |
| 299 | IGHV3-30-5 | IGHV3-43D | 399 | IGHV3-64 | IGHD6-25 |
| 300 | IGHV1-3 | IGHD6-13 | 400 | IGHV3-64D | IGHV3-73 |

[Table 11-7]

| 401- | Gene 1 | Gene 2 | 501- | Gene 1 | Gene 2 |
|---|---|---|---|---|---|
| 401 | IGHV3/OR16-9 | IGHD1-26 | 501 | IGHV1-8 | IGHV3-64D |
| 402 | IGHV3/OR16-13 | IGHD1-26 | 502 | IGHV1/OR15-5 | IGHV4-31 |
| 403 | IGHD3-22 | IGHD6-13 | 503 | IGHV3-9 | IGHJ6 |
| 404 | IGHV1-3 | IGHV3-30 | 504 | IGHV3-23 | IGHV3-43D |
| 405 | IGHV3-11 | IGHD1-26 | 505 | IGHV3-30 | IGHV4-34 |
| 406 | IGHV3-23 | IGHJ6 | 506 | IGHV3-64 | IGHD4-4 |
| 407 | IGHV3-30 | IGHV3-33 | 507 | IGHV3-64 | IGHD4-23 |
| 408 | IGHV3-30 | IGHD4-17 | 508 | IGHV3-64 | IGHD7-27 |
| 409 | IGHV3-38 | IGHD1-26 | 509 | IGHV3/OR15-7 | IGHD4-11 |
| 410 | IGHV5-51 | IGHJ6 | 510 | IGHV5-51 | IGHD6-6 |
| 411 | IGHD3-3 | IGHJ6 | 511 | IGHV1-3 | IGHV3-30-5 |
| 412 | IGHD6-6 | IGHJ6 | 512 | IGHV1-3 | IGHV5-51 |
| 413 | IGHV1-8 | IGHV3-38 | 513 | IGHV1-69 | IGHD4-23 |
| 414 | IGHV1-69D | IGHD1-26 | 514 | IGHV1-69-2 | IGHV5-51 |
| 415 | IGHV3-23 | IGHD6-6 | 515 | IGHV1-69D | IGHV3-23 |
| 416 | IGHV3-30 | IGHD4-11 | 516 | IGHV3-23 | IGHV3-30 |
| 417 | IGHV3-33 | IGHD1-26 | 517 | IGHV3-30-5 | IGHV3-73 |
| 418 | IGHV3-35 | IGHD1-26 | 518 | IGHV3-64 | IGHV3-66 |
| 419 | IGHV4-39 | IGHJ6 | 519 | IGHV3-64 | IGHD3-10 |
| 420 | IGHGP | IGHV3-73 | 520 | IGHV3/OR15-7 | IGHD1/OR15-1a/b |

(continued)

| 401- | Gene 1 | Gene 2 | 501- | Gene 1 | Gene 2 |
|------|--------|--------|------|--------|--------|
| 421 | IGHV1-38-4 | IGHD1-26 | 521 | IGHV4-31 | IGHV5-10-1 |
| 422 | IGHV1/OR15-9 | IGHV4-31 | 522 | IGHV4-31 | IGHD4-23 |
| 423 | IGHV1/OR15-9 | IGHD4-23 | 523 | IGHV4-34 | IGHD1-20 |
| 424 | IGHV3-20 | IGHD1-26 | 524 | IGHV2-5 | IGHV3-30 |
| 425 | IGHV3-30 | IGHV3-38-3 | 525 | IGHV5-10-1 | IGHD6-6 |
| 426 | IGHV3-64D | IGHD6-13 | 526 | IGHV1-69-2 | IGHV3-64 |
| 427 | IGHD1-14 | IGHD1-26 | 527 | IGHV1/OR15-5 | IGHJ6 |
| 428 | IGHD1-26 | IGHJ4 | 528 | IGHV3-30 | IGHD7-27 |
| 429 | IGHV1-8 | IGHV3/OR16-6 | 529 | IGHV3-38 | IGHJ6 |
| 430 | IGHV3-23 | IGHV3-48 | 530 | IGHV3-64 | IGHV4-34 |
| 431 | IGHV3-64 | IGHJ6 | 531 | IGHV3-23 | IGHV4-39 |
| 432 | IGHV3-NL1 | IGHD1-26 | 532 | IGHV3-23 | IGHD4-23 |

[Table 11-8]

| 433 | IGHV1/OR15-5 | IGHD4-23 | 533 | IGHV3-30 | IGHV3/OR16-6 |
|------|--------------|---------|------|----------|----------------|
| 434 | IGHV2-26 | IGHD1-26 | 534 | IGHV3-30-5 | IGHD4-4 |
| 435 | IGHV3-30-5 | IGHV3-64D | 535 | IGHV3-33 | IGHD4-23 |
| 436 | IGHV3-33 | IGHV3-64D | 536 | IGHV3-43D | IGHJ6 |
| 437 | IGHV3-64 | IGHD1/OR15-1a/b | 537 | IGHV3-64 | IGHD4-11 |
| 438 | IGHV3-64 | IGHD4-17 | 538 | IGHV3-64D | IGHD6-6 |
| 439 | IGHV3-64D | IGHV3/OR16-9 | 539 | IGHV3-NL1 | IGHJ6 |
| 440 | IGHV3-64D | IGHV7-81 | 540 | IGHV3/OR15-7 | IGHV3/OR16-10 |
| 441 | IGHV3-73 | IGHD1/0R15-1a/b | 541 | IGHV4-31 | IGHD6-6 |
| 442 | IGHV3-73 | IGHD4-23 | 542 | IGHD1/OR15-1a/b | IGHD4-23 |
| 443 | IGHD1-26 | IGHD2-2 | 543 | IGHD4-4 | IGHD4-23 |
| 444 | IGHD4-11 | IGHD4-23 | 544 | IGHV1-3 | IGHV1/OR15-9 |
| 445 | IGHG1 | IGHV1-3 | 545 | IGHV1-58 | IGHJ6 |
| 446 | IGHV1/OR21-1 | IGHV4-31 | 546 | IGHV1/OR15-9 | IGHV3-23 |
| 447 | IGHV3-23 | IGHD5-12 | 547 | IGHV3-16 | IGHD1/OR15-1a/b |
| 448 | IGHV3-30-5 | IGHD2-21 | 548 | IGHV3-30 | IGHD1-7 |
| 449 | IGHD3-22 | IGHD4-23 | 549 | IGHV3-64 | IGHD1-20 |
| 450 | IGHGP | IGHJ6 | 550 | IGHV3-64D | IGHV4-39 |
| 451 | IGHD4-17 | IGHJ6 | 551 | IGHV3-NL1 | IGHD6-6 |
| 452 | IGHV2-5 | IGHD1-26 | 552 | IGHV3/OR16-13 | IGHJ6 |
| 453 | IGHV3-30-5 | IGHD4-11 | 553 | IGHG1 | IGHV3-64D |
| 454 | IGHV3-64 | IGHD6-13 | 554 | IGHV1-8 | IGHV3-30 |
| 455 | IGHV3/OR15-7 | IGHD4-4 | 555 | IGHV3-30-5 | IGHV5-10-1 |
| 456 | IGHV3/OR16-10 | IGHD1-7 | 556 | IGHV3-72 | IGHD4-23 |
| 457 | IGHV4-4 | IGHJ6 | 557 | IGHV3/OR15-7 | IGHD1-7 |
| 458 | IGHD1-1 | IGHD1-26 | 558 | IGHV1-3 | IGHV1/OR15-5 |
| 459 | IGHD3-22 | IGHD6-6 | 559 | IGHV1-3 | IGHV3-64 |
| 460 | IGHV1-18 | IGHD1-26 | 560 | IGHV1-3 | IGHD3-10 |
| 461 | IGHV1-45 | IGHD1-26 | 561 | IGHV1/OR15-9 | IGHV4-34 |
| 462 | IGHV1-69-2 | IGHJ6 | 562 | IGHV3-9 | IGHV3-30 |
| 463 | IGHV1/OR15-5 | IGHV4-34 | 563 | IGHV3-15 | IGHJ6 |
| 464 | IGHV1/OR15-9 | IGHV3-NL1 | 564 | IGHV3-23 | IGHV3/OR16-8 |
| 465 | IGHV1/OR21-1 | IGHJ6 | 565 | IGHV3-30 | IGHV3-66 |
| 466 | IGHV3-64 | IGHV3/OR16-6 | 566 | IGHV3-30-5 | IGHV3-38-3 |

[Table 11-9]

| | | | | |
|---|---|---|---|---|
| 467 | IGHV3-72 | IGHV3/OR15-7 | 567 | IGHV3-30-5 | IGHD1-7 |
| 468 | IGHV3-74 | IGHD1-26 | 568 | IGHV3/OR15-7 | IGHV5-10-1 |
| 469 | IGHV4-34 | IGHD6-25 | 569 | IGHV1-3 | IGHV3-43D |
| 470 | IGHV4-59 | IGHD1-26 | 570 | IGHV1-3 | IGHD4-23 |
| 471 | IGHV6-1 | IGHD1-26 | 571 | IGHV1/OR15-1 | IGHV3-64 |
| 472 | IGHD5-12 | IGHD6-6 | 572 | IGHV1/OR15-5 | IGHV3-30 |
| 473 | IGHV3-64 | IGHD2-8 | 573 | IGHV3-25 | IGHV3-30 |
| 474 | IGHG4 | IGHV3-30-5 | 574 | IGHV3-43D | IGHV3-64 |
| 475 | IGHGP | IGHV3-64D | 575 | IGHV3-64 | IGHV3/OR15-7 |
| 476 | IGHGP | IGHV4-31 | 576 | IGHV3-64 | IGHV5-10-1 |
| 477 | IGHV1-8 | IGHV3-30-5 | 577 | IGHV3-64 | IGHD2-15 |
| 478 | IGHV1/OR15-5 | IGHV3-30-5 | 578 | IGHV1-3 | IGHV3-48 |
| 479 | IGHV3-64 | IGHD5-12 | 579 | IGHV3-30 | IGHV3-35 |
| 480 | IGHV3/OR16-10 | IGHD1/OR15-1a/b | 580 | IGHV3-30 | IGHV3/OR16-13 |
| 481 | IGHD2-21 | IGHJ6 | 581 | IGHV3-30 | IGHV5-51 |
| 482 | IGHD4-23 | IGHD6-6 | 582 | IGHV3-38 | IGHV3-64 |
| 483 | IGHG4 | IGHD4-23 | 583 | IGHV3-64 | IGHD1-7 |
| 484 | IGHG4 | IGHD6-6 | 584 | IGHV3-73 | IGHD6-6 |
| 485 | IGHGP | IGHV5-51 | 585 | IGHV4-28 | IGHD4-23 |
| 486 | IGHGP | IGHD1-7 | 586 | IGHV4-34 | IGHV5-10-1 |
| 487 | IGHV1-3 | IGHD5-12 | 587 | IGHD4/OR15-4a/b | IGHJ6 |
| 488 | IGHV1-8 | IGHV3-NL1 | 588 | IGHG1 | IGHV3-64 |
| 489 | IGHV1/OR15-9 | IGHJ6 | 589 | IGHG2 | IGHV3-30 |
| 490 | IGHV1/OR21-1 | IGHD2-21 | 590 | IGHGP | IGHV1/OR15-9 |
| 491 | IGHV3-23 | IGHV3-30-5 | 591 | IGHV1-3 | IGHV2/OR16-5 |
| 492 | IGHV3-30 | IGHV3-43D | 592 | IGHV1/OR15-9 | IGHD2-21 |
| 493 | IGHV3-48 | IGHV3-64D | 593 | IGHV1/OR21-1 | IGHV3-64 |
| 494 | IGHV4-34 | IGHD1/OR15-1a/b | 594 | IGHV2-26 | IGHV3-64 |
| 495 | IGHV1-3 | IGHV1/OR21-1 | 595 | IGHV3-30 | IGHV3-30-5 |
| 496 | IGHV1-69-2 | IGHV3-30-5 | 596 | IGHV3-30-5 | IGHV3-64 |
| 497 | IGHV3-30 | IGHV3/OR16-8 | 597 | IGHV3-33 | IGHD1/OR15-1a/b |
| 498 | IGHV3/OR15-7 | IGHD6-6 | 598 | IGHV3-64 | IGHD2-2 |
| 499 | IGHV4-38-2 | IGHD1-26 | 599 | IGHV3-64D | IGHD2-21 |
| 500 | IGHGP | IGHV3-48 | 600 | IGHV4-34 | IGHV7-81 |

[Table 11-10]

| 600- | Gene 1 | Gene 2 |
|---|---|---|
| 600 | IGHV7-81 | IGHJ6 |
| 601 | IGHD5-12 | IGHD6-25 |
| 602 | IGHV3-64 | IGHV7-4-1 |
| 603 | IGHG4 | IGHV3-23 |
| 604 | IGHV1-69-2 | IGHD4-23 |
| 605 | IGHV3-23 | IGHD2-15 |
| 606 | IGHV3-30 | IGHV4-28 |
| 607 | IGHV1-3 | IGHV3/OR16-8 |
| 608 | IGHV1-8 | IGHV3/OR16-9 |
| 609 | IGHV1-8 | IGHD3-22 |
| 610 | IGHV3-23 | IGHV3-64D |
| 611 | IGHV3-30 | IGHV3-38 |

(continued)

| 600- | Gene 1 | Gene 2 |
|---|---|---|
| 612 | IGHV3-30 | IGHD1-14 |
| 613 | IGHV3-33 | IGHD6-6 |
| 614 | IGHV3-48 | IGHV3-64 |
| 615 | IGHV3-64 | IGHJ1 |
| 616 | IGHD3-9 | IGHD4-23 |
| 617 | IGHD6-25 | IGHJ6 |
| 618 | IGHG1 | IGHD6-6 |
| 619 | IGHGP | IGHV3-33 |
| 620 | IGHV1-3 | IGHV5-10-1 |
| 621 | IGHV1-8 | IGHV5-10-1 |
| 622 | IGHV1-45 | IGHV3-30 |
| 623 | IGHV1-58 | IGHV3-30 |
| 624 | IGHV1/OR15-5 | IGHD2-21 |
| 625 | IGHV1/OR15-9 | IGHD6-13 |
| 626 | IGHV3-7 | IGHV3-64D |
| 627 | IGHV3-23 | IGHV3-64 |
| 628 | IGHV3-30 | IGHV3-72 |
| 629 | IGHV3-30 | IGHV3/OR16-9 |
| 630 | IGHV3-30 | IGHV4-30-4 |
| 631 | IGHV3-30-5 | IGHV3/OR16-12 |

[Table 11-11]

| 632 | IGHV3-30-5 | IGHV5-51 |
|---|---|---|
| 633 | IGHV3-30-5 | IGHV7-81 |
| 634 | IGHV3-33 | IGHD6-13 |
| 635 | IGHV3-33 | IGHJ6 |
| 636 | IGHV3-43D | IGHV4-31 |

[Example 5]

Multivariate logistic regression analysis using a plurality of IGH genes

1. Method

Multivariate logistic regression analysis was performed for every combination of any two types of IGH genes from 74 types of IGHV, 32 types of IGHD, 6 types of IGHJ, and 5 types of IGHC for a total of 117 types of IGH genes. ROC curves were created using a prediction value for dependent variables of the regression formula that was obtained to compute an AUC value. Simple regression and logistic regression analysis utilized glm() of R, and ROC analysis utilized pROC of the R package.

2. Results

30 sets (0.44%) out of the total of 6786 sets of combinations exhibited an AUC value of 0.8 or greater in multivariate logistic regression analysis and ROC analysis using any two variables from 117 types of IGH (Table 12). 26 IGH genes were used in the combinations (Table 13). When any three variables were used, 4469 sets (1.7%) out of 260130 sets exhibited an AUC value of 0.8 or greater, 349 sets (0.13%) exhibited an AUC value of 0.85 or greater, and 4 sets (0.0015%) exhibited an AUC value 0.9 or greater (Table 12). IGH genes used in these combinations were 117 genes (100%), 102 genes (87%), and 6 genes (5.1%), respectively (Table 13). When four variables were used, 3264 sets (0.044%) exhibited an AUC value of 0.9 or greater, 99458 sets (1.4%) exhibited an AUC value of 0.85 or greater, and 489529 sets (6.6%) exhibited an AUC value of 0.8 or greater. When four variables were used, at least one of all 117 genes was used in combinations exhibiting an AUC of 0.8 or greater. IGH genes that can be utilized in prediction and differentiation of ME/CFS are shown in Table 14. Combinations of genes with an AUC value of 0.9 or greater that are understood to exhibit high predictive performance are shown in Tables 15 and 16.

[Table 12]

Number of combinations and frequencies of IGH genes exhibiting a high AUC value

| Used variables | Total number | Number of combinations (%) | | |
|---|---|---|---|---|
| | | AUC≥0.9 | AUC≥0.85 | AUC≥0.8 |
| 2 | 6,786 | 0 (0) | 0 (0) | 30 (0.44) |
| 3 | 260,130 | 4 (0.1) | 349 (0.13) | 4,469 (1.72) |
| 4 | 7,413, 705 | 3,264 (0.044) | 99,458 (1.4) | 489,529 (6.6) |

[Table 13]

Table 13 Number and frequency of IGH genes that can be used in a combination exhibiting a high AUC value

| Used variables | Total number | Number of combinations (%) | | |
|---|---|---|---|---|
| | | AUC≥0.9 | AUC≥0.85 | AUC≥0.8 |
| 2 | 117 | 0 (0) | 0 (0) | 26 (22.2) |
| 3 | 117 | 6 (5.1) | 102 (87.2) | 117 (100) |
| 4 | 117 | 117 (100) | 117 (100) | 117 (100) |

[Table 14-1]

Table 14 IGH genes that can be utilized in prediction and differentiation of ME/CFS

| Variables | AUC | List of genes |
|---|---|---|
| 2 | 0.8 or greater | IGHV3-49, IGHV3-30-3, IGHGP, IGHD6-6, IGHV3-30, IGHV3-64D, IGHD1-26, IGHG4, IGHV1-3, IGHV1-69-2, IGHV1/OR15-9, IGHV1/OR21-1, IGHV3-30-5, IGHV3-38, IGHV3-38-3, IGHV3-43D, IGHV3-64, IGHV3-73, IGHV3-NL1, IGHV3/OR16-6, IGHV4-31, IGHD3-22, IGHD4-23, IGHD5-12, IGHD6-13, IGHJ6 (26 genes) |
| 3 | 0.9 or greater | IGHGP,IGHV3-30-3,IGHV3-49,IGHV3-30,IGHD6-6,IGHV3-64D (4 genes) |
| 3 | 0.85 or greater | IGHV3-49, IGHV3-30-3, IGHGP, IGHV1-3, IGHV3-64D, IGHD3-22, IGHD6-6, IGHV3-30-5, IGHD1-26, IGHV3-30, IGHD4-23, IGHV3-38, IGHV3/OR16-6, IGHJ6, IGHV1/OR21-1, IGHV3-73, IGHD4-4, IGHV3-33, IGHV3-64, IGHD4-11, IGHG4, IGHV1-69-2, IGHV3/OR16-8, IGHG1, IGHD5-12, IGHV3-NL1, IGHV4-30-4, IGHV4-34, IGHV1/OR15-5, IGHV1/OR15-9, IGHV2/OR16-5, IGHV3-43D, IGHV3-48, IGHV3/OR16-9, IGHD1/OR15-1a/b, IGHD3-3, IGHD4/OR15-4a/b, IGHV3-15, IGHV3-20, IGHV3-25, IGHD2-2, IGHV3/OR16-13, IGHV4-31, IGHV5-51, IGHD5-18, IGHD5-5, IGHD6-13, IGHV7-81, IGHV3/OR15-7, IGHV3-38-3, IGHV4-39, IGHD1-20, IGHD2-15, IGHJ5, IGHV4-28, IGHV5-10-1, IGHV1-18, IGHV1-58, IGHV1-69D, IGHV1-8, IGHV2-26, IGHV3-21, IGHV3-23D, IGHV3-7, IGHV3-23, IGHV4-30-2, IGHD1-7, IGHD3/OR15-3a/b, IGHD6-25, IGHV3-72, IGHV4-61, IGHG2, IGHG3, IGHV1-2, IGHV1/OR15-1, IGHV3-35, IGHV3-9, IGHD3-16, IGHV1-24, IGHV1-69, IGHV2-5, IGHV2-70, IGHV3-13, IGHV3-16, IGHD1-1, IGHD2-21, IGHD2-8, IGHD3-10, IGHD4-17, IGHD5-24, IGHD5/OR15-5a/b, IGHD6-19, IGHD7-27, IGHJ2, IGHJ3, IGHJ4, IGHV3-43, IGHV3/OR16-12, IGHV4-4, IGHV4-59, IGHV4/OR15-8, IGHV7-4-1 (102 genes) |
| 3 | 0.8 or greater | IGHV3-49, IGHV3-30-3, IGHGP, IGHD1-26, IGHD6-6, IGHV3-64D, IGHV3-30, IGHV1/OR15-9, IGHJ6, IGHV3-30-5, IGHV1-69-2, IGHV3-73, IGHV1/OR21-1, IGHG4, IGHD3-22, IGHV1-3, IGHD6-13, IGHD4/OR15-4a/b, IGHD5-12, IGHV3-64, IGHD4-23, IGHV3-38, IGHV4-31, IGHD4-11, IGHD4-4, IGHV3/OR16-6, IGHV3-43D, IGHD1/OR15-1a/b, IGHV3-NL1, IGHV4-30-4, IGHV1/OR15-5, IGHV3-38-3, IGHV3-23, IGHV3-20, IGHV3/OR16-8 |

[Table 14-2]

IGHV5-10-1, IGHD1-7, IGHV3-33, IGHV4-39, IGHV4-30-2, IGHV3-48, IGHV2-5, IGHV7-81, IGHV3-23D, IGHD3-3, IGHD1-20, IGHG1, IGHD2-21, IGHV3/OR16-13, IGHV3-15, IGHV3/OR16-9, IGHV4-34, IGHV2/OR16-5, IGHD3-16, IGHD4-17, IGHV3-25, IGHD5-5, IGHV4-28, IGHD5-18, IGHD3-10, IGHD6-25, IGHD2-2, IGHV1-8, IGHD2-15, IGHV3-35, IGHV1/OR15-1, IGHV3/OR16-12, IGHV2-70, IGHV3-11, IGHV1-2, IGHD1-14, IGHV4-61, IGHV3/OR15-7, IGHJ3, IGHG3, IGHV3-66, IGHV3-9, IGHV5-51, IGHD2-8, IGHV4-4, IGHJ5, IGHV1-69D, IGHV2-26, IGHG2, IGHV1-58, IGHV3-43, IGHV7-4-1, IGHD1-1, IGHV3-7, IGHV4-59, IGHD7-27, IGHV3-13, IGHV3-16, IGHV3-21, IGHJ2, IGHV1-38-4, IGHV3-72, IGHV4-38-2, IGHV4/OR15-8, IGHD5-24, IGHJ4, IGHV1-18, IGHV1-69, IGHD2/OR15-2a/b, IGHD3-9, IGHD6-19, IGHV1-45, IGHV3/OR16-10, IGHV2-70D, IGHV3-53, IGHV1-24, IGHV1-46, IGHV3-74, IGHD5/OR15-5a/b, IGHV6-1, IGHD3/OR15-3a/b, IGHJ1 (117 genes)

| 4 | 0.9 or greater | The 117 genes described above |
|---|---|---|

[Table 15]

Table 15 Combinations of IGH genes exhibiting high predictive performance (AUC value of 0.9 or greater) (3 types)

| IGH gene 1 | IGH gene 2 | IGH gene 3 | AUC value |
|---|---|---|---|
| IGHGP | IGHV3-30-3 | IGHV3-49 | 0.925969 |
| IGHGP | IGHV3-30-3 | IGHD6-6 | 0.920094 |
| IGHGP | IGHV3-30 | IGHV3-49 | 0.902468 |
| IGHGP | IGHV3-30-3 | IGHV3-64D | 0.900118 |

[Table 16-1]

Table 16 Combinations of IGH genes exhibiting high predictive performance (AUC value of 0.9 or greater) (4 types) (up to 100th combination in order of AUC value)

| IGH gene 1 | IGH gene 2 | IGH gene 3 | IGH gene 4 | AUC value |
|---|---|---|---|---|
| IGHGP | IGHV3-30-3 | IGHV3-49 | IGHD6-6 | 0.963572 |
| IGHGP | IGHV3-30-3 | IGHD6-6 | IGHV3-49 | 0.963572 |
| IGHV3-30-3 | IGHV3-49 | IGHD6-6 | IGHGP | 0.963572 |
| IGHGP | IGHV3-49 | IGHD6-6 | IGHV3-30-3 | 0.963572 |
| IGHGP | IGHV3-30-3 | IGHJ6 | IGHG1 | 0.954172 |
| IGHG1 | IGHV3-30-3 | IGHJ6 | IGHGP | 0.954172 |
| IGHG1 | IGHGP | IGHV3-30-3 | IGHJ6 | 0.954172 |
| IGHG1 | IGHGP | IGHJ6 | IGHV3-30-3 | 0.954172 |
| IGHGP | IGHV3-30-3 | IGHV3-49 | IGHV3-33 | 0.951821 |
| IGHGP | IGHV3-30-3 | IGHD6-6 | IGHV4-28 | 0.951821 |
| IGHV3-30-3 | IGHV3-33 | IGHV3-49 | IGHGP | 0.951821 |
| IGHGP | IGHV3-30-3 | IGHV4-28 | IGHD6-6 | 0.951821 |
| IGHGP | IGHV3-30-3 | IGHV3-33 | IGHV3-49 | 0.951821 |
| IGHV3-30-3 | IGHV4-28 | IGHD6-6 | IGHGP | 0.951821 |
| IGHGP | IGHV4-28 | IGHD6-6 | IGHV3-30-3 | 0.951821 |
| IGHGP | IGHV3-33 | IGHV3-49 | IGHV3-30-3 | 0.951821 |
| IGHGP | IGHV3-30-3 | IGHV3-49 | IGHV3-25 | 0.950646 |
| IGHGP | IGHV3-30-3 | IGHV3-49 | IGHD3-22 | 0.950646 |
| IGHGP | IGHV3-30-3 | IGHV3-49 | IGHJ6 | 0.950646 |
| IGHGP | IGHV3-30-3 | IGHJ6 | IGHV3-49 | 0.950646 |
| IGHGP | IGHV3-25 | IGHV3-30-3 | IGHV3-49 | 0.950646 |
| IGHGP | IGHV3-49 | IGHD3-22 | IGHV3-30-3 | 0.950646 |

(continued)

Table 16 Combinations of IGH genes exhibiting high predictive performance (AUC value of 0.9 or greater) (4 types) (up to 100th combination in order of AUC value)

| IGH gene 1 | IGH gene 2 | IGH gene 3 | IGH gene 4 | AUC value |
|---|---|---|---|---|
| IGHV3-30-3 | IGHV3-49 | IGHD3-22 | IGHGP | 0.950646 |
| IGHGP | IGHV3-30-3 | IGHD3-22 | IGHV3-49 | 0.950646 |
| IGHV3-30-3 | IGHV3-49 | IGHJ6 | IGHGP | 0.950646 |
| IGHV3-25 | IGHV3-30-3 | IGHV3-49 | IGHGP | 0.950646 |
| IGHGP | IGHV3-25 | IGHV3-49 | IGHV3-30-3 | 0.950646 |
| IGHGP | IGHV3-49 | IGHJ6 | IGHV3-30-3 | 0.950646 |
| IGHGP | IGHV3-30-3 | IGHV3-49 | IGHD1-20 | 0.949471 |
| IGHGP | IGHV3-30-3 | IGHD1-20 | IGHV3-49 | 0.949471 |
| IGHGP | IGHV3-49 | IGHD1-20 | IGHV3-30-3 | 0.949471 |

[Table 16-2]

| | IGHV3-30-3 | IGHV3-49 | IGHD1-20 | IGHGP | 0.949471 |
|---|---|---|---|---|---|
| | IGHGP | IGHV3-30-3 | IGHD6-6 | IGHV3-64D | 0.948296 |
| | IGHGP | IGHV3-30 | IGHV3-49 | IGHD6-6 | 0.948296 |
| | IGHGP | IGHV3-30-3 | IGHV3-64D | IGHD6-6 | 0.948296 |
| | IGHGP | IGHV3-30 | IGHD6-6 | IGHV3-49 | 0.948296 |
| | IGHV3-49 | IGHV3-73 | IGHD3-22 | IGHGP | 0.948296 |
| | IGHGP | IGHV3-49 | IGHD3-22 | IGHV3-73 | 0.948296 |
| | IGHGP | IGHV3-49 | IGHD6-6 | IGHV3-30 | 0.948296 |
| | IGHGP | IGHV3-49 | IGHV3-73 | IGHD3-22 | 0.948296 |
| | IGHV3-30-3 | IGHV3-64D | IGHD6-6 | IGHGP | 0.948296 |
| | IGHV3-30 | IGHV3-49 | IGHD6-6 | IGHGP | 0.948296 |
| | IGHGP | IGHV3-64D | IGHD6-6 | IGHV3-30-3 | 0.948296 |
| | IGHGP | IGHV3-73 | IGHD3-22 | IGHV3-49 | 0.948296 |
| | IGHGP | IGHV3-30-3 | IGHV3-49 | IGHV3/OR16-6 | 0.947121 |
| | IGHGP | IGHV3-30-3 | IGHV3-49 | IGHV4-34 | 0.947121 |
| | IGHV3-30-3 | IGHV3-49 | IGHV3/OR16-6 | IGHGP | 0.947121 |
| | IGHGP | IGHV3-30-3 | IGHV4-34 | IGHV3-49 | 0.947121 |
| | IGHGP | IGHV3-49 | IGHV4-34 | IGHV3-30-3 | 0.947121 |
| | IGHGP | IGHV3-30-3 | IGHV3/OR16-6 | IGHV3-49 | 0.947121 |
| | IGHV3-30-3 | IGHV3-49 | IGHV4-34 | IGHGP | 0.947121 |
| | IGHGP | IGHV3-49 | IGHV3/OR16-6 | IGHV3-30-3 | 0.947121 |
| | IGHGP | IGHV3-30-3 | IGHV3-49 | IGHD2-2 | 0.945946 |
| | IGHGP | IGHV3-30-3 | IGHV3-49 | IGHD4-11 | 0.945946 |
| | IGHV3-30-3 | IGHV3-49 | IGHD4-11 | IGHGP | 0.945946 |
| | IGHV3-30-3 | IGHV3-49 | IGHD2-2 | IGHGP | 0.945946 |
| | IGHGP | IGHV3-30-3 | IGHD4-11 | IGHV3-49 | 0.945946 |
| | IGHGP | IGHV3-30-3 | IGHD2-2 | IGHV3-49 | 0.945946 |
| | IGHGP | IGHV3-49 | IGHD4-11 | IGHV3-30-3 | 0.945946 |
| | IGHGP | IGHV3-49 | IGHD2-2 | IGHV3-30-3 | 0.945946 |
| | IGHGP | IGHV3-30-3 | IGHV3-49 | IGHG4 | 0.944771 |
| | IGHGP | IGHV3-30-3 | IGHV3-49 | IGHV3-64D | 0.944771 |
| | IGHGP | IGHV3-30-3 | IGHV3-64D | IGHV3-49 | 0.944771 |
| | IGHG4 | IGHV3-30-3 | IGHV3-49 | IGHGP | 0.944771 |
| | IGHV3-30-3 | IGHV3-49 | IGHV3-64D | IGHGP | 0.944771 |
| | IGHG4 | IGHGP | IGHV3-30-3 | IGHV3-49 | 0.944771 |

[Table 16-3]

| | | | | |
|---|---|---|---|---|
| IGHGP | IGHV3-49 | IGHV3-64D | IGHV3-30-3 | 0.944771 |
| IGHG4 | IGHGP | IGHV3-49 | IGHV3-30-3 | 0.944771 |
| IGHGP | IGHV3-30-3 | IGHV3-49 | IGHV3/OR16-13 | 0.943596 |
| IGHGP | IGHV3-30-5 | IGHV3-49 | IGHV4-34 | 0.943596 |
| IGHGP | IGHV3-30-3 | IGHV3/OR16-13 | IGHV3-49 | 0.943596 |
| IGHV3-30-3 | IGHV3-49 | IGHV3/OR16-13 | IGHGP | 0.943596 |
| IGHGP | IGHV3-49 | IGHV4-34 | IGHV3-30-5 | 0.943596 |
| IGHGP | IGHV3-30-5 | IGHV4-34 | IGHV3-49 | 0.943596 |
| IGHV3-30-5 | IGHV3-49 | IGHV4-34 | IGHGP | 0.943596 |
| IGHGP | IGHV3-49 | IGHV3/OR16-13 | IGHV3-30-3 | 0.943596 |
| IGHGP | IGHV3-30-3 | IGHV3-49 | IGHV2/OR16-5 | 0.942421 |
| IGHGP | IGHV3-30-3 | IGHV3-49 | IGHV3/OR16-8 | 0.942421 |
| IGHGP | IGHV3-30-3 | IGHV3-49 | IGHD4-4 | 0.942421 |
| IGHGP | IGHV3-30-3 | IGHV3-49 | IGHD5-12 | 0.942421 |
| IGHV3-30-3 | IGHV3-49 | IGHD4-4 | IGHGP | 0.942421 |
| IGHGP | IGHV3-30-3 | IGHV3/OR16-8 | IGHV3-49 | 0.942421 |
| IGHGP | IGHV3-30-3 | IGHD5-12 | IGHV3-49 | 0.942421 |
| IGHV3-30-3 | IGHV3-49 | IGHD5-12 | IGHGP | 0.942421 |
| IGHGP | IGHV3-30-3 | IGHD4-4 | IGHV3-49 | 0.942421 |
| IGHGP | IGHV2/OR16-5 | IGHV3-30-3 | IGHV3-49 | 0.942421 |
| IGHV2/OR16-5 | IGHV3-30-3 | IGHV3-49 | IGHGP | 0.942421 |
| IGHV3-30-3 | IGHV3-49 | IGHV3/OR16-8 | IGHGP | 0.942421 |
| IGHGP | IGHV3-49 | IGHV3/OR16-8 | IGHV3-30-3 | 0.942421 |
| IGHGP | IGHV3-49 | IGHD4-4 | IGHV3-30-3 | 0.942421 |
| IGHGP | IGHV2/OR16-5 | IGHV3-49 | IGHV3-30-3 | 0.942421 |
| IGHGP | IGHV3-30-3 | IGHV3-49 | IGHV5-51 | 0.941246 |
| IGHGP | IGHV3-30-3 | IGHD6-6 | IGHD2-2 | 0.941246 |
| IGHGP | IGHV3-30-3 | IGHV5-51 | IGHV3-49 | 0.941246 |
| IGHGP | IGHV3-30-3 | IGHD2-2 | IGHD6-6 | 0.941246 |
| IGHV3-30-3 | IGHV3-49 | IGHV5-51 | IGHGP | 0.941246 |
| IGHV3-30-3 | IGHD2-2 | IGHD6-6 | IGHGP | 0.941246 |
| IGHGP | IGHV3-49 | IGHV5-51 | IGHV3-30-3 | 0.941246 |
| IGHGP | IGHV3-30-3 | IGHV3-49 | IGHV3-38-3 | 0.941246 |
| IGHGP | IGHV3-30-3 | IGHD6-6 | IGHV3-38-3 | 0.941246 |

[Example 6]

Multivariate logistic regression analysis with combination of variables

1. Method

Multivariate logistic regression analysis was performed with a combination of 2 variables, 3 variables, and 4 variables using 46 types of variables (35 types of IGH, 8 types of cell subpopulations, and 3 types of diversity indices) satisfying the significance level of $p < 0.2$ by simple regression analysis. A ROC curve was created using prediction values for dependent variables of a regression formula that was obtained to calculate an AUC value. Simple regression and logistic regression analysis utilized glm() of R, and ROC analysis utilized pROC of the R package.

2. Results

102 sets (9.9%) exhibited an AUC value of 0.8 or greater and 382 sets (36.9%) exhibited an AUC value of 0.7 or greater and less than 0.8 from multivariate logistic regression analysis and ROC analysis using any two variables from the 46 types of selected variables (Table 17). When three variables were used, 85 sets (0.6%) exhibited an AUC value of 0.9 or greater, 3472 sets (22.9%) exhibited an AUC value of 0.8 or greater and less than 0.9, and 8826 sets (58.1%) exhibited an AUC value of 0.7 or greater and less than 0.8. When 4 variables were used, 5330 sets (3.3%) exhibited an AUC value of 0.9 or greater, 63981 sets (39.2%) exhibited an AUC value of 0.8 or greater and less than 0.9, and 87291 sets (53.5%) exhibited an AUC value of 0.7 or greater and less than 0.8. It is understood that the predictive performance of ME/CFS is improved by combining many variables. Table 18 shows combinations exhibiting an AUC value of 0.8 or greater with 2 variables, and Table 19 shows combinations exhibiting an AUC value of 0.9 or greater with 3 variables. Since combinations with Treg accounted for the majority of the top rankings, it is inferred that predictive performance is enhanced by using Treg as a variable.

[Table 17]

Table 17 Number of combinations exhibiting high AUC value from 46 types of selected variables

| Number of variables | Total combinations | Number of combinations (%ratio) | | |
|---|---|---|---|---|
| | | AUC≧0.9 | 0.9>AUC≧0.8 | 0.8>AUC≧0.7 |
| 2 | 1035 | 0 | 102 (9.9) | 382 (36.9) |
| 3 | 15180 | 85 (0.6) | 3472 (22.9) | 8826 (58.1) |
| 4 | 163185 | 5330 (3.3) | 63981 (39.2) | 87291 (53.5) |

[Table 18-1]

Table 18 Predictive performance of ME/CFS patient with a combination of any two variables from 46 types (AUC ≥ 0.8)

| Classification* 1 | Variable 1 | Variable 2 | Number of data | AUC value |
|---|---|---|---|---|
| IGH/FCM | Treg*2 | IGHV1-3 | 43 | 0.891304 |
| IGH/FCM | Treg | IGHV3-23 | 43 | 0.886957 |
| IGH/FCM | Treg | IGHGP | 43 | 0.871739 |
| FCM | Treg | Tfh17 | 43 | 0.869565 |
| FCM | Treg | Tfh2 | 43 | 0.867391 |
| IGH/FCM | Treg | IGHV1/15-9 | 43 | 0.867391 |
| IGH/FCM | IGHV3-49 | Treg | 43 | 0.865217 |
| IGH/FCM | Treg | IGHV3-30-3 | 43 | 0.865217 |
| IGH/FCM | Treg | IGHV3-23D | 43 | 0.863044 |
| IGH/FCM | Treg | IGHV3-64D | 43 | 0.86087 |
| IGH/FCM | Treg | IGHV3-30 | 43 | 0.858696 |
| IGH/FCM | Treg | IGHD3-22 | 43 | 0.858696 |
| IGH/FCM | Treg | IGHV1/15-5 | 43 | 0.858696 |
| IGH/FCM | B-cell | IGHV3-49 | 60 | 0.857814 |
| IGH/FCM | Treg | IGHV3-30-5 | 43 | 0.856522 |
| IGH/FCM | B-cell | IGHV3-30-3 | 60 | 0.854289 |
| IGH/FCM | Treg | IGHV3-64 | 43 | 0.852174 |
| IGH/FCM | Treg | IGHV3-33 | 43 | 0.85 |
| IGH/FCM | Treg | IGHV1-69-2 | 43 | 0.85 |
| IGH | IGHV3-30-3 | IGHGP | 60 | 0.848414 |
| IGH/FCM | Treg | IGHV4-31 | 43 | 0.845652 |
| IGH | IGHV3-49 | IGHV3-30-3 | 60 | 0.843713 |
| IGH/FCM | Treg | IGHD6-6 | 43 | 0.843478 |
| IGH | IGHV3-49 | IGHV3-64D | 60 | 0.842538 |
| IGH/FCM | B-cell | IGHD1-26 | 60 | 0.841363 |
| IGH | IGHV3-49 | IGHV1-3 | 60 | 0.841363 |
| FCM | B-cell | Treg | 43 | 0.841304 |
| IGH/FCM | B-cell | IGHV3-23 | 60 | 0.840188 |
| IGH | IGHV3-49 | IGHD3-22 | 60 | 0.840188 |
| IGH/FCM | Treg | IGHJ6 | 43 | 0.83913 |

(continued)

Table 18 Predictive performance of ME/CFS patient with a combination of any two variables from 46 types (AUC ≥ 0.8)

| Classification* 1 | Variable 1 | Variable 2 | Number of data | AUC value |
|---|---|---|---|---|
| IGH/FCM | Treg | IGHD4/15-4a/b | 43 | 0.83913 |

[Table 18-2]

| | | | | |
|---|---|---|---|---|
| IGH/FCM | Treg | IGHV3-73 | 43 | 0.834783 |
| FCM | Treg | Th17 | 43 | 0.834783 |
| IGH/FCM | Treg | IGHD1-26 | 43 | 0.832609 |
| IGH | IGHV3-49 | IGHV3-30-5 | 60 | 0.831962 |
| IGH/FCM | IGHV3-49 | aN | 50 | 0.830882 |
| IGH | IGHD1-26 | IGHD6-6 | 60 | 0.829612 |
| IGH/FCM | B-cell | IGHGP | 60 | 0.828437 |
| IGH/FCM | Treg | IGHG1 | 43 | 0.828261 |
| IGH/FCM | Treg | IGHV5-51 | 43 | 0.828261 |
| IGH/FCM | Treg | IGHV5-10-1 | 43 | 0.828261 |
| IGH/FCM | Treg | IGHG4 | 43 | 0.828261 |
| IGH/FCM | Treg | IGHV3/16-9 | 43 | 0.828261 |
| FCM | B-cell | Tfh2 | 43 | 0.826087 |
| IGH | IGHV3-49 | IGHD1-26 | 60 | 0.826087 |
| IGH/FCM | Treg | IGHV3-48 | 43 | 0.826087 |
| IGH/FCM | Treg | Inverse | 43 | 0.826087 |
| IGH/FCM | Treg | IGHD4-17 | 43 | 0.826087 |
| IGH/FCM | Treg | IGHV3-21 | 43 | 0.826087 |
| IGH/FCM | B-cell | IGHD3-22 | 60 | 0.824912 |
| IGH/FCM | B-cell | IGHV3-64 | 60 | 0.824912 |
| IGH | IGHV3-30-3 | IGHD6-6 | 60 | 0.824912 |
| FCM | Treg | mB | 43 | 0.823913 |
| FCM | Treg | nB | 43 | 0.823913 |
| FCM/diversity | Treg | Shannon | 43 | 0.823913 |
| IGH/FCM | Treg | IGHV3-43D | 43 | 0.823913 |
| IGH | IGHV3-49 | IGHD4-23 | 60 | 0.823737 |
| IGH/FCM | B-cell | IGHD4-23 | 60 | 0.822562 |
| IGH/FCM | IGHV3-30-3 | Tfh2 | 43 | 0.821739 |
| IGH/FCM | B-cell | IGHV3-64D | 60 | 0.820212 |
| IGH/FCM | Treg | IGHD5-12 | 43 | 0.819565 |
| FCM/diversity | Treg | Pielou | 43 | 0.819565 |
| IGH | IGHV3-49 | IGHV3-30 | 60 | 0.819036 |
| IGH/FCM | B-cell | IGHV3-33 | 60 | 0.817861 |
| IGH/FCM | B-cell | IGHD6-13 | 60 | 0.817861 |

[Table 18-3]

| | | | | |
|---|---|---|---|---|
| IGH/FCM | Treg | IGHD1-7 | 43 | 0.817391 |
| IGH/FCM | B-cell | IGHV3-30 | 60 | 0.816686 |
| IGH/FCM | B-cell | IGHV3-30-5 | 60 | 0.815511 |
| IGH/FCM | B-cell | IGHV3-48 | 60 | 0.815511 |
| IGH | IGHV3-30-3 | IGHJ6 | 60 | 0.815511 |
| IGH/FCM | B-cell | IGHV5-10-1 | 60 | 0.814336 |
| FCM | B-cell | Tfh17 | 43 | 0.813044 |

(continued)

| | | | | |
|---|---|---|---|---|
| IGH/FCM | B-cell | IGHV1-69-2 | 60 | 0.811986 |
| IGH | IGHV3-49 | IGHV3-64 | 60 | 0.811986 |
| IGH/FCM | Treg | IGHD4-23 | 43 | 0.81087 |
| IGH/FCM | IGHV3-30-3 | Th17 | 43 | 0.81087 |
| IGH | IGHV3-49 | IGHV3-73 | 60 | 0.810811 |
| IGH | IGHV3-30-3 | IGHV3-64D | 60 | 0.810811 |
| IGH | IGHV3-30 | IGHGP | 60 | 0.810811 |
| IGH | IGHV3-30-3 | IGHV1/15-9 | 60 | 0.809636 |
| IGH | IGHV3-49 | IGHD6-6 | 60 | 0.808461 |
| IGH | IGHV3-49 | IGHV4-31 | 60 | 0.807286 |
| IGH | IGHV3-30-3 | IGHD6-13 | 60 | 0.807286 |
| IGH | IGHV3-30-3 | IGHD5-12 | 60 | 0.807286 |
| IGH/FCM | Treg | IGHD6-13 | 43 | 0.806522 |
| IGH/FCM | Treg | IGHV4-39 | 43 | 0.806522 |
| IGH/FCM | IGHV3-30-3 | Tfh17 | 43 | 0.806522 |
| IGH/FCM | B-cell | IGHV4-39 | 60 | 0.806111 |
| IGH/FCM | B-cell | IGHJ6 | 60 | 0.804935 |
| IGH/FCM | IGHV3-49 | mB | 60 | 0.804935 |
| IGH/FCM | Tfh17 | IGHD6-6 | 43 | 0.804348 |
| IGH/FCM | IGHV3-49 | nB | 60 | 0.80376 |
| IGH | IGHV3-49 | IGHV1-69-2 | 60 | 0.80376 |
| IGH | IGHV3-49 | IGHG4 | 60 | 0.80376 |
| IGH/FCM | B-cell | IGHD6-6 | 60 | 0.802585 |
| IGH | IGHV3-49 | IGHGP | 60 | 0.802585 |
| IGH | IGHV3-30-3 | IGHV3-43D | 60 | 0.802585 |
| IGH/FCM | IGHV3-30-5 | Tfh2 | 43 | 0.802174 |
| IGH/FCM | B-cell | IGHV3-23D | 60 | 0.80141 |

[Table 18-4]

| | | | | |
|---|---|---|---|---|
| IGH/FCM | B-cell | IGHD4/15-4a | 60 | 0.80141 |
| IGH/FCM | B-cell | IGHG4 | 60 | 0.80141 |
| IGH/FCM | B-cell | IGHD5-12 | 60 | 0.800235 |

*1: IGH/FCM: composite of IGH variable and cell subpopulation, IGH: only IGH variable, FCM: only cell subpopulation variable *2: Treg is indicated by an underline

[Table 19-1]

Table 19 Prediction of ME/CFS patient with combination of any 3 variables from 46 types (AUC ≥ 0.9)

| Classification* 1 | Variable 1 | Variable 2 | Variable 3 | Data | AUC value |
|---|---|---|---|---|---|
| IGH/FCM | Treg | IGHGP | IGHV1-3 | 43 | 0.952174 |
| IGH/FCM | Treg | IGHGP | IGHV3-23 | 43 | 0.936957 |
| IGH/FCM | Treg | IGHV3-30-3 | IGHGP | 43 | 0.934783 |
| IGH/FCM | Treg | IGHGP | IGHV3-30-5 | 43 | 0.932609 |
| IGH/FCM | Treg | IGHV3-33 | IGHV3-23 | 43 | 0.932609 |
| IGH/FCM | Treg | IGHD3-22 | IGHV3-23 | 43 | 0.930435 |
| IGH/FCM | Treg | IGHV1-3 | IGHV1/OR15-9 | 43 | 0.930435 |
| IGH/FCM | Treg | IGHV3-30 | IGHGP | 43 | 0.930435 |
| IGH/FCM | Treg | IGHV3-30-3 | IGHV1/OR15-9 | 43 | 0.928261 |

(continued)

Table 19 Prediction of ME/CFS patient with combination of any 3 variables from 46 types (AUC ≥ 0.9)

| Classification* 1 | Variable 1 | Variable 2 | Variable 3 | Data | AUC value |
|---|---|---|---|---|---|
| IGH/FCM | IGHV3-49 | Treg | IGHV1-3 | 43 | 0.928261 |
| IGH/FCM | Treg | IGHV3-23 | IGHV1/OR15-9 | 43 | 0.926087 |
| IGH | IGHV3-49 | IGHV3-30-3 | IGHGP | 60 | 0.925969 |
| IGH/FCM | Treg | IGHV1-3 | IGHV1/OR15-5 | 43 | 0.923913 |
| IGH/FCM | Treg | IGHV1-3 | IGHD4/OR15-4a/b | 43 | 0.923913 |
| IGH/FCM | Treg | IGHV3-30-3 | IGHV3-64D | 43 | 0.921739 |
| IGH | IGHV3-30-3 | IGHGP | IGHD6-6 | 60 | 0.920094 |
| IGH/FCM | Treg | Tfh2 | IGHV3-64 | 43 | 0.919565 |
| IGH/FCM | Treg | IGHV3-30-5 | IGHV1/OR15-9 | 43 | 0.919565 |
| IGH/FCM | IGHV3-49 | IGHD3-22 | aN | 50 | 0.919118 |
| IGH/FCM | B-cell | IGHV3-49 | IGHD3-22 | 60 | 0.918919 |
| IGH/FCM | B-cell | Treg | Tfh2 | 43 | 0.917391 |
| IGH/FCM | Treg | IGHD3-22 | Tfh17 | 43 | 0.917391 |
| IGH/FCM | Treg | IGHV1-3 | IGHV3-64D | 43 | 0.917391 |
| IGH/FCM | Treg | Tfh17 | IGHV1/OR15-9 | 43 | 0.917391 |
| IGH/FCM | IGHV3-49 | Treg | IGHD3-22 | 43 | 0.917391 |

[Table 19-2]

| | | | | | |
|---|---|---|---|---|---|
| IGH/FCM | Treg | IGHD6-6 | IGHV3-23 | 43 | 0.915217 |
| IGH/FCM | Treg | IGHD3-22 | IGHV1/OR15-9 | 43 | 0.915217 |
| IGH/FCM | Treg | IGHV3-30-3 | IGHJ6 | 43 | 0.915217 |
| IGH/FCM | B-cell | Treg | IGHV3-23 | 43 | 0.913044 |
| IGH/FCM | Treg | IGHV3-30 | IGHV1/OR15-9 | 43 | 0.913044 |
| IGH/FCM | Treg | IGHV1-3 | Tfh2 | 43 | 0.91087 |
| IGH/FCM | Treg | IGHV1-3 | Tfh17 | 43 | 0.91087 |
| IGH/FCM | Treg | IGHV3-23 | IGHV3-64D | 43 | 0.91087 |
| IGH/FCM | Treg | IGHV3-30 | IGHV3-33 | 43 | 0.91087 |
| IGH/FCM | Treg | IGHV3-23 | Tfh2 | 43 | 0.908696 |
| IGH/FCM | Treg | IGHD3-22 | IGHV3-64D | 43 | 0.908696 |
| IGH/FCM | IGHV3-49 | Treg | IGHV3-30-3 | 43 | 0.908696 |
| IGH/FCM | IGHV3-49 | Treg | IGHV3-23 | 43 | 0.908696 |
| IGH/FCM | Treg | IGHV3-30-3 | IGHV3-23 | 43 | 0.908696 |
| IGH/FCM | Treg | IGHV3-30-5 | IGHV1/OR15-5 | 43 | 0.908696 |
| IGH/FCM | Treg | IGHV3-23 | IGHV1/OR15-5 | 43 | 0.908696 |
| IGH/FCM | Treg | IGHV3-30-3 | IGHV1-3 | 43 | 0.908696 |
| IGH/FCM | Treg | IGHV3-64D | Tfh2 | 43 | 0.906522 |
| IGH/FCM | Treg | Tfh17 | IGHV3-64D | 43 | 0.906522 |
| IGH/FCM | Treg | IGHV3-23 | IGHV3-64 | 43 | 0.906522 |
| IGH/FCM | Treg | IGHD3-22 | IGHV3-33 | 43 | 0.906522 |
| IGH/FCM | Treg | IGHV3-30-3 | IGHV1/OR15-5 | 43 | 0.906522 |
| IGH/FCM | B-cell | Treg | IGHV1-3 | 43 | 0.906522 |
| IGH/FCM | Treg | IGHV3-23 | IGHD6-13 | 43 | 0.906522 |
| IGH/FCM | Treg | IGHV3-23 | Th17 | 43 | 0.904348 |
| IGH/FCM | Treg | IGHV3-23 | IGHV3-48 | 43 | 0.904348 |
| IGH | IGHV3-49 | IGHV3-30 | IGHGP | 60 | 0.902468 |
| IGH/FCM | IGHV3-49 | IGHD3-22 | Th17 | 43 | 0.902174 |
| IGH/FCM | Treg | IGHV3-23 | IGHV4-31 | 43 | 0.902174 |

(continued)

| | | | | | |
|---|---|---|---|---|---|
| IGH/FCM | Treg | Tfh2 | IGHV4-31 | 43 | 0.902174 |
| IGH/FCM | Treg | Tfh2 | IGHV3-73 | 43 | 0.902174 |
| IGH/FCM | Treg | IGHGP | IGHV3-64D | 43 | 0.902174 |
| IGH/FCM | Treg | IGHV1-3 | IGHV3-48 | 43 | 0.902174 |
| IGH/FCM | Treg | IGHV3-23 | IGHV3-43D | 43 | 0.902174 |

[Table 19-3]

| | | | | | |
|---|---|---|---|---|---|
| IGH/FCM | Treg | IGHV3-30-3 | IGHV3-33 | 43 | 0.902174 |
| IGH/FCM | Treg | IGHV1-3 | IGHV3-23D | 43 | 0.902174 |
| IGH/FCM | Treg | Tfh17 | IGHV3-23 | 43 | 0.902174 |
| IGH/FCM | Treg | IGHD4-23 | IGHV3-23 | 43 | 0.902174 |
| IGH/FCM | Treg | IGHV3-23 | IGHV3-21 | 43 | 0.902174 |
| IGH/FCM | Treg | IGHV3-23D | IGHV1/OR15-9 | 43 | 0.902174 |
| IGH/FCM | Treg | IGHV1-3 | IGHV1-69-2 | 43 | 0.902174 |
| IGH/FCM | Treg | Tfh17 | IGHV1-69-2 | 43 | 0.902174 |
| IGH/FCM | Treg | IGHV3-23 | IGHG1 | 43 | 0.902174 |
| IGH/FCM | Treg | IGHGP | IGHD6-6 | 43 | 0.902174 |
| IGH | IGHV3-30-3 | IGHGP | IGHV3-64D | 60 | 0.900118 |
| IGH/FCM | Treg | IGHGP | Tfh17 | 43 | 0.9 |
| IGH/FCM /diversity | Treg | IGHGP | Pielou | 43 | 0.9 |
| IGH/FCM | Treg | IGHV1-3 | Inverse | 43 | 0.9 |
| IGH/FCM | Treg | IGHV1-3 | IGHV4-39 | 43 | 0.9 |
| IGH/FCM | Treg | IGHV3-64 | IGHV3/OR16-9 | 43 | 0.9 |
| IGH/FCM | Treg | IGHD3-22 | IGHV3-64 | 43 | 0.9 |
| IGH/FCM | IGHV3-49 | Treg | IGHV3-30-5 | 43 | 0.9 |
| IGH/FCM | B-cell | Treg | IGHV3-30-3 | 43 | 0.9 |
| IGH/FCM | Treg | IGHGP | IGHV3-23D | 43 | 0.9 |
| IGH/FCM | Treg | IGHV1-3 | IGHV3-21 | 43 | 0.9 |
| IGH/FCM | Treg | IGHV3-23 | IGHV1-69-2 | 43 | 0.9 |
| IGH/FCM | Treg | Tfh17 | IGHG4 | 43 | 0.9 |
| IGH/FCM | Treg | IGHV1-3 | IGHD6-6 | 43 | 0.9 |
| IGH/FCM | Treg | IGHD3-22 | IGHD6-6 | 43 | 0.9 |
| IGH/FCM | Treg | IGHV3-23 | IGHD4/OR15-4a/b | 43 | 0.9 |

*1: IGH/FCM: composite of IGH variable and cell subpopulation, IGH: only IGH variable, FCM: only cell subpopulation variable *2: Treg is indicated by an underline

While the maximum value of AUC was 0.87 (Treg, Tfh17) for combinations of cell subpopulation variables, an AUC exceeding 0.87 was obtained when combining a cell subpopulation variable with an IGH gene in Treg+IGHV1-3, Treg+IGHV3-23, and Treg+IGHGP. Further, a B cell count, when combined with IGHV3-49, IGHV3-30-3, or IGHD1-26, resulted in a higher AUC value than the maximum AUC = 0.84 in a combination with Treg. Higher predictive performance can be attained by using a specific IGH gene in addition to a cell subpopulation variable than performance attained by a combination of cell subpopulation variables.

[Example 7]

Differentiation of ME/CFS patient from patient of another disease

1. Method

In addition to the samples from 37 myalgic encephalomyelitis/chronic fatigue syndrome (ME/CFS) patients in Example 1, whole blood was newly collected from 10 multiple sclerosis (MS) patients. PBMC was separated and RNA was extracted in accordance with the method described in 1. Materials and Methods of Example 1. Subsequently, complementary DNA and double stranded complementary DNA were synthesized, and subjected to PCR, next generation sequence analysis, and then analysis with repertoire analysis software. From the read data of each sample, the IGH gene V region sequence (IGHV), D region sequence (IGHD), J region sequence (IGHJ), and C region sequence (IGHC) were collated, and CDR3 sequences were determined. The number of unique reads for each sample was tallied, and usage frequencies for 74 types of IGHV, 32 types of IGHD, 6 types of IGHJ, and 5 types of IGHC were computed. Significance test was conducted on read data and IGHV, IGHD, IGHJ, and IGHC usage frequencies using Mann-Whitney test between the ME/CFS and MS groups. Simple regression and logistic regression analysis utilized glm of R, and ROC analysis utilized pROC of the R package, by using usage frequency data for ME/CFS patients and MS patients. A Receiver Operating Characteristic (ROC) curve was created using prediction values for dependent variables of a regression formula in ROC analysis. The ROC curve Area Under the Curve (AUC) value was found as a performance evaluation value for the prediction and determination of these variables.

2. Results

2-1. Difference in IGH gene usage frequencies between ME/CFS patients and MS patients

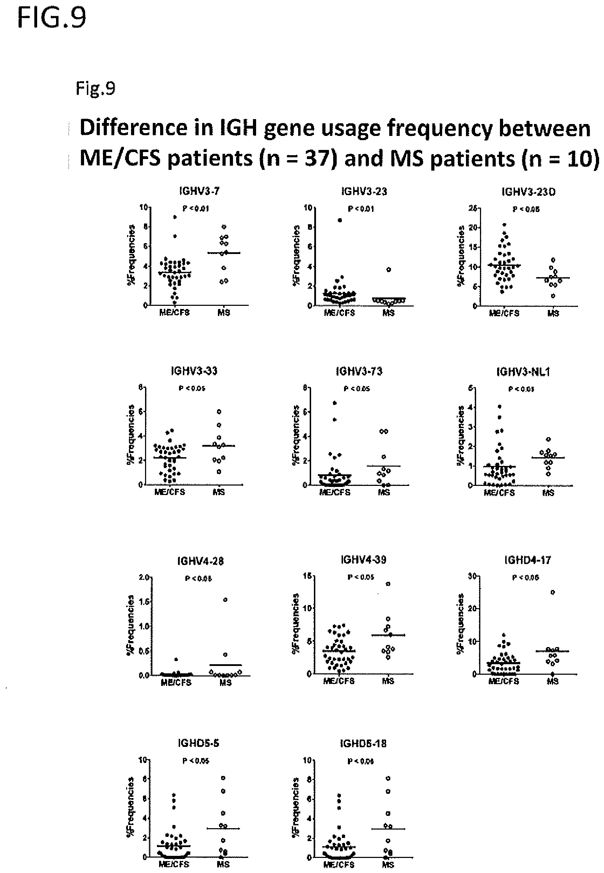

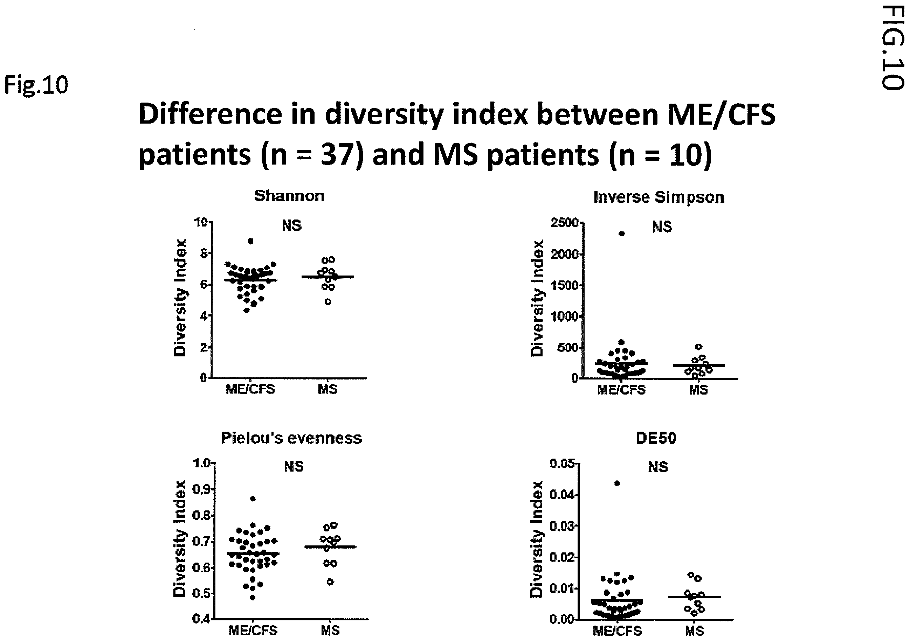

Sequence data for 140 to 280 thousand reads was obtained from 10 MS patient samples (Table 20). A significant difference was not found between ME/CFS patients and MS patients in the total number of reads, assigned reads, number of in-frame reads, and number of unique reads. As a result, IGH gene usage frequency was significantly higher in MS patients compared to ME/CFS patients in IGHV3-7**, IGHV3-33*, IGHV3-73*, IGHV3-NL1*, IGHV4-28*, IGHV4-39*, IGHD4-17*, IGHD5-5*, and IGHD5-18* (Mann-Whitney test: *P < 0.05, **P < 0.01). Meanwhile, significantly lower usage frequencies of IGHV3-23** and IGHV3-23D* were exhibited in MS patients compared to ME/CFS patients (Figure **9).** Similarly, variables other than IGH gene such as diversity indices (Shannon index, inverse Simpson index, Pielou's index, and DE50 index) were studied as to whether there is a difference between ME/CFS patients and MS patients (Figure **10).** A significant difference was found in any of the diversity indices between ME/CFS patients and MS patients. These results show that diversity indices are not useful in the differentiation of ME/CFS patients from MS patients, but usage frequencies of IGH genes reflect disease specificity and are useful in differentiation.

[Table 20]

| Number of reads | Number of reads (mean ± SD) | | P value |
|---|---|---|---|
| | ME/CFS patients (37 cases) | MS patients (10 cases) | |
| Total reads | 289342.6 ± 112471.3 | 271898.7 ± 47747.6 | 0.47 |
| Assigned reads | 162337.5 ± 40656.5 | 148545.3 ± 35911 | 0.31 |
| In-frame reads | 158459 ± 39046.6 | 145064.7 ± 35436.6 | 0.32 |
| Unique reads | 16936.5 ± 9067.3 | 14962.8 ± 4643.3 | 0.35 |

2-2. Differentiation of ME/CFS patients from MS patients using multivariate logistic analysis

Next, the above 11 types of IGH genes detected to have a significant difference between ME/CFS patients and MS patients were studied as to whether the genes can differentiate ME/CFS patients from MS patients by using multivariate logistic analysis. Logistic regression analysis was performed utilizing usage frequencies of any two types of IGH genes or any three types of IGH genes among the 11 types of IGH genes to compute an AUC value. Combinations with a high AUC value were listed as combinations of IGH genes with excellent predictive performance (Tables 21 and 22) .

[Table 21]

Table 21 Differentiation using combinations of any two variables of IGH genes found to have a significant difference between ME/CFS groups and MS groups (AUC ≥ 0.8)

| IGH gene 1 | IGH gene 2 | Number of data | AUC value |
|---|---|---|---|
| IGHD5-18 | IGHV4-39 | 47 | 0.845946 |
| IGHD5-5 | IGHV4-39 | 47 | 0.843243 |
| IGHV3-73 | IGHV4-28 | 47 | 0.837838 |
| IGHD5-5 | IGHV3-23D | 47 | 0.835135 |
| IGHD5-18 | IGHV3-23D | 47 | 0.835135 |

(continued)

Table 21 Differentiation using combinations of any two variables of IGH genes found to have a significant difference between ME/CFS groups and MS groups (AUC $\geq$ 0.8)

| IGH gene 1 | IGH gene 2 | Number of data | AUC value |
|---|---|---|---|
| IGHD5-18 | IGHV3-7 | 47 | 0.824324 |
| IGHD5-5 | IGHV3-7 | 47 | 0.821622 |
| IGHV3-23D | IGHV3-7 | 47 | 0.816216 |
| IGHV3-23 | IGHV3-7 | 47 | 0.813514 |
| IGHV3-7 | IGHV4-39 | 47 | 0.813514 |
| IGHD4-17 | IGHV3-7 | 47 | 0.813514 |
| IGHV3-23 | IGHV4-28 | 47 | 0.810811 |
| IGHV3-23D | IGHV3-NL1 | 47 | 0.808108 |
| IGHV3-NL1 | IGHV4-28 | 47 | 0.808108 |
| IGHV3-7 | IGHV3-73 | 47 | 0.805405 |
| IGHV3-73 | IGHV3-NL1 | 47 | 0.802703 |
| IGHV3-7 | IGHV4-28 | 47 | 0.8 |

[Table 22]

Table 22 Differentiation using combinations of any three variables of IGH genes found to have a significant difference between ME/CFS groups and MS groups (AUC $\geq$ 0.85)

| IGH gene 1 | IGH gene 2 | IGH gene 3 | Number of data | AUC value |
|---|---|---|---|---|
| IGHD5-5 | IGHV3-7 | IGHV4-39 | 47 | 0.908108 |
| IGHD5-18 | IGHV3-7 | IGHV4-39 | 47 | 0.908108 |
| IGHD5-18 | IGHV3-23D | IGHV3-7 | 47 | 0.883784 |
| IGHD5-18 | IGHV3-23D | IGHV4-39 | 47 | 0.878378 |
| IGHD5-5 | IGHV3-23D | IGHV3-7 | 47 | 0.875676 |
| IGHD5-5 | IGHV3-23D | IGHV4-39 | 47 | 0.872973 |
| IGHD5-5 | IGHV3-7 | IGHV3-73 | 47 | 0.867568 |
| IGHD5-18 | IGHV3-7 | IGHV3-73 | 47 | 0.867568 |
| IGHD5-18 | IGHV3-73 | IGHV4-39 | 47 | 0.867568 |
| IGHD5-18 | IGHD5-5 | IGHV3-23D | 47 | 0.864865 |
| IGHD5-5 | IGHV3-33 | IGHV4-39 | 47 | 0.862162 |
| IGHD5-5 | IGHV3-73 | IGHV4-39 | 47 | 0.862162 |
| IGHD4-17 | IGHD5-5 | IGHV4-39 | 47 | 0.862162 |
| IGHD5-18 | IGHV3-33 | IGHV4-39 | 47 | 0.859459 |
| IGHD4-17 | IGHD5-18 | IGHV4-39 | 47 | 0.859459 |
| IGHD5-18 | IGHV3-NL1 | IGHV4-39 | 47 | 0.856757 |
| IGHD5-18 | IGHV4-28 | IGHV4-39 | 47 | 0.856757 |
| IGHD5-18 | IGHV3-23 | IGHV4-39 | 47 | 0.854054 |
| IGHD5-18 | IGHV3-23D | IGHV4-28 | 47 | 0.854054 |
| IGHD5-5 | IGHV3-NL1 | IGHV4-39 | 47 | 0.854054 |
| IGHD5-18 | IGHV3-23 | IGHV3-7 | 47 | 0.851351 |
| IGHD5-5 | IGHV4-28 | IGHV4-39 | 47 | 0.851351 |

Multi-variable logistic analysis was performed to study whether ME/CFS patients can be differentiated from MS patients using any two or three variables for IGH gene that were demonstrated to be capable of differentiating healthy individuals from ME/CFs patients (Tables 23 to 30). As a result, a large number of combinations with a high AUC value exhibiting high multivariate logistic model performance was obtained, which were found to be capable of predicting ME/CFS patients and MS patients with high specificity and sensitivity. When any two variables of 35 types of IGH genes exhibiting a significant difference at p < 0.2 between healthy individuals and ME/CFS patients were used, 48 combinations

exhibited AUC $\geq$ 0.8, and when any three variables were used, 21 combinations exhibited AUC $\geq$ 0.9. When any two or three variables of 18 types of IGH genes exhibiting a significant difference at p < 0.1 between healthy individuals and ME/CFS patients were used, 17 combinations (AUC $\geq$ 0.8) and 21 combinations (AUC $\geq$ 0.875) exhibited a high value, respectively. When any two or three variables of 8 types of IGH genes exhibiting a significant difference at p < 0.05 between healthy individuals and ME/CFS patients were used, 7 combinations (AUC $\geq$ 0.8) and 22 combinations (AUC $\geq$ 0.8) exhibited a high value, respectively. When any two or three variables of 6 types of IGH genes tested in Example 3-1 were used, 1 combination (AUC $\geq$ 0.7) and 11 combinations (AUC $\geq$ 0.7) exhibited a high value, respectively. The above results revealed that ME/CFS patients and MS patients can be differentiated with high accuracy by using a single or a plurality of IGH gene usage frequencies.

[Table 23-1]

Table 23 Differentiation of ME/CFS from MS with combination of any two variables from IGHs exhibiting a significant difference at p < 0.2 between healthy individuals and ME/CFS patients (AUC $\geq$ 0.8)

| IGH gene 1 | IGH gene 2 | Number of data | AUC value |
|---|---|---|---|
| IGHGP | IGHV4-31 | 47 | 0.886486 |
| IGHGP | IGHV3-49 | 47 | 0.87027 |
| IGHG1 | IGHGP | 47 | 0.864865 |
| IGHGP | IGHV3-30 | 47 | 0.864865 |
| IGHGP | IGHV1-69-2 | 47 | 0.862162 |
| IGHD4-OR15-4a/b | IGHGP | 47 | 0.862162 |
| IGHGP | IGHV3-23D | 47 | 0.859459 |
| IGHD4-17 | IGHGP | 47 | 0.859459 |
| IGHGP | IGHV5-10-1 | 47 | 0.859459 |
| IGHGP | IGHV3-64D | 47 | 0.854054 |
| IGHD4-17 | IGHV3-30 | 47 | 0.851351 |
| IGHGP | IGHV3-OR16-9 | 47 | 0.851351 |
| IGHGP | IGHV3-30-5 | 47 | 0.851351 |
| IGHD5-12 | IGHGP | 47 | 0.848649 |
| IGHD3-22 | IGHV3-23D | 47 | 0.845946 |
| IGHG4 | IGHGP | 47 | 0.843243 |
| IGHD1-7 | IGHGP | 47 | 0.843243 |
| IGHGP | IGHV3-30-3 | 47 | 0.843243 |
| IGHGP | IGHV3-33 | 47 | 0.840541 |
| IGHGP | IGHJ6 | 47 | 0.840541 |
| IGHGP | IGHV4-39 | 47 | 0.835135 |
| IGHD4-23 | IGHGP | 47 | 0.832432 |
| IGHGP | IGHV3-48 | 47 | 0.832432 |
| IGHGP | IGHV3-64 | 47 | 0.832432 |
| IGHGP | IGHV1-3 | 47 | 0.832432 |
| IGHGP | IGHV5-51 | 47 | 0.82973 |
| IGHGP | IGHV1-OR15-9 | 47 | 0.82973 |
| IGHGP | IGHV3-23 | 47 | 0.82973 |
| IGHD1-26 | IGHGP | 47 | 0.82973 |
| IGHD3-22 | IGHGP | 47 | 0.82973 |
| IGHV4-31 | IGHV4-39 | 47 | 0.827027 |

[Table 23-2]

| | | | |
|---|---|---|---|
| IGHV3-30 | IGHV4-39 | 47 | 0.827027 |
| IGHGP | IGHV3-43D | 47 | 0.827027 |
| IGHV1-OR15-5 | IGHV3-23 | 47 | 0.827027 |
| IGHGP | IGHV1-OR15-5 | 47 | 0.827027 |

(continued)

| | | | |
|---|---|---|---|
| IGHGP | IGHV3-21 | 47 | 0.827027 |
| IGHD6-13 | IGHGP | 47 | 0.827027 |
| IGHGP | IGHV3-73 | 47 | 0.824324 |
| IGHV4-39 | IGHV5-10-1 | 47 | 0.821622 |
| IGHV3-30-5 | IGHV3-73 | 47 | 0.818919 |
| IGHV3-49 | IGHV4-31 | 47 | 0.818919 |
| IGHD6-6 | IGHGP | 47 | 0.818919 |
| IGHV3-23 | IGHV5-10-1 | 47 | 0.808108 |
| IGHV4-31 | IGHV5-10-1 | 47 | 0.808108 |
| IGHV1-69-2 | IGHV4-39 | 47 | 0.802703 |
| IGHV3-23D | IGHV3-30 | 47 | 0.802703 |
| IGHV3-23D | IGHV4-31 | 47 | 0.8 |
| IGHD4-17 | IGHV3-30-3 | 47 | 0.8 |

[Table 24]

Table 24 Differentiation of ME/CFS from MS with combination of any three variables from IGHs exhibiting a significant difference at p < 0.2 between healthy individuals and ME/CFS patients (AUC $\geq$ 0.9)

| IGH gene 1 | IGH gene 2 | IGH gene 3 | Number of data | AUC value |
|---|---|---|---|---|
| IGHD4-17 | IGHGP | IGHV3-30 | 47 | 0.943243 |
| IGHGP | IGHV3-30 | IGHV3-30-5 | 47 | 0.937838 |
| IGHD4-17 | IGHV3-23D | IGHV3-30 | 47 | 0.927027 |
| IGHGP | IGHV4-31 | IGHV5-10-1 | 47 | 0.921622 |
| IGHGP | IGHV3-49 | IGHV4-31 | 47 | 0.918919 |
| IGHG1 | IGHGP | IGHV4-31 | 47 | 0.916216 |
| IGHGP | IGHV3-64D | IGHV4-31 | 47 | 0.910811 |
| IGHD3-22 | IGHV1-OR155 | IGHV3-23D | 47 | 0.910811 |
| IGHD4-OR15-4a/b | IGHGP | IGHV4-31 | 47 | 0.908108 |
| IGHGP | IGHV3-30 | IGHV4-31 | 47 | 0.908108 |
| IGHD4-17 | IGHV3-30 | IGHV3-73 | 47 | 0.905405 |
| IGHD4-OR15-4a/b | IGHGP | IGHV3-49 | 47 | 0.905405 |
| IGHGP | IGHV1-69-2 | IGHV4-31 | 47 | 0.902703 |
| IGHD4-17 | IGHGP | IGHV4-31 | 47 | 0.902703 |
| IGHGP | IGHV1-OR15-5 | IGHV4-31 | 47 | 0.902703 |
| IGHD1-7 | IGHGP | IGHV4-31 | 47 | 0.902703 |
| IGHGP | IGHV3-33 | IGHV4-31 | 47 | 0.902703 |
| IGHD3-22 | IGHV3-23D | IGHV3-33 | 47 | 0.902703 |
| IGHGP | IGHV3-23D | IGHV3-30 | 47 | 0.902703 |
| IGHV3-49 | IGHV4-31 | IGHV5-10-1 | 47 | 0.9 |
| IGHG1 | IGHGP | IGHV3-23D | 47 | 0.9 |

[Table 25]

Table 25 Differentiation of ME/CFS from MS with combination of any two variables from IGHs exhibiting a significant difference at p < 0.1 between healthy individuals and ME/CFS patients (AUC $\geq$ 0.8)

| IGH gene 1 | IGH gene 2 | Number of data | AUC value |
|---|---|---|---|
| IGHGP | IGHV3-49 | 47 | 0.87027 |
| IGHG1 | IGHGP | 47 | 0.864865 |
| IGHGP | IGHV3-30 | 47 | 0.864865 |
| IGHGP | IGHV3-64D | 47 | 0.854054 |

(continued)

Table 25 Differentiation of ME/CFS from MS with combination of any two variables from IGHs exhibiting a significant difference at p < 0.1 between healthy individuals and ME/CFS patients (AUC ≥ 0.8)

| IGH gene 1 | IGH gene 2 | Number of data | AUC value |
|---|---|---|---|
| IGHGP | IGHV3-30-5 | 47 | 0.851351 |
| IGHGP | IGHV3-30-3 | 47 | 0.843243 |
| IGHGP | IGHV3-33 | 47 | 0.840541 |
| IGHGP | IGHJ6 | 47 | 0.840541 |
| IGHD4-23 | IGHGP | 47 | 0.832432 |
| IGHGP | IGHV3-48 | 47 | 0.832432 |
| IGHGP | IGHV3-64 | 47 | 0.832432 |
| IGHGP | IGHV1-3 | 47 | 0.832432 |
| IGHGP | IGHV3-23 | 47 | 0.82973 |
| IGHD1-26 | IGHGP | 47 | 0.82973 |
| IGHD3-22 | IGHGP | 47 | 0.82973 |
| IGHD6-13 | IGHGP | 47 | 0.827027 |
| IGHD6-6 | IGHGP | 47 | 0.818919 |

[Table 26]

Table 26 Differentiation of ME/CFS from MS with combination of any three variables from IGHs exhibiting a significant difference at p < 0.1 between healthy individuals and ME/CFS patients (AUC ≥ 0.85)

| IGH gene 1 | IGH gene 2 | IGH gene 3 | Number of data | AUC value |
|---|---|---|---|---|
| IGHGP | IGHV3-30 | IGHV3-30-5 | 47 | 0.937838 |
| IGHD6-6 | IGHG1 | IGHGP | 47 | 0.894595 |
| IGHG1 | IGHGP | IGHV3-30-5 | 47 | 0.891892 |
| IGHG1 | IGHGP | IGHV3-23 | 47 | 0.891892 |
| IGHG1 | IGHGP | IGHV3-30 | 47 | 0.889189 |
| IGHGP | IGHV3-30-5 | IGHV3-64D | 47 | 0.886486 |
| IGHGP | IGHV3-30 | IGHV3-49 | 47 | 0.886486 |
| IGHGP | IGHV3-30 | IGHV3-33 | 47 | 0.883784 |
| IGHGP | IGHV3-30-3 | IGHV3-30-5 | 47 | 0.883784 |
| IGHGP | IGHV3-30 | IGHV3-64D | 47 | 0.881081 |
| IGHG1 | IGHGP | IGHV3-48 | 47 | 0.881081 |
| IGHD6-6 | IGHGP | IGHV3-49 | 47 | 0.878378 |
| IGHG1 | IGHGP | IGHV3-33 | 47 | 0.878378 |
| IGHD3-22 | IGHGP | IGHV3-33 | 47 | 0.878378 |
| IGHD4-23 | IGHG1 | IGHGP | 47 | 0.878378 |
| IGHGP | IGHV3-23 | IGHV3-49 | 47 | 0.878378 |
| IGHG1 | IGHGP | IGHV3-49 | 47 | 0.878378 |
| IGHG1 | IGHGP | IGHV3-64D | 47 | 0.875676 |
| IGHGP | IGHV3-49 | IGHV3-64D | 47 | 0.875676 |
| IGHGP | IGHV3-30 | IGHV3-48 | 47 | 0.875676 |
| IGHGP | IGHV1-3 | IGHV3-49 | 47 | 0.875676 |

[Table 27]

Table 27 Differentiation of ME/CFS from MS with combination of any two variables from IGHs exhibiting a significant difference at p < 0.05 between healthy individuals and ME/CFS patients (AUC ≥ 0.8)

| IGH gene 1 | IGH gene 2 | Number of data | AUC value |
|---|---|---|---|
| IGHGP | IGHV3-49 | 47 | 0.87027 |
| IGHGP | IGHV3-30 | 47 | 0.864865 |
| IGHGP | IGHV3-30-3 | 47 | 0.843243 |
| IGHGP | IGHJ6 | 47 | 0.840541 |
| IGHGP | IGHV1-3 | 47 | 0.832432 |
| IGHD1-26 | IGHGP | 47 | 0.82973 |
| IGHD3-22 | IGHGP | 47 | 0.82973 |

[Table 28]

Table 28 Differentiation of ME/CFS from MS with combination of any three variables from IGHs exhibiting a significant difference at p < 0.05 between healthy individuals and ME/CFS patients (AUC ≥ 0.8)

| IGH gene 1 | IGH gene 2 | IGH gene 3 | Number of data | AUC value |
|---|---|---|---|---|
| IGHGP | IGHV3-30 | IGHV3-49 | 47 | 0.886486 |
| IGHGP | IGHV1-3 | IGHV3-49 | 47 | 0.875676 |
| IGHD1-26 | IGHGP | IGHV3-49 | 47 | 0.87027 |
| IGHGP | IGHJ6 | IGHV3-49 | 47 | 0.867568 |
| IGHGP | IGHV3-30 | IGHV3-30-3 | 47 | 0.867568 |
| IGHGP | IGHV3-30-3 | IGHV3-49 | 47 | 0.864865 |
| IGHGP | IGHV1-3 | IGHV3-30 | 47 | 0.864865 |
| IGHD3-22 | IGHGP | IGHV3-49 | 47 | 0.862162 |
| IGHD1-26 | IGHGP | IGHV3-30 | 47 | 0.862162 |
| IGHGP | IGHJ6 | IGHV3-30 | 47 | 0.859459 |
| IGHD3-22 | IGHGP | IGHV3-30 | 47 | 0.851351 |
| IGHD1-26 | IGHGP | IGHJ6 | 47 | 0.840541 |
| IGHGP | IGHJ6 | IGHV1-3 | 47 | 0.840541 |
| IGHGP | IGHV1-3 | IGHV3-30-3 | 47 | 0.837838 |
| IGHD1-26 | IGHGP | IGHV3-30-3 | 47 | 0.832432 |
| IGHD3-22 | IGHGP | IGHV3-30-3 | 47 | 0.832432 |
| IGHD3-22 | IGHGP | IGHJ6 | 47 | 0.832432 |
| IGHD1-26 | IGHGP | IGHV1-3 | 47 | 0.82973 |
| IGHD1-26 | IGHD3-22 | IGHGP | 47 | 0.82973 |
| IGHD3-22 | IGHGP | IGHV1-3 | 47 | 0.82973 |
| IGHD1-26 | IGHD3-22 | IGHV3-49 | 47 | 0.818919 |
| IGHGP | IGHJ6 | IGHV3-30-3 | 47 | 0.818919 |

[Table 29]

Table 29 Differentiation of ME/CFS from MS with combination of any two variables from 6 types of IGHs tested in Example 3-1 (AUC ≥ 0.7)

| IGH gene 1 | IGH gene 2 | Number of data | AUC value |
|---|---|---|---|
| IGHV3-30 | IGHV3-49 | 47 | 0.735135 |

[Table 30]

Table 30 Differentiation of ME/CFS from MS with combination of any three variables from 6 types of IGHs tested in Example 3-1 (AUC ≥ 0.7)

| IGH gene 1 | IGH gene 2 | IGH gene 3 | Number of data | AUC value |
|---|---|---|---|---|
| IGHD1-26 | IGHV3-30 | IGHV3-49 | 47 | 0.764865 |
| IGHV1-3 | IGHV3-30 | IGHV3-49 | 47 | 0.737838 |
| IGHD1-26 | IGHV1-3 | IGHV3-49 | 47 | 0.737838 |
| IG HV3-30 | IGHV3-30-3 | IGHV3-49 | 47 | 0.737838 |
| IGHD1-26 | IGHJ6 | IGHV3-49 | 47 | 0.737838 |
| IGHJ6 | IGHV3-30 | IGHV3-49 | 47 | 0.735135 |
| IGHD1-26 | IGHV3-30-3 | IGHV3-49 | 47 | 0.716216 |
| IGHJ6 | IGHV3-30-3 | IGHV3-49 | 47 | 0.710811 |
| IGHD1-26 | IGHJ6 | IGHV3-30 | 47 | 0.710811 |
| IGHJ6 | IGHV1-3 | IGHV3-49 | 47 | 0.705405 |
| IGHD1-26 | IGHJ6 | IGHV3-30-3 | 47 | 0.705405 |

2-3. Differentiation of ME/CFS patients from MS patients by multivariate logistic analysis using any IGH gene

Polynomial logistic analysis was performed using combinations of any two types of IGH from 74 types of IGHV, 32 types of IGHD, 6 types of IGHJ, and 5 types of IGHC for a total of 117 types of IGH genes (total of 6786 sets of combinations), and those with a high AUC value indicating high predictive performance were selected (Table 31). There were 4, 252, and 1879 sets of combinations exhibiting AUC ≥ 0.9, AUC ≥ 0.8, and AUC ≥ 0.7, respectively. Two variables IGHD3-3 and IGHGP showed the highest AUC of 0.92. Next, polynomial logistic analysis was performed using combinations of any three types of IGH from 117 types of IGH genes (total of 260130 sets of combinations), and those with a high AUC value indicating high predictive performance were selected (Table 32).

[Table 31-1]

Table 31 Differentiation of ME/CFS from MS with combination of any two IGH genes (top 50)

| Rank | IGH gene 1 | IGH gene 2 | Number of data | AUC value |
|---|---|---|---|---|
| 1 | IGHD3-3 | IGHGP | 47 | 0.921622 |
| 2 | IGHGP | IGHV4-34 | 47 | 0.913514 |
| 3 | IGHD2-21 | IGHGP | 47 | 0.910811 |
| 4 | IGHGP | IGHV3-72 | 47 | 0.902703 |
| 5 | IGHGP | IGHV4-38-2 | 47 | 0.9 |
| 6 | IGHD1-1 | IGHGP | 47 | 0.894595 |
| 7 | IGHD3-3 | IGHV3-7 | 47 | 0.891892 |
| 8 | IGHD3-OR15-3a/b | IGHGP | 47 | 0.889189 |
| 9 | IGHGP | IGHV4-31 | 47 | 0.886486 |
| 10 | IGHGP | IGHV3-7 | 47 | 0.883784 |
| 11 | IGHV3-7 | IGHV4-38-2 | 47 | 0.883784 |
| 12 | IGHD2-2 | IGHGP | 47 | 0.881081 |
| 13 | IGHD5-5 | IGHGP | 47 | 0.878378 |
| 13 | IGHD5-18 | IGHGP | 47 | 0.878378 |
| 15 | IGHV3-7 | IGHV3-72 | 47 | 0.875676 |
| 15 | IGHD3-22 | IGHV3-7 | 47 | 0.875676 |
| 17 | IGHGP | IGHV4-30-2 | 47 | 0.872973 |
| 18 | IGHD3-3 | IGHV4-39 | 47 | 0.87027 |
| 19 | IGHGP | IGHV3-49 | 47 | 0.87027 |
| 19 | IGHGP | IGHV3/OR16-6 | 47 | 0.87027 |
| 19 | IGHGP | IGHV4-28 | 47 | 0.87027 |
| 19 | IGHGP | IGHJ1 | 47 | 0.87027 |
| 19 | IGHV1-46 | IGHV3-7 | 47 | 0.87027 |

(continued)

Table 31 Differentiation of ME/CFS from MS with combination of any two IGH genes (top 50)

| Rank | IGH gene 1 | IGH gene 2 | Number of data | AUC value |
|---|---|---|---|---|
| 24 | IGHGP | IGHV3 43 | 47 | 0.867568 |
| 24 | IGHGP | IGHV4-61 | 47 | 0.867568 |
| 26 | IGHG1 | IGHGP | 47 | 0.864865 |

[Table 31-2]

| | | | | |
|---|---|---|---|---|
| 31 | IGHGP | IGHV3/OR16-10 | 47 | 0.859459 |
| 31 | IGHGP | IGHV5-10-1 | 47 | 0.359455 |
| 31 | IGHD1-14 | IGHGP | 47 | 0.859459 |
| 31 | IGHD4-17 | IGHGP | 47 | 0.859459 |
| 31 | IGHGP | IGHJ3 | 47 | 0.359459 |
| 31 | IGHV1-69-2 | IGHV3-7 | 47 | 0.859459 |
| 38 | IGHGP | IGHV3/OR15-7 | 47 | 0.856757 |
| 38 | IGHD1/OR15-1a/b | IGHGP | 47 | 0.856757 |
| 40 | IGHGP | IGHV2-26 | 47 | 0.854054 |
| 40 | IGHGP | IGHV3-64D | 47 | 0.854054 |
| 40 | IGHJ4 | IGHV3-7 | 47 | 0.854054 |
| 43 | IGHGP | IGHV3-30-5 | 47 | 0.851351 |
| 44 | IGHG3 | IGHGP | 47 | 0.851351 |
| 44 | IGHGP | IGHV3/OR16-9 | 47 | 0.851351 |
| 44 | IGHGP | IGHV4/OR15-8 | 47 | 0.851351 |
| 44 | IGHD4-17 | IGHV3-30 | 47 | 0.351351 |
| 44 | IGHJ1 | IGHV4-39 | 47 | 0.851351 |
| 49 | IGHGP | IGHV1-45 | 47 | 0.848649 |
| 49 | IGHGP | IGHV3/OR16-12 | 47 | 0.848649 |
| 49 | IGHGP | IGHV4-59 | 47 | 0.848649 |
| 49 | IGHD5-12 | IGHGP | 47 | 0.848649 |
| 49 | IGHGP | IGHJ4 | 47 | 0.848649 |

[Table 32-1]

Table 31 Differentiation of ME/CFS from MS with combination of any three IGH genes (top 50)

| Rank | IGH gene 1 | IGH gene 2 | IGH gene 3 | Number of data | AUC value |
|---|---|---|---|---|---|
| 1 | IGHD1.14 | IGHD3.3 | IGHGP | 47 | 0.989189 |
| 2 | IGHD3.3 | IGHGP | IGHV3.38.3 | 47 | 0.983784 |
| 3 | IGHGP | IGHV3.7 | IGHV3.72 | 47 | 0.978378 |
| 4 | IGHD5.5 | IGHGP | IGHV4.34 | 47 | 0.975676 |
| 4 | IGHD5.18 | IGHGP | IGHV4.34 | 47 | 0.975676 |
| 6 | IGHD1.1 | IGHGP | IGHV4.34 | 47 | 0.972973 |
| 7 | IGHGP | IGHV3.64D | IGHV4.34 | 47 | 0.967568 |
| 8 | IGHD3.3 | IGHGP | IGHV3.72 | 47 | 0.964865 |
| 8 | IGHD2.21 | IGHD3.3 | IGHGP | 47 | 0.964865 |
| 10 | IGHD2.21 | IGHGP | IGHV4.31 | 47 | 0.962162 |
| 10 | IGHD2.21 | IGHGP | IGHV4.34 | 47 | 0.962162 |
| 12 | IGHGP | IGHV3.30 | IGHV4.34 | 47 | 0.959459 |
| 12 | IGHD3.3 | IGHGP | IGHV3.30 | 47 | 0.959459 |
| 12 | IGHGP | IGHV4.34 | IGHV5.10.1 | 47 | 0.959459 |

(continued)

Table 31 Differentiation of ME/CFS from MS with combination of any three IGH genes (top 50)

| Rank | IGH gene 1 | IGH gene 2 | IGH gene 3 | Number of data | AUC value |
|---|---|---|---|---|---|
| 12 | IGHGP | IGHJ1 | IGHV4.34 | 47 | 0.959459 |
| 12 | IGHV3.64 | IGHV3.7 | IGHV3.72 | 47 | 0.959459 |
| 12 | IGHD3.3 | IGHV3.30 | IGHV4.39 | 47 | 0.959459 |
| 18 | IGHD3.3 | IGHGP | IGHV3.49 | 47 | 0.956757 |
| 19 | IGHD3.3 | IGHGP | IGHV3.7 | 47 | 0.954054 |
| 19 | IGHGP | IGHV3.64 | IGHV4.34 | 47 | 0.954054 |
| 19 | IGHGP | IGHV3.OR15.7 | IGHV4.34 | 47 | 0.954054 |
| 19 | IGHGP | IGHJ1 | IGHV4.28 | 47 | 0.954054 |
| 19 | IGHD3.OR15.3a | IGHGP | IGHV4.34 | 47 | 0.954054 |
| 19 | IGHD2.2 | IGHD2.21 | IGHGP | 47 | 0.954054 |
| 25 | IGHD3.3 | IGHGP | IGHV2.5 | 47 | 0.951351 |
| 25 | IGHGP | IGHV3.49 | IGHV4.34 | 47 | 0.951351 |
| 25 | IGHD3.3 | IGHV3.64 | IGHV3.7 | 47 | 0.951351 |
| 25 | IGHD3.22 | IGHV3.7 | IGHV3.72 | 47 | 0.951351 |
| 25 | IGHD3.22 | IGHV3.7 | IGHV4.38.2 | 47 | 0.951351 |
| 30 | IGHGP | IGHV3.43 | IGHV4.34 | 47 | 0.948649 |
| 30 | IGHD2.21 | IGHGP | IGHV3.43 | 47 | 0.948649 |

[Table 32-2]

| | Rank | IGH gene 1 | IGH gene 2 | IGH gene 3 | Number of data | AUC value |
|---|---|---|---|---|---|---|
| | 30 | IGHD2.21 | IGHGP | IGHV4.38.2 | 47 | 0.948649 |
| | 33 | IGHD3.3 | IGHG1 | IGHGP | 47 | 0.948649 |
| | 33 | IGHGP | IGHV1.46 | IGHV4.34 | 47 | 0.948649 |
| | 33 | IGHD2.21 | IGHGP | IGHV3.23D | 47 | 0.948649 |
| | 33 | IGHD1.1 | IGHGP | IGHV3.72 | 47 | 0.948649 |
| | 33 | IGHGP | IGHV4.34 | IGHV4.38.2 | 47 | 0.948649 |
| | 38 | IGHG1 | IGHGP | IGHV4.34 | 47 | 0.945946 |
| | 38 | IGHD2.21 | IGHGP | IGHV3.7 | 47 | 0.945946 |
| | 38 | IGHGP | IGHV3.72 | IGHV4.34 | 47 | 0.945946 |
| | 38 | IGHD3.3 | IGHGP | IGHV3.OR16.13 | 47 | 0.945946 |
| | 38 | IGHD3.3 | IGHGP | IGHV4.34 | 47 | 0.945946 |
| | 38 | IGHGP | IGHJ3 | IGHV4.34 | 47 | 0.945946 |
| | 38 | IGHD3.3 | IGHGP | IGHV4.38.2 | 47 | 0.945946 |
| | 38 | IGHD3.3 | IGHV2.5 | IGHV4.39 | 47 | 0.945946 |
| | 46 | IGHD2.21 | IGHG1 | IGHGP | 47 | 0.943243 |
| | 46 | IGHD3.3 | IGHGP | IGHV3.25 | 47 | 0.943243 |
| | 46 | IGHD4.17 | IGHGP | IGHV3.30 | 47 | 0.943243 |
| | 46 | IGHGP | IGHV3.53 | IGHV4.34 | 47 | 0.943243 |
| | 46 | IGHD3.3 | IGHGP | IGHV3.64D | 47 | 0.943243 |
| | 46 | IGHD2.21 | IGHGP | IGHV3.72 | 47 | 0.943243 |
| | 46 | IGHGP | IGHV3.OR15.7 | IGHV4.38.2 | 47 | 0.943243 |
| | 46 | IGHGP | IGHV3.OR16.6 | IGHV4.34 | 47 | 0.943243 |
| | 46 | IGHGP | IGHV3.OR16.6 | IGHV4.38.2 | 47 | 0.943243 |
| | 46 | IGHD3.3 | IGHGP | IGHV4.61 | 47 | 0.943243 |
| | 46 | IGHD1.1 | IGHD2.2 | IGHGP | 47 | 0.943243 |
| | 46 | IGHD1.1 | IGHD3.3 | IGHGP | 47 | 0.943243 |
| | 46 | IGHD2.21 | IGHGP | IGHJ3 | 47 | 0.943243 |
| | 46 | IGHV3.7 | IGHV3.OR15.7 | IGHV4.38.2 | 47 | 0.943243 |

(continued)

| 46 | IGHD2.8 | IGHD3.3 | IGHV3.7 | 47 | 0.943243 |
| 46 | IGHD3.3 | IGHJ1 | IGHV4.39 | 47 | 0.943243 |

2-4. Comparison of ME/CFS patients with non-ME/CFS patients

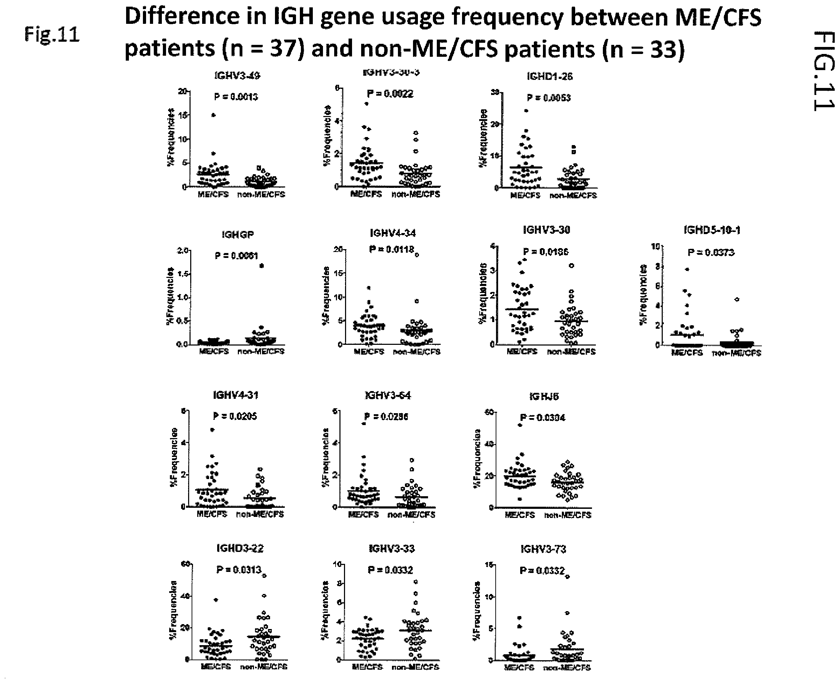

In order to study IGH genes that differentiate ME/CFS patients from non-ME/CFS patients, 23 healthy individuals were added to 10 MS patients as the non-ME/CFS group, and the usage frequencies of IGH genes were compared between said group and a group of 37 ME/CFS patients. Mann-Whitney test between the two groups elucidated that 9 genes, i.e., IGHV3-49 (P = 0.0013), IGHV3-30-3 (P = 0.0022), IGHD1-26 (P = 0.0053), IGHV4-34 (P = 0.0118), IGHV3-30 (P = 0.186), IGHV4-31 (P = 0.0205), IGHV3-64 (P = 0.0286), IGHJ6 (P = 0.0304), and IGHD5-10-1 (P = 0.0373), were higher in ME/CFS patients compared to non-ME/CFS patients. Meanwhile, IGHGP (P = 0.0061), IGHD3-22 (P = 0.0313), IGHV3-33 (P = 0.0332), and IGHV3-73 (P = 0.0332) were significantly higher in the non-ME/CFS patient group (Table 33, Figure **11**).

[Table 33]

Table 33 IGH genes detected to have a significant difference between ME/CFS group and ME/CFS group

| IGH gene | Mean value | | Standard deviation | | |
|---|---|---|---|---|---|
| | **Non-ME/CFS** | ME/CFS | **Non-ME-CFS** | ME/CFS | **P value\*** |
| IGHV3-49 | 1.11 | 2.63 | 0.99 | 2.63 | 0.001 |
| IGHV3 30 3 | 0.81 | 1.44 | 0.75 | 1.05 | 0.002 |
| IGHD1-26 | 2.88 | 6.31 | 3.23 | 5.84 | 0.005 |
| IGHGP | 0.13 | 0.03 | 0.29 | 0.03 | 0.006 |
| IGHV4-34 | 3.06 | 3.97 | 3.37 | 2.45 | 0.011 |
| IGHV3-30 | 0.96 | 1.43 | 0.67 | 0.87 | 0.018 |
| IGHV4-31 | 0.56 | 1.06 | 0.67 | 1.08 | 0.020 |
| IGHV3-64 | 0.67 | 1.04 | 0.70 | 0.99 | 0.028 |
| IGHJ6 | 16.00 | 20.06 | 6.10 | 7.87 | 0.030 |
| IGHD3-22 | 14.36 | 8.91 | 11.61 | 7.21 | 0.031 |
| IGHV3-33 | 3.08 | 2.21 | 1.82 | 1.09 | 0.033 |
| IGHV3-73 | 1.82 | 0.81 | 2.69 | 1.45 | 0.033 |
| IGHV5-10-1 | 0.34 | 1.04 | 0.91 | 1.85 | 0.037 |

*Mann-Whitney test

2-5. Differentiation of ME/CFS patients from non-ME/CFS patients by multivariate logistic analysis using any IGH gene

Polynomial logistic regression analysis was performed using any two IGHs from 74 types of IGHV, 32 types of IGHD, 6 types of IGHJ, and 5 types of IGHC for a total of 117 types of IGH genes (total of 6786 combinations), and those with a high AUC value indicating high predictive performance in differentiating ME/CFS patients from non-ME/CFS patients were selected (Table 34). IGHs exhibiting a high AUC value when using three variables (total of 260130 sets of combinations) were also selected in the same manner (Table 35). With any two variables, there were 5 and 469 sets of combinations exhibiting AUC $\geq$ 0.8 and AUC $\geq$ 0.7, respectively. With any three variables, there were 37 and 1164 sets of combinations exhibiting AUC $\geq$ 0.85 and AUC $\geq$ 0.8, respectively. With two variables, the combination of IGHGP and IGH3-30-3 showed the highest AUC of 0.820. With any three variables, the combination of IGHGP, IGHV3-30, and IGHV3-49 showed the highest AUC of 0.889.

[Table 34-1]

Table 34 Differentiation of ME/CFS from non-ME/CFS with combination of any two variables (top 50)

| Rank | IGH gene 1 | IGH gene 2 | Number of data | AUC value |
|---|---|---|---|---|
| 1 | IGHGP | IGHV3-30-3 | 70 | 0.81982 |
| 1 | IGHD3-22 | IGHV3-49 | 70 | 0.81982 |
| 3 | IGHGP | IGHV3-30 | 70 | 0.812449 |
| 4 | IGHGP | IGHV3-49 | 70 | 0.804259 |

(continued)

Table 34 Differentiation of ME/CFS from non-ME/CFS with combination of any two variables (top 50)

| Rank | IGH gene 1 | IGH gene 2 | Number of data | AUC value |
|---|---|---|---|---|
| 5 | IGHGP | IGHV4-34 | 70 | 0.801802 |
| 6 | IGHV3-49 | IGHV4-31 | 70 | 0.799345 |
| 7 | IGHV3-49 | IGHV3-64D | 70 | 0.796888 |
| 8 | IGHV3-30-3 | IGHV3-49 | 70 | 0.796069 |
| 9 | IGHD1-26 | IGHD6-6 | 70 | 0.790336 |
| 10 | IGHV1-3 | IGHV3-49 | 70 | 0.788698 |
| 10 | IGHV3-30 | IGHV3-49 | 70 | 0.788698 |
| 12 | IGHD6-6 | IGHGP | 70 | 0.78706 |
| 13 | IGHD4-17 | IGHV3-30-3 | 70 | 0.786241 |
| 14 | IGHD1-26 | IGHV3-49 | 70 | 0.782146 |
| 14 | IGHD4-23 | IGHV3-49 | 70 | 0.782146 |
| 16 | IGHD3-3 | IGHV3-49 | 70 | 0.781327 |
| 17 | IGHV1-69-2 | IGHV3-49 | 70 | 0.779689 |
| 18 | IGHV3-49 | IGHV3-73 | 70 | 0.777232 |
| 19 | IGHG4 | IGHV3-49 | 70 | 0.775594 |
| 19 | IGHV1/OR15-9 | IGHV3-30-3 | 70 | 0.775594 |
| 21 | IGHV3-30-3 | IGHV3-64D | 70 | 0.774775 |
| 22 | IGHV3-49 | IGHV3-7 | 70 | 0.773956 |
| 22 | IGHJ6 | IGHV3-30-3 | 70 | 0.773956 |
| 22 | IGHV3-33 | IGHV3-49 | 70 | 0.773956 |
| 25 | IGHD3-22 | IGHV3-30-3 | 70 | 0.773137 |
| 26 | IGHV3-49 | IGHV3-64 | 70 | 0.773137 |
| 26 | IGHV3-49 | IGHV4-30-4 | 70 | 0.773137 |
| 28 | IGHGP | IGHV4-31 | 70 | 0.771499 |
| 29 | IGHD1-26 | IGHV3-73 | 70 | 0.769861 |
| 30 | IGHV3-30-3 | IGHV4-28 | 70 | 0.768223 |
| 31 | IGHD6-6 | IGHV3-49 | 70 | 0.765766 |

[Table 34-2]

| 32 | IGHV1/OR21-1 | IGHV3-49 | 70 | 0.764947 |
|---|---|---|---|---|
| 33 | IGHGP | IGHV3-23 | 70 | 0.764128 |
| 34 | IGHV1-69-2 | IGHV3-30-3 | 70 | 0.763309 |
| 34 | IGHV3-49 | IGHV4-39 | 70 | 0.763309 |
| 36 | IGHV1/OR15-5 | IGHV3-49 | 70 | 0.76249 |
| 37 | IGHG4 | IGHV3-30-3 | 70 | 0.761671 |
| 38 | IGHV3-30-3 | IGHV3-43D | 70 | 0.761671 |
| 39 | IGHV3-49 | IGHV5-10-1 | 70 | 0.760852 |
| 40 | IGHV3-30-3 | IGHV4-31 | 70 | 0.760033 |
| 40 | IGHD3-3 | IGHV3-30-3 | 70 | 0.760033 |
| 42 | IGHD1-20 | IGHGP | 70 | 0.759214 |
| 43 | IGHV3-49 | IGHV3/CH16-12 | 70 | 0.758395 |
| 43 | IGHV3-49 | IGHV4-30-2 | 70 | 0.758395 |
| 45 | IGHD6-6 | IGHV3-30-3 | 70 | 0.757576 |
| 46 | IGHV1/OR15-9 | IGHV3-49 | 70 | 0.756757 |
| 46 | IGHV3-30-5 | IGHV3-49 | 70 | 0.756757 |
| 48 | IGHV3-49 | IGHV3/OR16-13 | 70 | 0.755938 |
| 49 | IGHV3-20 | IGHV3-49 | 70 | 0.755119 |

(continued)

| | | | | |
|---|---|---|---|---|
| 49 | IGHJ4 | IGHV3-30-3 | 70 | 0.755119 |
| 49 | IGHD4/OR15-4a/b | IGHV3-49 | 70 | 0.755119 |

[Table 35-1]

Table 35 Differentiation of ME/CFS from non-ME/CFS with combination of any three variables (top 50)

| Rank | IGH gene 1 | IGH gene 2 | IGH gene 3 | Number of data | AUC value |
|---|---|---|---|---|---|
| 1 | IGHGP | IGHV3-30 | IGHV3-49 | 70 | 0.8886 |
| 2 | IGHGP | IGHV3-30 | IGHV4-34 | 70 | 0.8878 |
| 2 | IGHGP | IGHV3-30-3 | IGHV3-49 | 70 | 0.8878 |
| 4 | IGHGP | IGHV3-30-3 | IGHV4-28 | 70 | 0.8763 |
| 4 | GHD6-6 | IGHGP | IGHV3-30-3 | 70 | 0.8763 |
| 6 | IGHGP | IGHV3-30 | IGHV3-72 | 70 | 0.8731 |
| 6 | IGHD1-20 | IGHGP | IGHV3-49 | 70 | 0.8731 |
| 8 | IGHV3-49 | IGHV3-64D | IGHV3-7 | 70 | 0.8714 |
| 9 | IGHD4-17 | IGHGP | IGHV3-30 | 70 | 08690 |
| 10 | IGHD6-6 | IGHGP | IGHV3-30 | 70 | 0.8673 |
| 11 | IGHD3-22 | IGHGP | IGHV3-49 | 70 | 0.8657 |
| 12 | IGHD3-3 | IGHGP | IGHV3-30 | 70 | 0.8649 |
| 12 | IGHV3-49 | IGHV3-64D | IGHV4-31 | 70 | 0.8649 |
| 12 | IGHD3-22 | IGHV3-49 | IGHV3-73 | 70 | 0.8649 |
| 15 | IGHGP | IGHV3-30-3 | IGHV4-34 | 70 | 0.8624 |
| 16 | IGHD3-22 | IGHV3-49 | IGHV4-31 | 70 | 08616 |
| 17 | IGHGP | IGHV3-30-3 | IGHV3-72 | 70 | 0.8608 |
| 17 | IGHD3-22 | IGHV1-69-2 | IGHV3-49 | 70 | 0.8608 |
| 19 | IGHGP | IGHV3-30 | IGHV4-28 | 70 | 0.8591 |
| 20 | IGHD3-22 | IGHV3-49 | IGHV3-64D | 70 | 0.8583 |
| 21 | IGHD1-20 | IGHGP | IGHV3-30 | 70 | 0.8575 |
| 21 | IGHD3-3 | IGHGP | IGHV3-30-3 | 70 | 0.8575 |
| 21 | IGHGP | IGHV3-49 | IGHV4-34 | 70 | 0.8575 |
| 24 | IGHD2-21 | IGHGP | IGHV3-30-3 | 70 | 0.8559 |
| 25 | IGHGP | IGHJ6 | IGHV3-30-3 | 70 | 0.8550 |
| 25 | IGHGP | IGHV3-64D | IGHV4-34 | 70 | 0.8550 |
| 27 | IGHGP | IGHV3-30-3 | IGHV3/OR16-13 | 70 | 0.8542 |
| 27 | IGHGP | IGHV3-49 | IGHV4-31 | 70 | 0.8542 |
| 27 | IGHD3-22 | IGHD4/OR15-4a/b | IGHV3-49 | 70 | 0.8542 |
| 30 | IGHGP | IGHV3-49 | IGHV3-73 | 70 | 0.8534 |
| 31 | IGHD6-6 | IGHGP | IGHV3-49 | 70 | 0.8518 |

[Table 35-2]

| | | | | | |
|---|---|---|---|---|---|
| 31 | IGHV1-69-2 | IGHV3-49 | IGHV4-31 | 70 | 0.8518 |
| 33 | IGHGP | IGHV3-30 | IGHV3/OR16-8 | 70 | 0.8509 |
| 33 | IGHD4-17 | IGHGP | IGHV3-3C-3 | 70 | 0.8509 |
| 33 | IGHGP | IGHV3/OR16-13 | IGHV4-34 | 70 | 0.8509 |
| 36 | IGHGP | IGHV3-30 | IGHV3-33 | 70 | 0.8501 |
| 36 | IGHD3-22 | IGHV3-49 | IGHV3-64 | 70 | 0.8501 |
| 38 | IGHGP | IGHV3-30-3 | IGHV3/OR16-8 | 70 | 0.8493 |
| 39 | IGHGP | IGHV1/OR15-9 | IGHV3-3C-3 | 70 | 0.8493 |
| 40 | IGHGP | IGHV3-30-3 | IGHV3-64D | 70 | 0.8485 |

(continued)

| 41 | IGHGP | IGHV3-30 | IGHV7-81 | 70 | 0.8477 |
|----|-------|----------|----------|-----|--------|
| 41 | IGHD3-22 | IGHV1-3 | IGHV3-49 | 70 | 0.8477 |
| 43 | IGHGP | IGHV4-34 | IGHV5-10-1 | 70 | 0.8468 |
| 43 | IGHD1-26 | IGHGP | IGHV4-34 | 70 | 0.8468 |
| 43 | IGHD1-26 | IGHD6-6 | IGHGP | 70 | 0.8468 |
| 43 | IGHD3-22 | IGHV3-38 | IGHV3-49 | 70 | 0.8468 |
| 47 | IGHGP | IGHV1-3 | IGHV3-49 | 70 | 0.8460 |
| 47 | IGHV3-30-3 | IGHV3-33 | IGHV3-49 | 70 | 0.8460 |
| 47 | IGHD3-22 | IGHV3-49 | IGHV4-28 | 70 | 0.8460 |
| 50 | IGHV3-30 | IGHV3-33 | IGHV3-49 | 70 | 0.8460 |

3. Prediction differentiation model for ME/CFS differential diagnosis

A prediction model formula for differentiating ME/CFS patients was created using usage frequency data for IGH genes obtained from BCR repertoire analysis. A differential model according to a logistic regression formula was created using a combination of IGH genes with a high AUC value in polynomial logistic analysis or significance test between two groups. An example of differential diagnosis is shown below.
For example, a combination of variables can be identified as follows.

(1) Healthy individuals and ME/CFS patients are compared, and a variable detected to have a significant difference (e.g., usage frequency of a gene in an IgG H chain variable region of a BCR) is selected; or
(2) Univariate logistic analysis is performed with one variable (e.g., one gene in an IgG H chain variable region of a BCR) as an independent variable, or multivariate logistic analysis is performed with two or more variables (e.g., two or more genes) as independent variables to obtain a logistic regression model formula. ROC analysis that measures the degree of fit of the regression model is performed to select a variable exhibiting a higher AUC value.

When a combination of variables is provided, univariate or multivariate logistic regression analysis is performed for each variable (e.g., frequency data for genes) $(x_1, x_2, x_3, ...)$, with ME/CFS patient (y = 1) or healthy individual (y = 0) as the objective variable. In case of (2), logistic analysis is performed for every combination using a plurality of variables.
While a function for generalized linear models glm of the R package can be utilized for logistic regression analysis, analysis is not limited to this package. With logistic regression analysis, the following logit model formula is obtained with the probability of having ME/CFS as n, and the constant of b0 and partial regression coefficients corresponding to b1 to bp are found at the same time. Specifically, the coefficients of a logit model formula can be determined by using a data set of variables (gene frequency and the like) and distinction of patient/healthy individual.

[Numeral 1]

$$\ln\left(\frac{\pi}{1-\pi}\right) = b_0 + b_1 x_1 + b_2 x_2 + \cdots + b_p x_p$$

Π: probability of having ME/CFS, b0: constant, b1 to bp: partial regression coefficients
The probability Π of having ME/CFS can be determined by the following equation once the constant and partial regression coefficients are determined. If frequency data is newly obtained, differentiation and prediction can be performed by inputting the values thereof (differentiation and prediction with 0.5 or greater as ME/CFS or the like).

[Numeral 2]

$$\pi = \frac{1}{1 + e^{-(b_0 + b_1 x_1 + b_2 x_2 + \cdots + b_p x_p)}}$$

Two steps enable a prediction method for predicting ME/CFS with a differentiation formula for distinguishing ME/CFS from healthy individuals and excluding MS patients with a differentiation formula for distinguishing ME/CFS from MS. Basically, frequency data can be inputted into the same logit model formula described above to predict the probability thereof.

3-1. One step prediction model

ME/CFS and non-ME/CFS (healthy individuals and MS patients) are predicted with a single differentiation formula. Patients are differentiated with a single step by one of the following examples or a combination of other predictor variables.

[Numeral 3]

(Example using 13 types of IGHs found to have a significant difference between the two groups)

**Predictor variables:** IGHV3-49, IGHV3-30-3, IGHD1-26, IGHGP, IGHV4-34, IGHV3-30, IGHV4-31, IGHV3-64, IGHJ6, IGHD3-22, IGHV3-33, IGHV3-73, IGHV5-10-1
**Differentiation formula:**

$$\log\left(\frac{\pi}{1-\pi}\right) = -2.445e^2 + 1.830e^2(\%IGHV3\text{-}49) + 1.348e^2(\%IGHV3\text{-}30\text{-}3) + 3.975e^{-1}(\%IGHD1\text{-}26)$$
$$- 2.328e^3(\%IGHGP) + 3.728e^1(\%IGHV4\text{-}34) + 8.355e^1(\%IGHV3\text{-}30)$$
$$+ 7.964e^1(\%IGHV4\text{-}31) - 2.840(\%IGHV3\text{-}64) + 5.919(\%IGHJ6) - 1.543e^1(\%IGHD3\text{-}22)$$
$$- 1.146e^2(\%IGHV3\text{-}33) - 1.287e^1(\%IGHV3\text{-}73) + 1.306e^2(\%IGHV5\text{-}10\text{-}1)$$

Π: probability of having ME/CFS, Π/(1-Π) is the odds ratio

(Example using IGHs of two variables with a high AUC value)

Predictor variables: IGHGP, IGHV3-30-3
**Differentiation formula:**

$$\log\left(\frac{\pi}{1-\pi}\right) = -3.094e^{-1} - 2.0.05e^1(\%IGHGP) + 1.496e^1(\%IGHV3\text{-}30\text{-}3)$$

(Example using IGHs of three variables with a high AUC value)

**Predictor variables:** IGHGP, IGHV3-30, IGHV3-49
**Differentiation formula:**

$$\log\left(\frac{\pi}{1-\pi}\right) = -1.770 - 2.269e^1(\%IGHGP) + 1.778(\%IGHV3\text{-}30\text{-}3) + 8.467e^{-1}(\%IGHV3\text{-}49)$$

3-2. Two step prediction model

ME/CFS is differentiated from healthy individuals with differentiation formula 1, and then patients with other diseases such as MS are excluded with differentiation formula 2 to predict ME/CFS patients. Patients are differentiated with two steps by one of the following examples or a combination of other predictor variables.

1) Differentiation of ME/CFS patients from healthy individuals (differentiation formula 1)

[Numeral 4]

(Example using 6 types of IGHs found to have a significant difference between the two groups)

Predictor variables: IGHV1-3, IGHV3-30, IGHV3-30-3, IGHD1-26, IGHV3-49, IGHJ6
**Differentiation formula:**

$$\log\left(\frac{\pi}{1-\pi}\right) = -6.104 + 4.368e^{1}(\%IGHV1\text{-}3) - 6.1560e^{-1}(\%IGHV3\text{-}30) + 1.838(\%IGHV3\text{-}30\text{-}3) \\ + 9.945e^{-1}(\%IGHV3\text{-}49) + 1.7019e^{-1}(\%IGHD1\text{-}26) + 1.1956e^{-1}(\%IGHJ6)$$

(Example using IGHs of two variables with a high AUC value)

Predictor variables: IGHGP, IGHV3-30-3

$$\log\left(\frac{\pi}{1-\pi}\right) = -3.883e^{-1} - 2.096e^{1}(\%IGHGP) + 2.169(\%IGHV3\text{-}30\text{-}3)$$

(Example using IGHs of three variables with a high AUC value)

Predictor variables: IGHGP, IGHV3-30-3. IGHV3-49

$$\log\left(\frac{\pi}{1-\pi}\right) = -2.464 - 2.561e^{1}(\%IGHGP) + 2.877(\%IGHV3\text{-}30\text{-}3) + 1.154(\%IGHV3\text{-}49)$$

2) Differentiation to exclude other diseases (MS patient) from ME/CFS patients (differentiation formula 2)

[Numeral 5]

**Predictor variables:** IGHV3-7, IGHV3-23, IGHV3-23D, IGHV3-33, IGHV3-73, IGHV3-NL1, IGHV4-28, ICHV4-39, IGHD4-17, IGHD5-5, IGHD5-18
**Differentiation formula:**

$$\log\left(\frac{\pi}{1-\pi}\right) = 4.815 - 4.252e^{-1}(\%IGHV3\text{-}7) - 6.898e^{-2}(\%IGHV3\text{-}23) + 5.111(\%IGHV3\text{-}23D) \\ - 3.214e^{-1}(\%IGH3\text{-}33) - 5.660e^{-1}(\%IGHV3\text{-}73) - 9.159e^{-1}(\%IGHV3\text{-}NL1) \\ - 4.658e^{1}(\%IGHD4\text{-}28) - 8.966e^{-2}(\%IGHV4\text{-}39) - 9.326e^{-2}(\%IGHD4\text{-}17) \\ + 3.157e^{1}(\%IGHD5\text{-}5) - 3.240e^{1}(\%IGD5\text{-}18)$$

Differentiation formula using two types of IGHs as a predictor variable

**Predictor variables:** IGHGP, IGHV3-3

$$\log\left(\frac{\pi}{1-\pi}\right) = 3.023 + 6.026e^{-1}(\%IGHD3\text{-}3) - 7.002e^{1}(\%IGHGP)$$

Differentiation formula using three types of IGHs as a predictor variable

**Predictor variables:** IGHGP, IGHV3-3, IGHD1-14

$$\log\left(\frac{\pi}{1-\pi}\right) = 5.240 + 1.492(\%IGHD3\text{-}3) - 1.379(\%IGHGP) - 7.432(\%IGHD1\text{-}14)$$

(Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein.

(Related application)

The present application claims priority to Japanese Patent Application No. 2018-155380 filed on August 22, 2018 and Japanese Patent Application No. 2019-44885 filed on March 12, 2019. The entire content thereof is incorporated herein by reference for any purpose.

[Industrial Applicability]

The present disclosure can be used in diagnosis/diagnostic drug for myalgic encephalomyelitis/chronic fatigue syndrome (ME/CFS).

[Sequence Listing Free Text]

SEQ ID NO: 1: BSL18E primer
SEQ ID NO: 2: P20EA primer
SEQ ID NO: 3: P10EA primer
SEQ ID NO: 4: CG1 primer
SEQ ID NO: 5: CG2 primer
SEQ ID NO: 6: P22EA-ST1-R primer
SEQ ID NO: 7: CG-ST1-R primer

**Claims**

1. A method of using a B cell receptor (BCR) repertoire in a subject as an indicator of myalgic encephalomyelitis/chronic fatigue syndrome (ME/CFS) in the subject.

2. The method of claim 1, using one or more variables comprising a usage frequency of one or more genes in an IgG H chain variable region of a BCR in a subject as an indicator of myalgic encephalomyelitis/chronic fatigue syndrome (ME/CFS) in the subject.

3. The method of claim 2, further **characterized in that** the one or more variables is an indicator of the subject suffering from ME/CFS, and not another disease.

4. The method of any one of claims 1 to 3, wherein the one or more genes comprise at least one gene selected from the group consisting of IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81, IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27, IGHJ1, IGHJ2, IGHJ3, IGHJ4, IGHJ5, IGHJ6, IGHG1, IGHG2, IGHG3, IGHG4, and IGHGP.

5. The method of claim 4, wherein the one or more genes comprise at least one gene selected from the group consisting of IGHV3-73, IGHV1-69-2, IGHV5-51, IGHV4-31, IGHV3-23D, IGHV1/OR15-9, IGHV4-39, IGHD5-12, IGHV3-43D, IGHD4-17, IGHV5-10-1, IGHD4/OR15-4a/b, IGHG4, IGHV1/OR15-5, IGHV3/OR16-9, IGHD1-7, IGHV3-21, IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22.

6. The method of claim 5, wherein the one or more genes comprise at least one gene selected from the group consisting of IGHD6-6, IGHV3-33, IGHD4-23, IGHV3-30-5, IGHV3-23, IGHD6-13, IGHV3-64D, IGHV3-48, IGHV3-64, IGHG1, IGHV3-49, IGHV3-30-3, IGHD1-26, IGHJ6, IGHV3-30, IGHGP, IGHV1-3, and IGHD3-22.

7. The method of claim 6, wherein the one or more genes comprise at least one gene selected from the group consisting of IGHV3-49, IGHV3-30-3, IGHD1-26, IGHJ6, IGHV3-30, IGHGP, IGHV1-3, and IGHD3-22.

8. The method of claim 7, wherein the one or more genes comprise at least one gene selected from the group consisting of IGHV1-3, IGHV3-30, IGHV3-30-3, IGHV3-49, IGHD1-26, and IGHJ6.

9. The method of claim 2, wherein the one or more variables further comprise one or more immune cell subpopulation counts.

10. The method of claim 9, wherein the one or more variables exhibit AUC $\geq$ 0.7 under a ROC curve in regression analysis for differentiating a normal control from ME/CFS.

11. The method of claim 10, wherein the one or more variables exhibit AUC $\geq$ 0.8 under a ROC curve in regression analysis for differentiating a normal control from ME/CFS.

12. The method of any one of claims 9 to 11, wherein the immune cell subpopulation count is selected from a B cell count, a naive B cell count, a memory B cell count, a plasmablast count, an activated naive B cell count, a transitional B cell count, a regulatory T cell count, a memory T cell count, a follicular helper T cell count, a Tfh1 cell count, a Tfh2 cell count, a Tfh17 cell count, a Th1 cell count, a Th2 cell count, and a Th17 cell count.

13. The method of claim 2, wherein the one or more variables comprise usage frequencies of two or more genes in an IgG H chain variable region of a BCR in the subject.

14. The method of claim 13, wherein the one or more variables exhibit AUC $\geq$ 0.7 under a ROC curve in regression analysis for differentiating a normal control from ME/CFS.

15. The method of claim 14, wherein the one or more variables exhibit AUC $\geq$ 0.8 under a ROC curve in regression analysis for differentiating a normal control from ME/CFS.

16. The method of claim 15, wherein the one or more variables exhibit AUC $\geq$ 0.9 under a ROC curve in regression analysis for differentiating a normal control from ME/CFS.

17. The method of claim 2, wherein the one or more variables comprise a combination of two or more variables selected from the group consisting of a usage frequency of one or more genes in an IgG H chain variable region of a BCR in the subject, a BCR diversity index in the subject, and one or more immune cell subpopulation counts in the subject, and wherein the one or more variables exhibit AUC $\geq$ 0.7 under a ROC curve in regression analysis for differentiating a normal control from ME/CFS.

**18.** The method of claim 17, wherein the one or more variables comprise three of more variables selected from the group.

**19.** The method of claim 18, wherein the one or more variables comprise four of more variables selected from the group.

**20.** The method of claim 19, wherein the one or more variables comprise five of more variables selected from the group.

**21.** The method of claim 20, wherein the one or more variables comprise six of more variables selected from the group.

**22.** The method of any one of claims 17 to 21, wherein the one or more variables exhibit AUC $\geq$ 0.8 under a ROC curve in regression analysis for differentiating a normal control from ME/CFS.

**23.** The method of any one of claims 17 to 22, wherein the one or more variables exhibit AUC $\geq$ 0.9 under a ROC curve in regression analysis for differentiating a normal control from ME/CFS.

**24.** The method of claim 2, wherein the one or more genes comprise IGHV1-3, IGHV3-30, IGHV3-30-3, IGHV3-49, IGHD1-26, and IGHJ6.

**25.** The method of any one of claims 2 to 24, wherein the one or more variables comprise a B cell count in the subject.

**26.** The method of any one of claims 2 to 25, wherein the one or more variables comprise a regulatory T cell (Treg) count in the subject.

**27.** The method of any one of claims 2 to 26, wherein the usage frequency of the one or more genes is determined by a method comprising large scale highly efficient BCR repertoire analysis.

**28.** The method of any one of claims 2 to 27, wherein the one or more variables comprise a usage frequency of at least one gene selected from the group consisting of IGHV3-49, IGHV3-30-3, IGHD1-26, IGHV3-30, IGHJ6, IGHGP, IGHV4-31, IGHV3-64, IGHD3-22, IGHV3-33, IGHV3-73, IGHV5-10-1, and IGHV4-34.

**29.** The method of claims 1 to 27, comprising:

(a) using a part of the one or more variables as an indicator of ME/CFS in the subject; and
(b) using a part of the one or more variables as an indicator of the subject having ME/CFS, and not another disease.

**30.** The method of claim 29, wherein (b) is performed a plurality of times for a plurality of other diseases.

**31.** The method of claim 28 or 29, wherein the another disease comprises multiple sclerosis (MS).

**32.** A method of using one or more variables comprising a usage frequency of one or more genes in an IgG H chain variable region of a BCR in a subject as an indicator of the subject suffering from myalgic encephalomyelitis/chronic fatigue syndrome (ME/CFS), and not multiple sclerosis (MS).

**33.** The method of claim 32, wherein the one or more genes comprise at least one gene selected from the group consisting of IGHV1-2, IGHV1-3, IGHV1-8, IGHV1-18, IGHV1-24, IGHV1-38-4, IGHV1-45, IGHV1-46, IGHV1-58, IGHV1-69, IGHV1-69-2, IGHV1-69D, IGHV1/OR15-1, IGHV1/OR15-5, IGHV1/OR15-9, IGHV1/OR21-1, IGHV2-5, IGHV2-26, IGHV2-70, IGHV2-70D, IGHV2/OR16-5, IGHV3-7, IGHV3-9, IGHV3-11, IGHV3-13, IGHV3-15, IGHV3-16, IGHV3-20, IGHV3-21, IGHV3-23, IGHV3-23D, IGHV3-25, IGHV3-30, IGHV3-30-3, IGHV3-30-5, IGHV3-33, IGHV3-35, IGHV3-38, IGHV3-38-3, IGHV3-43, IGHV3-43D, IGHV3-48, IGHV3-49, IGHV3-53, IGHV3-64, IGHV3-64D, IGHV3-66, IGHV3-72, IGHV3-73, IGHV3-74, IGHV3-NL1, IGHV3/OR15-7, IGHV3/OR16-6, IGHV3/OR16-8, IGHV3/OR16-9, IGHV3/OR16-10, IGHV3/OR16-12, IGHV3/OR16-13, IGHV4-4, IGHV4-28, IGHV4-30-2, IGHV4-30-4, IGHV4-31, IGHV4-34, IGHV4-38-2, IGHV4-39, IGHV4-59, IGHV4-61, IGHV4/OR15-8, IGHV5-10-1, IGHV5-51, IGHV6-1, IGHV7-4-1, IGHV7-81, IGHD1-1, IGHD1-7, IGHD1-14, IGHD1-20, IGHD1-26, IGHD1/OR15-1a/b, IGHD2-2, IGHD2-8, IGHD2-15, IGHD2-21, IGHD2/OR15-2a/b, IGHD3-3, IGHD3-9, IGHD3-10, IGHD3-16, IGHD3-22, IGHD3/OR15-3a/b, IGHD4-4, IGHD4-11, IGHD4-17, IGHD4-23, IGHD4/OR15-4a/b, IGHD5-5, IGHD5-12, IGHD5-18, IGHD5-24, IGHD5/OR15-5a/b, IGHD6-6, IGHD6-13, IGHD6-19, IGHD6-25, IGHD7-27, IGHJ1, IGHJ2, IGHJ3, IGHJ4, IGHJ5, IGHJ6, IGHG1, IGHG2, IGHG3, IGHG4, and IGHGP.

**34.** The method of claim 32 or 33, wherein the one or more genes comprise at least one gene selected from the group consisting of IGHV1-3, IGHV3-30, IGHV3-30-3, IGHD1-26, IGHV3-49, and IGHJ6.

**35.** The method of claim 32, wherein the one or more variables comprise usage frequencies of two or more genes in an IgG H chain variable region of a BCR in the subject.

**36.** The method of claim 35, wherein the one or more variables exhibit AUC $\geq$ 0.7 under a ROC curve in regression analysis for differentiating MS from ME/CFS.

**37.** The method of claim 36, wherein the one or more variables exhibit AUC $\geq$ 0.8 under a ROC curve in regression analysis for differentiating MS from ME/CFS.

**38.** The method of claim 37, wherein the one or more variables exhibit AUC $\geq$ 0.9 under a ROC curve in regression analysis for differentiating MS from ME/CFS.

**39.** The method of claim 32, wherein the one or more variables comprise usage frequencies of three or more genes in an IgG H chain variable region of a BCR in the subject.

**40.** The method of claim 39, wherein the one or more variables exhibit AUC $\geq$ 0.7 under a ROC curve in regression analysis for differentiating MS from ME/CFS.

**41.** The method of claim 40, wherein the one or more variables exhibit AUC $\geq$ 0.8 under a ROC curve in regression analysis for differentiating MS from ME/CFS.

**42.** The method of claim 41, wherein the one or more variables exhibit AUC $\geq$ 0.9 under a ROC curve in regression analysis for differentiating MS from ME/CFS.

**43.** The method of claim 31, wherein (b) is performed by the method of any one of claims 32 to 42.

# FIG.1

Fig. 1

V gene family usage of BCR repertoire
Usage frequency distribution of IGHV chains

FIG.2

EP 3 842 547 A1

Fig. 2

## Usage frequency distributions of IGHD chains and IGHJ chains

Mann-Whitney U-test, *p<0.05, **p<0.01

EP 3 842 547 A1

Fig. 3A  |  IGHV chain family found to have a significant difference between patient/healthy individual

EP 3 842 547 A1

Fig. 3B | IGHD chain/IGHJ chain families found to have a significant difference between patient/healthy individual

IGHD1-26
p=0.0025

IGHJ6
p=0.025

# FIG.4

Fig. 4

# FIG.5

Fig. 5

FIG.6

Fig. 6

FIG.7

Fig. 7

FIG.8

Fig. 8

# FIG.9

Fig.9

## Difference in IGH gene usage frequency between ME/CFS patients (n = 37) and MS patients (n = 10)

FIG.10

Difference in diversity index between ME/CFS patients (n = 37) and MS patients (n = 10)

Fig.10

FIG.11

Difference in IGH gene usage frequency between ME/CFS patients (n = 37) and non-ME/CFS patients (n = 33)

Fig.11

**INTERNATIONAL SEARCH REPORT**

| International application No. |
| --- |
| PCT/JP2019/032673 |

**A. CLASSIFICATION OF SUBJECT MATTER**
Int.Cl.  C12Q1/6809(2018.01)i, C12Q1/02(2006.01)i, C12N15/13(2006.01)n, C12Q1/6869(2018.01)n, C12Q1/6883(2018.01)n

According to International Patent Classification (IPC) or to both national classification and IPC

**B. FIELDS SEARCHED**

Minimum documentation searched (classification system followed by classification symbols)
Int.Cl. C12Q1/6809, C12Q1/02, C12N15/13, C12Q1/6869, C12Q1/6883

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | ONO, H. et al., "Dysregulation of T and B cells in myalgic encephalomyelitis/chronic fatigue syndrome", Journal of the Neurological Science, 2017, pp. 899-900, 2502, WCN17-1676, in particular, page 899, right column, line 8 from the bottom to page 900, left column, line 8 | 1<br>2-28 |
| X<br>Y | 小野紘彦ほか, 筋痛性脳脊髄炎／慢性疲労症候群(myalgic encephalomyelitis/chronic fatigue syndrome:ME/CFS)における B 細胞異常の解析, 日本臨床免疫学会会誌, 2017, vol. 40, no. 4, page 306, P1-34, in particular, section "method and result", non-official translation (ONO, Hirohiko et al., "Analysis of B-cell abnormalities in myalgic encephalomyelitis/chronic fatigue syndrome (ME/CFS)", Japanese Journal of Clinical Immunology) | 1<br>2-28 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 November 2019 (14.11.2019) | 26 November 2019 (26.11.219) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
|---|
| PCT/JP2019/032673 |

C (Continuation).　DOCUMENTS CONSIDERED TO BE RELEVANT

| Category* | Citation of document, with indication, where appropriate, of the relevant passages | Relevant to claim No. |
|---|---|---|
| Y | WO 2017/222056 A1 (RIKEN, INSTITUTE OF PHYSICAL AND CHEMICAL RESEARCH) 28 December 2017, in particular, claims, paragraphs [0273], [0274], [0292], [0296] & US 2019/0169679 A1, claims & EP 3486321 A1 & CN 109312327 A | 2-28 |
| Y | WO 2015/075939 A1 (REPERTOIRE GENESIS INCORPORATION) 28 May 2015, in particular, claims, preparation example 1 1& US 2016/0289760 A1, claims, analytical test 1 & EP 3091074 A1 & CN 106103711 A | 2-28 |
| A | JAIN, Vageesh et al., "Prevalence of and risk factors for severe cognitive and sleep symptoms in ME/CFS and MS", BMC Neurology, 2017, vol. 17, no. 117, pp. 1-10, in particular, abstract | 31-43 |
| PA | 佐藤和貴郎ほか，筋痛性脳脊髄炎／慢性疲労症候群の末梢血リンパ球解析 －B細胞受容体レパトアの偏倚の同定－，神経免疫学，14 September 2018 (accession date), vol. 23, no. 1, page 86, WS1-03, in particular, section "Method, result and conclusion", non-official translation (SATO, Wakiro et al., "Analysis of peripheral blood lymphocyte of myalgic encephalomyelitis/chronic fatigue syndrome – Identification of bias of the B-cell receptor repertoire-", Neuroimmunology) | 39-31 |
| A | HARDCASTLE, Sharni Lee et al., "Characterisation of cell functions and receptors in chronic Fatigue Syndrome/Myalgic Encephalomyelitis (CSF/ME)", BMC Immunology, 2015, vol. 16, no. 35, pp. 1-12 | 1-43 |
| A | NGUYEN, Chinh Bkrong et al., "Whole blood gene expression in adolescent chronic fatigue syndrome:an exploratry cross-sectional study suggesting altered B cell differentiation and survival", J. Transl. Med, 2017, vol. 15, no. 102, pp. 1-21 | 1-43 |
| A | BOUQUET, Jerome et al., "RNA-Seq Analysis of Gene Expression, Viral Pathogen, and B-Cell/T-Cell Receptor Signatures in Complex Chronic Disease", Clinical Infectious Diseases, 2017, vol. 64, no. 4, pp. 476-81 | 1-43 |
| A | RAMOS, S. et al., "Characterisation of B cell Subsets and Receptors in Chronic Fatigue Syndrome Patients", J.Clin.Cell Immunol, 2015, vol. 6, no. 1, pp. 1-5 | 1-43 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015075939 A
- JP 2018155380 A
- JP 2019044885 A

SEQUENCE LISTING

<110> NATIONAL CENTER OF NEUROLOGY AND PSYCHIATRY
REPERTOIRE GENESIS INCORPORATION

<120> Biomarker of Myalgic Encephalomyelitis/Chronic Fatigue Syndrome (ME/CFS)

<130> J1190MD032

<150> JP 2018-155380
<151> 2018-08-22

<160> 7

<170> PatentIn version 3.5

<210> 1
<211> 35
<212> DNA
<213> artificial sequence

<220>
<223> BSL18E Primer

<220>
<221> misc_feature
<222> (35)..(35)
<223> n is a, c, g, t or u

<400> 1
aaagcggccg catgcttttt tttttttttt tttvn                                    35

<210> 2
<211> 10
<212> DNA
<213> artificial sequence

<220>
<223> P20EA Primer

<400> 2
gggaattcgg                                                               10

<210> 3
<211> 20
<212> DNA
<213> artificial sequence

<220>
<223> P10EA Primer

<400> 3
taatacgact ccgaattccc                                                    20

<210> 4
<211> 21
<212> DNA
<213> artificial sequence

```
<220>
<223>  CG1 Primer

<400>  4
caccttggtg ttgctgggct t                                              21


<210>  5
<211>  21
<212>  DNA
<213>  artificial sequence

<220>
<223>  CG2 Primer

<400>  5
tcctgaggac tgtaggacag c                                              21


<210>  6
<211>  55
<212>  DNA
<213>  artificial sequence

<220>
<223>  P22EA-ST1-R Primer

<400>  6
gtctcgtggg ctcggagatg tgtataagag acagctaata cgactccgaa ttccc         55


<210>  7
<211>  54
<212>  DNA
<213>  artificial sequence

<220>
<223>  CG-ST1-R Primer

<400>  7
tcgtcggcag cgtcagatgt gtataagaga cagtgagttc cacgacaccg tcac          54
```